# EUROPEAN PATENT APPLICATION

(11) **EP 4 471 028 A1**
(43) Date of publication of application: **04.12.2024**
(21) Application number: 23746164.5
(22) Date of filing: 18.01.2023
(51) Int. Cl.: C07D 471/04, C07D 519/00, A61K 31/4745, A61K 31/444, A61K 31/437, A61P 35/00

(54) **HYDRAZINO GROUP-CONTAINING COMPOUND**

(30) Priority: 26.01.2022 CN 202210092492; 09.09.2022 CN 202211104139; 10.01.2023 CN 202310035797
(71) Applicant: Chia Tai Tianqing Pharmaceutical Group Co., Ltd., Lianyungang, Jiangsu 222062 (CN)
(72) Inventor: ZHANG, Yinsheng, Lianyungang, Jiangsu 222062 (CN); AO, Wangwei, Lianyungang, Jiangsu 222062 (CN); ZHANG, Li, Lianyungang, Jiangsu 222062 (CN); JIN, Hui, Lianyungang, Jiangsu 222062 (CN); HAN, Xulin, Lianyungang, Jiangsu 222062 (CN)
(74) Representative: Grünecker Patent- und Rechtsanwälte PartG mbB
(86) International application number: PCT/CN2023/072792
(87) International publication number: WO 2023/143282

(57) **Abstract**

The present application relates to the field of pharmaceutical chemistry, provides a hydrazino group-containing compound, specifically relates to a compound of formula (I-A), a pharmaceutically acceptable salt thereof, a pharmaceutical composition, or a preparation method therefor, and relates to a use thereof in the preparation of a drug for treating tumors.

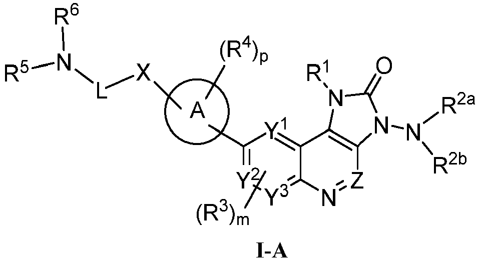

## Description

### CROSS-REFERENCE TO RELATED APPLICATIONS

The present application claims the priority to and the benefit of the Chinese Patent Application No. 202210092492.6 filed with China National Intellectual Property Administration on Jan. 26, 2022, the Chinese Patent Application No. 202211104139.1 filed with China National Intellectual Property Administration on Sept. 9, 2022, and the Chinese Patent Application No. 202310035797.8 filed with China National Intellectual Property Administration on Jan. 10, 2023, the disclosure of each of which is incorporated herein by reference in its entirety.

### TECHNICAL FIELD

The present application belongs to the field of pharmaceutical chemistry, provides a hydrazine group-containing compound, a pharmaceutically acceptable salt thereof, a pharmaceutical composition thereof, or a preparation method thereof, and relates to use thereof in the preparation of a medicament for treating tumors.

### BACKGROUND

Ataxia telangiectasia mutated (ATM) gene is closely related to DNA damage response (DDR) of organism. Patients with an ATM gene mutation generally show to be particularly sensitive to X-ray, and their DNA repair capability is significantly reduced. The product encoded by the ATM gene is an ATM protein, which is a serine/threonine protein kinase, is one member of the phosphatidylinositol 3-kinase (PI3K) related kinase (PI3K-related kinase, PIKK) protein family, and has a catalytic domain which is homologous with PI3K. ATM kinase is mainly located in the nucleus and microsomes. It primarily functions in S/G2/M cell cycle transitions and at replication forks to initiate cell cycle checkpoints, chromatin modification, HR repair, and pro-survival signaling cascades. ATM kinase is involved in a variety of key cellular functions such as cell growth, cell proliferation, migration, differentiation, survival, and cell adhesion. In particular, ATM kinase responds to DNA damage by activating cell cycle arrest and DNA repair programs, that is, when a DNA double strand breaks, ATM kinase is activated within minutes at the earliest, depolymerizes by autophosphorylation at Ser1981, and binds to the broken end of the DNA under the regulation of the MRN complex, thus helping the broken DNA to repair and maintain cell integrity.

In common anticancer therapy, there is a great amount of single-strand or double-strand break damage to DNA. The activation of ATM kinase allows cancer cells to have enhanced self-repair function and escape from the apoptosis, necrosis, or autophagy process, and thereby the effect of the anticancer therapy is poor. ATM inhibitors can enhance the activity and sensitivity of anticancer therapeutic agents. Therefore, based on the role of ATM inhibitors in the treatment of malignant tumors, there is a great clinical need and market value for finding new effective ATM inhibitors.

### SUMMARY

The present application relates to a compound of formula I-A, a pharmaceutically acceptable salt thereof, or a stereoisomer thereof, 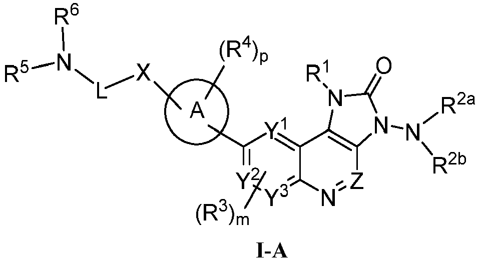 wherein,
R¹ is selected from the group consisting of hydrogen, C₁₋₁₀ alkyl, C₃₋₁₀ cycloalkyl, 3-10 membered heterocycloalkyl, C₃₋₁₀ aryl, and 3-10 membered heteroaryl, the C₁₋₁₀ alkyl, C₃₋₁₀ cycloalkyl, 3-10 membered heterocycloalkyl, C₃₋₁₀ aryl, or 3-10 membered heteroaryl being optionally substituted with one or more halogen, hydroxy, amino, cyano, C₁₋₆ alkyl, C₁₋₆ alkoxy, C₁₋₆ alkylNH-, (C₁₋₆ alkyl)₂N-, C₃₋₁₀ cycloalkyl, 3-10 membered heterocycloalkyl, C₃₋₁₀ aryl, or 3-10 membered heteroaryl;
R^{2a} and R^{2b} are each independently selected from the group consisting of hydrogen, C₁₋₆ alkyl, C₃₋₁₀ cycloalkyl, 3-10 membered heterocycloalkyl, C₃₋₁₀ aryl, and 3-10 membered heteroaryl, or R^{2a} and R^{2b} are linked to each other to form 3-12 membered heterocycloalkyl, the C₁₋₆ alkyl, C₃₋₁₀ cycloalkyl, 3-10 membered heterocycloalkyl, C₃₋₁₀ aryl, 3-10 membered heteroaryl, or 3-12 membered heterocycloalkyl being optionally substituted with one or more deuterium, halogen, hydroxy, amino, cyano, C₁₋₆ alkyl, C₁₋₆ alkoxy, C₁₋₆ alkylNH-, (C₁₋₆ alkyl)₂N-, C₃₋₁₀ cycloalkyl, 3-10 membered heterocycloalkyl, C₃₋₁₀ aryl, 3-10 membered heteroaryl, -COC₁₋₆ alkyl, -COOC₁₋₆ alkyl, -OCOC₁₋₆ alkyl, -CONHC₁₋₆ alkyl, -CON(C₁₋₆ alkyl)₂, -SO₂NHC₁₋₆ alkyl, or -SO₂N(C₁₋₆ alkyl)₂;
R³ and R⁴ are each independently selected from the group consisting of halogen, hydroxy, amino, cyano, nitro, C₁₋₆ alkyl, C₁₋₆ alkoxy, C₁₋₆ alkylNH-, and (C₁₋₆ alkyl)₂N-, the C₁₋₆ alkyl, C₁₋₆ alkoxy, C₁₋₆ alkylNH-, or (C₁₋₆ alkyl)₂N- being optionally substituted with one or more halogen, hydroxy, amino, cyano, nitro, or -COOH;
p and m are independently selected from the group consisting of 0, 1, 2, 3, and 4;
ring A is selected from the group consisting of phenyl and 5-10 membered heteroaryl;
X is selected from the group consisting of a single bond, -NR^{a}-, -O-, and -S-;
Y¹, Y², Y³, and Z are each independently selected from the group consisting of N and CH, and at least one of Y¹, Y², Y³, and Z is CH;
R^{a} is selected from the group consisting of hydrogen and C₁₋₆ alkyl;
L is selected from the group consisting of C₁₋₆ alkylene, C₃₋₁₀ cycloalkyl, 3-10 membered heterocycloalkyl, -C₁₋₆ alkylene-C₃₋₁₀ cycloalkyl-, -C₁₋₆ alkylene-3-10 membered heterocycloalkyl, -C₃₋₁₀ cycloalkyl-C₁₋₆ alkylene-, and -3-10 membered heterocycloalkyl-C₁₋₆ alkylene-;
R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-12 membered heterocycloalkyl, the C₁₋₆ alkyl, C₃₋₁₀ cycloalkyl, 3-10 membered heterocycloalkyl, or 3-12 membered heterocycloalkyl being optionally substituted with one or more groups selected from the group consisting of the following groups: deuterium, halogen, hydroxy, amino, cyano, C₁₋₆ alkyl, C₁₋₆ alkoxy, C₁₋₆ alkylNH-, (C₁₋₆ alkyl)₂N-, or C₁₋₆ alkyl substituted with one or more halogen, hydroxy, amino, or cyano.

The present application relates to a compound of formula **I,** a pharmaceutically acceptable salt thereof, or a stereoisomer thereof, 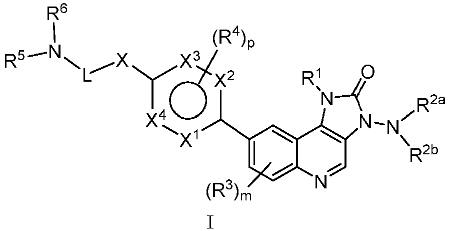 wherein,
R¹ is selected from the group consisting of hydrogen, C₁₋₆ alkyl, C₃₋₁₀ cycloalkyl, 3-10 membered heterocycloalkyl, C₃₋₁₀ aryl, and 3-10 membered heteroaryl, the C₁₋₆ alkyl, C₃₋₁₀ cycloalkyl, 3-10 membered heterocycloalkyl, C₃₋₁₀ aryl, or 3-10 membered heteroaryl being optionally substituted with one or more halogen, hydroxy, amino, cyano, C₁₋₆ alkyl, C₁₋₆ alkoxy, C₁₋₆ alkylNH-, or (C₁₋₆ alkyl)₂N-;
R^{2a} and R^{2b} are each independently selected from the group consisting of hydrogen and C₁₋₆ alkyl, or R^{2a} and R^{2b} are linked to each other to form 3-10 membered heterocycloalkyl, the C₁₋₆ alkyl or 3-10 membered heterocycloalkyl being optionally substituted with one or more deuterium, halogen, hydroxy, amino, cyano, C₁₋₆ alkyl, C₁₋₆ alkoxy, C₁₋₆ alkylNH-, or (C₁₋₆ alkyl)₂N-;
R³ and R⁴ are each independently selected from the group consisting of halogen, hydroxy, amino, cyano, C₁₋₆ alkyl, C₁₋₆ alkoxy, C₁₋₆ alkylNH-, and (C₁₋₆ alkyl)₂N-;
p and m are independently selected from the group consisting of 0, 1, and 2;
X¹, X², X³, and X⁴ are each independently selected from the group consisting of N and CH, and one or more of X¹, X², X³, and X⁴ are N;
X is selected from the group consisting of a single bond, -NR^{a}-, -O-, and -S-;
R^{a} is selected from the group consisting of hydrogen and C₁₋₆ alkyl;
L is selected from the group consisting of C₁₋₆ alkylene, C₃₋₁₀ cycloalkyl, 3-10 membered heterocycloalkyl, -C₁₋₆ alkylene-C₃₋₁₀ cycloalkyl-, -C₁₋₆ alkylene-3-10 membered heterocycloalkyl, -C₃₋₁₀ cycloalkyl-C₁₋₆ alkylene-, and -3-10 membered heterocycloalkyl-C₁₋₆ alkylene-;
R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more deuterium, halogen, hydroxy, amino, cyano, C₁₋₆ alkyl, C₁₋₆ alkoxy, C₁₋₆ alkylNH-, or (C₁₋₆ alkyl)₂N-.

In some embodiments, R¹ is selected from the group consisting of hydrogen, C₁₋₆ alkyl, C₃₋₁₀ cycloalkyl, 3-10 membered heterocycloalkyl, C₃₋₁₀ aryl, and 3-10 membered heteroaryl, the C₁₋₆ alkyl, C₃₋₁₀ cycloalkyl, 3-10 membered heterocycloalkyl, C₃₋₁₀ aryl, or 3-10 membered heteroaryl being optionally substituted with one or more halogen, hydroxy, amino, cyano, C₁₋₆ alkyl, C₁₋₆ alkoxy, C₁₋₆ alkylNH-, or (C₁₋₆ alkyl)₂N-.

In some embodiments, R¹ is selected from the group consisting of hydrogen, C₁₋₄ alkyl, C₃₋₈ cycloalkyl, 3-8 membered heterocycloalkyl, C₃₋₈ aryl, and 3-8 membered heteroaryl, the C₁₋₄ alkyl, C₃₋₈ cycloalkyl, 3-8 membered heterocycloalkyl, C₃₋₈ aryl, or 3-8 membered heteroaryl being optionally substituted with one or more halogen, hydroxy, amino, cyano, C₁₋₄ alkyl, C₁₋₄ alkoxy, C₁₋₄ alkylNH-, or (C₁₋₄ alkyl)₂N-.

In some embodiments, R¹ is selected from the group consisting of hydrogen, C₁₋₄ alkyl, C₃₋₆ cycloalkyl, 3-6 membered heterocycloalkyl, C₃₋₆ aryl, and 3-6 membered heteroaryl, the C₁₋₄ alkyl, C₃₋₆ cycloalkyl, 3-6 membered heterocycloalkyl, C₃₋₆ aryl, or 3-6 membered heteroaryl being optionally substituted with one or more halogen, hydroxy, amino, cyano, C₁₋₄ alkyl, C₁₋₄ alkoxy, C₁₋₄ alkylNH-, or (C₁₋₄ alkyl)₂N-.

In some embodiments, R¹ is selected from the group consisting of C₁₋₃ alkyl, C₃₋₆ cycloalkyl, 5-6 membered heterocycloalkyl, C₅₋₆ aryl, and 5-6 membered heteroaryl, the C₃₋₆ cycloalkyl, 5-6 membered heteroaryl, C₅₋₆ aryl, or 5-6 membered heteroaryl being optionally substituted with one or more halogen, hydroxy, amino, cyano, C₁₋₃ alkyl, or C₁₋₃ alkoxy.

In some embodiments, R¹ is selected from the group consisting of C₁₋₃ alkyl, C₃₋₅ cycloalkyl, 6-membered heterocycloalkyl, phenyl, and 6-membered heteroaryl, the C₃₋₅ cycloalkyl, 6-membered heterocycloalkyl, phenyl, or 6-membered heteroaryl being optionally substituted with one or more halogen or C₁₋₃ alkoxy.

In some embodiments, R¹ is selected from the group consisting of propyl, cyclopropyl, cyclobutyl, cyclopentyl, 6-membered oxygen-containing heterocycloalkyl, and 6-membered nitrogen-containing heteroaryl, the cyclobutyl, cyclopentyl, 6-membered oxygen-containing heterocycloalkyl, or 6-membered nitrogen-containing heteroaryl being optionally substituted with one or more fluoro or methoxy.

In some embodiments, R¹ is selected from the group consisting of propyl, cyclopropyl, cyclobutyl, cyclopentyl, tetrahydropyranyl, and pyridinyl, the cyclobutyl, cyclopentyl, or pyridinyl being optionally substituted with one or more fluoro or methoxy.

In some embodiments, R¹ is selected from the group consisting of isopropyl,

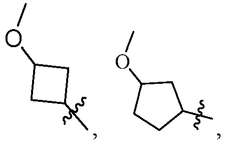

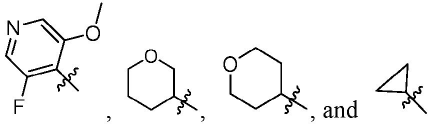

In some specific embodiments, R¹ is selected from the group consisting of hydrogen, C₁₋₆ alkyl, C₃₋₁₀ cycloalkyl, and 3-10 membered heterocycloalkyl, the C₁₋₆ alkyl, C₃₋₁₀ cycloalkyl, or 3-10 membered heterocycloalkyl being optionally substituted with one or more halogen, hydroxy, amino, cyano, C₁₋₆ alkyl, C₁₋₆ alkoxy, C₁₋₆ alkylNH-, or (C₁₋₆ alkyl)₂N-.

In some specific embodiments, R¹ is selected from the group consisting of C₁₋₄ alkyl, C₃₋₈ cycloalkyl, and 3-8 membered heterocycloalkyl, the C₁₋₆ alkyl, C₃₋₈ cycloalkyl, or 3-8 membered heterocycloalkyl being optionally substituted with one or more halogen, hydroxy, amino, cyano, C₁₋₄ alkyl, C₁₋₄ alkoxy, C₁₋₄ alkylNH-, or (C₁₋₄ alkyl)₂N-.

In some specific embodiments, R¹ is selected from the group consisting of C₁₋₄ alkyl, C₃₋₆ cycloalkyl, and 3-6 membered heterocycloalkyl, the C₁₋₄ alkyl, C₃₋₆ cycloalkyl, or 3-6 membered heterocycloalkyl being optionally substituted with one or more halogen, hydroxy, amino, cyano, C₁₋₄ alkyl, C₁₋₄ alkoxy, C₁₋₄ alkylNH-, or (C₁₋₄ alkyl)₂N-.

In some specific embodiments, R¹ is selected from the group consisting of C₁₋₃ alkyl, C₃₋₆ cycloalkyl, and 5-6 membered heterocycloalkyl, the C₁₋₃ alkyl, C₃₋₆ cycloalkyl, or 5-6 membered heterocycloalkyl being optionally substituted with one or more halogen, hydroxy, amino, cyano, C₁₋₃ alkyl, or C₁₋₃ alkoxy.

In some specific embodiments, R¹ is selected from the group consisting of C₁₋₃ alkyl, C₃₋₅ cycloalkyl, or 6-membered heterocycloalkyl, the C₁₋₃ alkyl, C₃₋₅ cycloalkyl, or 6-membered heterocycloalkyl being optionally substituted with one or more halogen or C₁₋₃ alkoxy.

In some specific embodiments, R¹ is selected from the group consisting of propyl, cyclopropyl, cyclobutyl, cyclopentyl, and 6-membered oxygen-containing heterocycloalkyl, the cyclopropyl, cyclobutyl, cyclopentyl, or 6-membered oxygen-containing heterocycloalkyl being optionally substituted with one or more fluoro or methoxy.

In some specific embodiments, R¹ is selected from the group consisting of isopropyl,

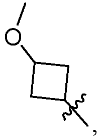

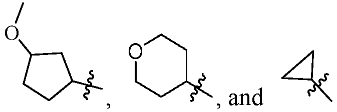

In some embodiments, R^{2a} and R^{2b} are each independently selected from the group consisting of hydrogen and C₁₋₆ alkyl, or R^{2a} and R^{2b} are linked to each other to form 3-10 membered heterocycloalkyl, the C₁₋₆ alkyl or 3-10 membered heterocycloalkyl being optionally substituted with one or more deuterium, halogen, hydroxy, amino, cyano, C₁₋₆ alkyl, C₁₋₆ alkoxy, C₁₋₆ alkylNH-, or (C₁₋₆ alkyl)₂N-.

In some embodiments, R^{2a} and R^{2b} are each independently selected from the group consisting of hydrogen and C₁₋₄ alkyl, or R^{2a} and R^{2b} are linked to each other to form 3-8 membered heterocycloalkyl, the C₁₋₄ alkyl or 3-8 membered heterocycloalkyl being optionally substituted with one or more deuterium, halogen, hydroxy, amino, cyano, C₁₋₄ alkyl, C₁₋₄ alkoxy, C₁₋₄ alkylNH-, or (C₁₋₄ alkyl)₂N-.

In some embodiments, R^{2a} and R^{2b} are each independently selected from the group consisting of hydrogen and C₁₋₄ alkyl, or R^{2a} and R^{2b} are linked to each other to form 3-6 membered heterocycloalkyl, the C₁₋₄ alkyl or 3-6 membered heterocycloalkyl being optionally substituted with one or more deuterium, halogen, hydroxy, amino, cyano, C₁₋₄ alkyl, C₁₋₄ alkoxy, C₁₋₄ alkylNH-, or (C₁₋₄ alkyl)₂N-.

In some embodiments, R^{2a} and R^{2b} are each independently selected from the group consisting of hydrogen and C₁₋₄ alkyl, or R^{2a} and R^{2b} are linked to each other to form 3-4 membered heterocycloalkyl, the C₁₋₄ alkyl or 3-4 membered heterocycloalkyl being optionally substituted with one or more deuterium, halogen, hydroxy, amino, cyano, C₁₋₄ alkyl, C₁₋₄ alkoxy, C₁₋₄ alkylNH-, or (C₁₋₄ alkyl)₂N-.

In some embodiments, R^{2a} and R^{2b} are each independently selected from the group consisting of hydrogen and C₁₋₄ alkyl, or R^{2a} and R^{2b} are linked to each other to form 3-4 membered heterocycloalkyl, the C₁₋₄ alkyl or 3-4 membered heterocycloalkyl being optionally substituted with one or more deuterium, halogen, hydroxy, amino, cyano, or C₁₋₃ alkyl.

In some embodiments, R^{2a} and R^{2b} are each independently selected from the group consisting of hydrogen and C₁₋₄ alkyl, the C₁₋₄ alkyl being optionally substituted with one or more deuterium, halogen, hydroxy, amino, or cyano.

In some embodiments, R^{2a} and R^{2b} are each independently selected from the group consisting of hydrogen and C₁₋₄ alkyl, the C₁₋₄ alkyl being optionally substituted with one or more deuterium, fluoro, chloro, or bromo.

In some embodiments, R^{2a} and R^{2b} are each independently selected from the group consisting of hydrogen and C₁₋₃ alkyl, the C₁₋₃ alkyl being optionally substituted with one or more deuterium.

In some embodiments, R^{2a} and R^{2b} are each independently selected from the group consisting of hydrogen and CH₃-, the CH₃- being optionally substituted with one or more deuterium.

In some embodiments, R^{2a} and R^{2b} are each independently selected from the group consisting of hydrogen, CH₃-, and CD₃-.

In some embodiments, R³ and R⁴ are each independently selected from the group consisting of halogen, hydroxy, amino, cyano, C₁₋₆ alkyl, C₁₋₆ alkoxy, C₁₋₆ alkylNH-, and (C₁₋₆ alkyl)₂N-.

In some embodiments, R³ and R⁴ are each independently selected from the group consisting of halogen, hydroxy, amino, cyano, C₁₋₃ alkyl, C₁₋₃ alkoxy, C₁₋₃ alkylNH-, and (C₁₋₃ alkyl)₂N-.

In some embodiments, R³ and R⁴ are each independently selected from the group consisting of halogen and C₁₋₃ alkoxy.

In some embodiments, R³ is selected from the group consisting of fluoro, chloro, bromo, and C₁₋₃ alkoxy.

In some embodiments, R³ is selected from the group consisting of fluoro and methoxy.

In some embodiments, R⁴ is selected from the group consisting of halogen, hydroxy, amino, cyano, methyl, and methoxy. In some embodiments, R⁴ is selected from the group consisting of halogen, hydroxy, amino, and cyano.

In some embodiments, p and m are independently selected from the group consisting of 0, 1, and 2.

In some embodiments, p is selected from the group consisting of 0 and 1. In some embodiments, p is 0.

In some embodiments, m is selected from the group consisting of 0 and 1. In some embodiments, m is 1. In some embodiments, m is 0.

In some embodiments, ring A is selected from the group consisting of phenyl, 5-6 membered heteroaryl, and 9-10 membered heteroaryl.

In some embodiments, ring A is selected from 5-6 membered heteroaryl. In some embodiments, ring A is selected from 6-membered heteroaryl. In some embodiments, ring A is selected from 5-6 membered nitrogen-containing heteroaryl. In some embodiments, ring A is selected from 6-membered nitrogen-containing heteroaryl.

In some embodiments, ring A is selected from the group consisting of 5-6 membered heteroaryl and 9-10 membered heteroaryl.

In some embodiments, ring A is selected from the group consisting of pyridinyl, pyrimidinyl, pyrazolyl, imidazolyl, triazolyl, pyrazinyl, quinolinyl, isoquinolinyl, indolyl, isoindolyl, triazinyl, benzimidazolyl, and imidazopyridinyl. In some embodiments, ring A is selected from the group consisting of pyridinyl and pyrimidinyl. In some embodiments, ring A is pyridinyl.

In some embodiments, X¹, X², X³, and X⁴ are each independently selected from the group consisting of N and CH, and one or more of X¹, X², X³, and X⁴ are N.

In some embodiments, X¹, X², X³, and X⁴ are each independently selected from the group consisting of N and CH, and 1 or 2 of X¹, X², X³, and X⁴ are N.

In some embodiments, X⁴ is selected from N, and X¹, X², and X³ are each independently selected from the group consisting of N and CH.

In some embodiments, X⁴ is selected from N, X³ is selected from the group consisting of N and CH, and X¹ and X² are CH.

In some embodiments, X⁴ is selected from N, and X¹, X², or X³ is CH.

In some embodiments, X³ and X⁴ are selected from N, and X¹ and X² are CH.

In some embodiments, X is selected from the group consisting of -NR^{a}- and -O-. In some embodiments, X is selected from the group consisting of -NH- and -O-. In some embodiments, X is -O-.

In some embodiments, Y¹, Y², Y³, and Z are each independently selected from the group consisting of N and CH, and 1, 2, 3, or 4 of Y¹, Y², Y³, and Z are CH.

In some embodiments, Y¹ is N, and Y² and Y³ are CH.

In some embodiments, Y² is N, and Y¹ and Y³ are CH.

In some embodiments, Y³ is N, and Y¹ and Y² are CH. In some embodiments, Y¹, Y², or Y³ is CH.

In some embodiments, Y¹, Y², Y³, or Z is CH.

In some embodiments, Z is selected from the group consisting of N and CH.

In some embodiments, Z is N. In some embodiments, Z is CH.

In some embodiments, R^{a} is selected from the group consisting of hydrogen and C₁₋₆ alkyl.

In some embodiments, R^{a} is selected from the group consisting of hydrogen and C₁₋₄ alkyl.

In some embodiments, R^{a} is selected from the group consisting of hydrogen and C₁₋₃ alkyl.

In some embodiments, R^{a} is selected from the group consisting of hydrogen and methyl. In some embodiments, R^{a} is hydrogen.

In some embodiments, L is selected from the group consisting of C₁₋₆ alkylene, C₃₋₁₀ cycloalkyl, 3-10 membered heterocycloalkyl, -C₁₋₆ alkylene-C₃₋₁₀ cycloalkyl-, -C₁₋₆ alkylene-3-10 membered heterocycloalkyl, -C₃₋₁₀ cycloalkyl-C₁₋₆ alkylene-, and -3-10 membered heterocycloalkyl-C₁₋₆ alkylene-.

In some embodiments, L is selected from the group consisting of C₁₋₄ alkylene, C₃₋₈ cycloalkyl, 3-8 membered heterocycloalkyl, -C₁₋₄ alkylene-C₃₋₈ cycloalkyl-, -C₁₋₄ alkylene-3-8 membered heterocycloalkyl-, -C₃₋₈ cycloalkyl-C₁₋₄ alkylene-, and -3-8 membered heterocycloalkyl-C₁₋₄ alkylene-.

In some embodiments, L is selected from the group consisting of C₁₋₄ alkylene, C₃₋₆ cycloalkyl, 3-6 membered heterocycloalkyl, -C₁₋₄ alkylene-C₃₋₆ cycloalkyl-, -C₁₋₄ alkylene-3-6 membered heterocycloalkyl-, -C₃₋₆ cycloalkyl-C₁₋₄ alkylene-, and -3-6 membered heterocycloalkyl-C₁₋₄ alkylene-.

In some embodiments, L is selected from the group consisting of C₁₋₄ alkylene, C₃₋₆ cycloalkyl, -C₁₋₄ alkylene-C₃₋₆ cycloalkyl-, and -C₃₋₆ cycloalkyl-C₁₋₄ alkylene-.

In some embodiments, L is selected from the group consisting of C₂₋₄ alkylene, C₄₋₆ cycloalkyl, -C₁₋₂ alkylene-C₄₋₆ cycloalkyl-, and -C₄₋₆ cycloalkyl-C₁₋₂ alkylene-.

In some embodiments, L is selected from the group consisting of C₂₋₃ alkylene, C₄₋₆ cycloalkyl, -CH₂-C₄ cycloalkyl-, and -C₄ cycloalkyl-CH₂-.

In some embodiments, L is selected from the group consisting of -CH₂CH₂-, -CH₂CH₂CH₂-,

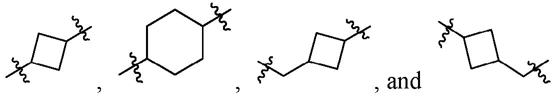

In some embodiments, L is selected from C₁₋₄ alkylene.

In some embodiments, L is selected from C₂₋₃ alkylene.

In some embodiments, L is selected from the group consisting of -CH₂CH₂- and -CH₂CH₂CH₂-. In some embodiments, L is -CH₂CH₂CH₂-.

In some embodiments, R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more groups selected from the group consisting of the following groups: deuterium, halogen, hydroxy, amino, cyano, C₁₋₆ alkyl, C₁₋₆ alkoxy, C₁₋₆ alkylNH-, (C₁₋₆ alkyl)₂N-, or C₁₋₆ alkyl substituted with one or more halogen, hydroxy, amino, or cyano.

In some embodiments, R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more groups selected from the group consisting of the following groups:deuterium, halogen, hydroxy, amino, cyano, C₁₋₆ alkyl, C₁₋₆ alkoxy, C₁₋₆ alkylNH-, (C₁₋₆ alkyl)₂N-, or C₁₋₆ alkyl substituted with one or more halogen.

In some embodiments, R⁵ and R⁶ are each independently selected from the group consisting of hydrogen, C₁₋₄ alkyl, C₃₋₈ cycloalkyl, and 3-8 membered heterocycloalkyl, or R⁵ and R⁶ are linked to each other to form 3-8 membered heterocycloalkyl, the C₁₋₄ alkyl, C₃₋₈ cycloalkyl, or 3-8 membered heterocycloalkyl being optionally substituted with one or more groups selected from the group consisting of the following groups:deuterium, halogen, hydroxy, amino, cyano, C₁₋₄ alkyl, C₁₋₄ alkoxy, C₁₋₄ alkylNH-, (C₁₋₄ alkyl)₂N-, or C₁₋₆ haloalkyl.

In some embodiments, R⁵ and R⁶ are each independently selected from the group consisting of C₁₋₄ alkyl and C₃₋₆ cycloalkyl, or R⁵ and R⁶ are linked to each other to form 3-7 membered heterocycloalkyl, the C₃₋₆ cycloalkyl or 3-7 membered heterocycloalkyl being optionally substituted with one or more groups selected from the group consisting of the following groups:deuterium, halogen, hydroxy, amino, cyano, C₁₋₃ alkyl, C₁₋₃ alkoxy, or C₁₋₄ haloalkyl.

In some embodiments, R⁵ and R⁶ are each independently selected from the group consisting of C₁₋₄ alkyl and C₃₋₆ cycloalkyl, or R⁵ and R⁶ are linked to each other to form 3-7 membered heterocycloalkyl, the 3-7 membered heterocycloalkyl being optionally substituted with one or more groups selected from the group consisting of the following groups:deuterium, halogen, hydroxy, amino, cyano, C₁₋₃ alkyl, C₁₋₃ alkoxy, or C₁₋₄ haloalkyl.

In some embodiments, R⁵ and R⁶ are each independently selected from the group consisting of C₁₋₃ alkyl and C₃₋₄ cycloalkyl, or R⁵ and R⁶ are linked to each other to form 4-, 5-, 6-, or 7-membered heterocycloalkyl, the heterocycloalkyl being optionally substituted with one or more halogen or C₁₋₃ haloalkyl.

In some embodiments, R⁵ and R⁶ are each independently selected from the group consisting of C₁₋₃ alkyl and C₃₋₄ cycloalkyl. In some embodiments, R⁵ and R⁶ are linked to each other to form 4-, 5-, 6-, or 7-membered heterocycloalkyl, the heterocycloalkyl being optionally substituted with one or more halogen or C₁₋₃ haloalkyl.

In some embodiments, R⁵ and R⁶ are linked to each other to form 4-, 5-, or 6-membered heterocycloalkyl, the heterocycloalkyl being optionally substituted with one or more halogen or C₁₋₃ haloalkyl. In some embodiments, R⁵ and R⁶ are linked to each other to form 4-membered heterocycloalkyl, the heterocycloalkyl being optionally substituted with one or more halogen or C₁₋₃ haloalkyl. In some embodiments, R⁵ and R⁶ are linked to each other to form 5-membered heterocycloalkyl, the heterocycloalkyl being optionally substituted with one or more halogen or C₁₋₃ haloalkyl. In some embodiments, R⁵ and R⁶ are linked to each other to form 6-membered heterocycloalkyl, the heterocycloalkyl being optionally substituted with one or more halogen or C₁₋₃ haloalkyl.

In some embodiments, R⁵ and R⁶ are each independently selected from the group consisting of methyl and cyclopropyl, or R⁵ and R⁶ are linked to each other to form azetidinyl, pyrrolidinyl, piperidinyl, azaspiroheptyl, and azabicycloheptyl, the azetidinyl or pyrrolidinyl being optionally substituted with one or more F or -CH₂F.

In some embodiments, R⁵ and R⁶ are each independently selected from the group consisting of methyl and cyclopropyl, or R⁵ and R⁶ are linked to each other to form azetidinyl, pyrrolidinyl, or piperidinyl, the azetidinyl, pyrrolidinyl, or piperidinyl being optionally substituted with one or more halogen or C₁₋₃ haloalkyl.

In some embodiments, R⁵ and R⁶ are each independently selected from the group consisting of methyl and cyclopropyl, or R⁵ and R⁶ are linked to each other to form azetidinyl, pyrrolidinyl, or piperidinyl, the azetidinyl or pyrrolidinyl being optionally substituted with one or more F or -CH₂F. In some specific embodiments, R⁵ and R⁶ are each independently selected from C₁₋₆ alkyl, or R⁵ and R⁶ are linked to each other to form 4-6 membered heterocycloalkyl, the C₁₋₆ alkyl or 4-6 membered heterocycloalkyl being optionally substituted with one or more groups selected from the group consisting of the following groups:halogen, hydroxy, amino, cyano, C₁₋₆ alkyl, C₁₋₆ alkoxy, or C₁₋₆ alkyl substituted with one or more halogen, hydroxy, amino, or cyano.

In some specific embodiments, R⁵ and R⁶ are each independently selected from C₁₋₆ alkyl, or R⁵ and R⁶ are linked to each other to form 4-6 membered heterocycloalkyl, the C₁₋₆ alkyl or 4-6 membered heterocycloalkyl being optionally substituted with one or more groups selected from the group consisting of the following groups:halogen, hydroxy, amino, cyano, or C₁₋₆ alkyl optionally substituted with halogen.

In some specific embodiments, R⁵ and R⁶ are each independently selected from C₁₋₃ alkyl, or R⁵ and R⁶ are linked to each other to form pyrrolidinyl or piperidinyl, the C₁₋₃ alkyl, pyrrolidinyl, or piperidinyl being optionally substituted with one or more groups selected from the group consisting of the following groups:halogen, hydroxy, amino, cyano, C₁₋₆ alkyl, C₁₋₆ alkoxy, or C₁₋₆ alkyl substituted with one or more halogen, hydroxy, amino, or cyano.

In some specific embodiments, R⁵ and R⁶ are each independently selected from C₁₋₃ alkyl, or R⁵ and R⁶ are linked to each other to form pyrrolidinyl or piperidinyl, the pyrrolidinyl or piperidinyl being optionally substituted with one or more groups selected from the group consisting of the following groups:halogen, hydroxy, amino, cyano, or C₁₋₆ alkyl optionally substituted with halogen.

In some specific embodiments, R⁵ and R⁶ are each independently selected from methyl, or R⁵ and R⁶ are linked to each other to form 5-6 membered heterocycloalkyl, the 5-6 membered heterocycloalkyl being optionally substituted with one or more -CH₂F.

In some specific embodiments, R⁵ and R⁶ are each independently selected from methyl, or R⁵ and R⁶ are linked to each other to form pyrrolidinyl or piperidinyl, the pyrrolidinyl or piperidinyl being optionally substituted with one or more -CH₂F.

In some other embodiments, R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more deuterium, halogen, hydroxy, amino, cyano, C₁₋₆ alkyl, C₁₋₆ alkoxy, C₁₋₆ alkylNH-, or (C₁₋₆ alkyl)₂N-.

In some other embodiments, R⁵ and R⁶ are each independently selected from the group consisting of hydrogen, C₁₋₄ alkyl, C₃₋₈ cycloalkyl, and 3-8 membered heterocycloalkyl, or R⁵ and R⁶ are linked to each other to form 3-8 membered heterocycloalkyl, the C₁₋₄ alkyl, C₃₋₈ cycloalkyl, or 3-8 membered heterocycloalkyl being optionally substituted with one or more deuterium, halogen, hydroxy, amino, cyano, C₁₋₄ alkyl, C₁₋₄ alkoxy, C₁₋₄ alkylNH-, or (C₁₋₄ alkyl)₂N-.

In some other embodiments, R⁵ and R⁶ are each independently selected from the group consisting of C₁₋₄ alkyl and C₃₋₆ cycloalkyl, or R⁵ and R⁶ are linked to each other to form 3-6 membered heterocycloalkyl, the C₃₋₆ cycloalkyl or 3-6 membered heterocycloalkyl being optionally substituted with one or more deuterium, halogen, hydroxy, amino, cyano, C₁₋₃ alkyl, or C₁₋₃ alkoxy.

In some other embodiments, R⁵ and R⁶ are each independently selected from the group consisting of C₁₋₃ alkyl and C₃₋₄ cycloalkyl or are linked to each other to form 5-6 membered heterocycloalkyl.

In some other embodiments, R⁵ and R⁶ are each independently selected from the group consisting of methyl and cyclopropyl or are linked to each other to form pyrrolidinyl or piperidinyl.

In some embodiments, when m is selected from 1, the linking position of R³ is as shown by

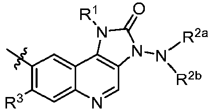

In some embodiments, R⁴ is linked to X¹ or X².

In some embodiments, the structural fragment 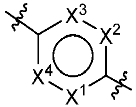 is selected from the group consisting of 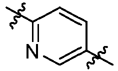 and 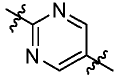 In some embodiments, the structural fragment 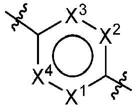 is

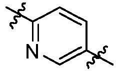

In some specific embodiments, the structural fragment 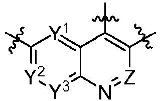 is selected from the group consisting of 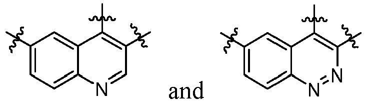 In some specific embodiments, the structural fragment

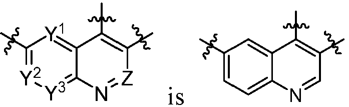

In some embodiments, the structural fragment 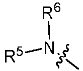 is selected from the group consisting of

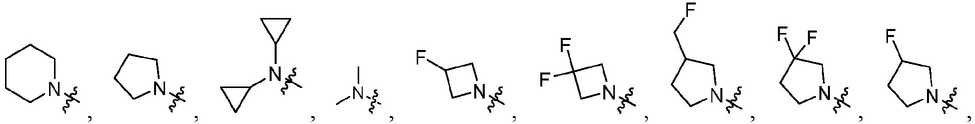

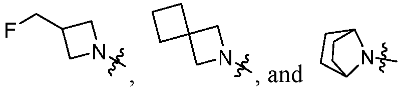

In some specific embodiments, the structural fragment 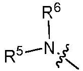 is selected from the group consisting of

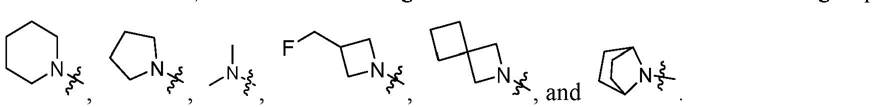

In some other embodiments, the structural fragment 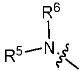 is selected from the group consisting of

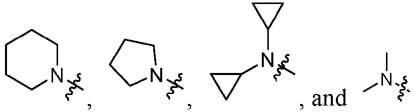

In some embodiments, the structural fragment 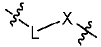 is selected from the group consisting of 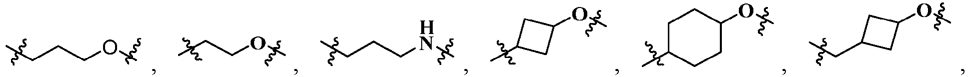 and 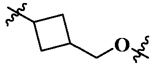 In some embodiments, the structural fragment 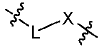 is selected from the group 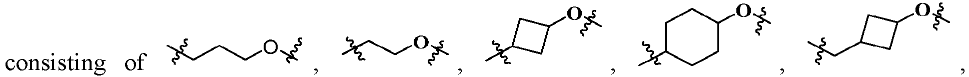 and 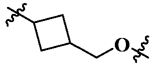 In some embodiments, the structural fragment 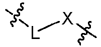 is selected from the group consisting of

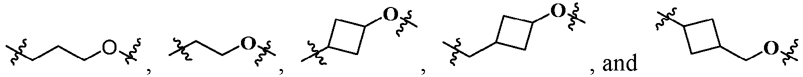

In some embodiments, the structural fragment 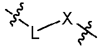 is selected from the group consisting of 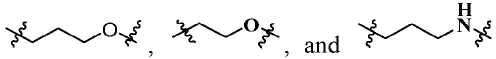 In some embodiments, the structural fragment 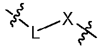 is selected from the group consisting of 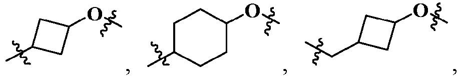 and 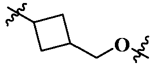 In some embodiments, the structural fragment 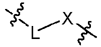 is selected from the group consisting of 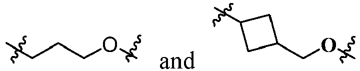 In some embodiments, the structural fragment 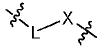 is selected from the group consisting of 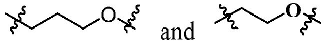 In some embodiments, the structural fragment 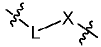 is selected from the group consisting of

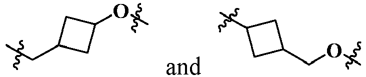

In some embodiments, the structural fragment 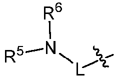 is selected from the group consisting of

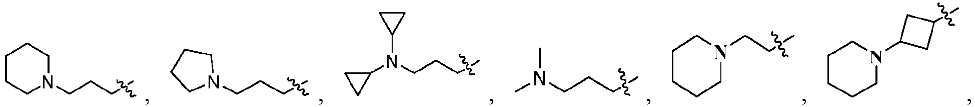

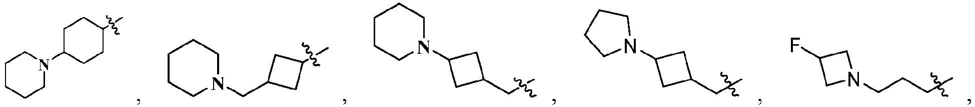

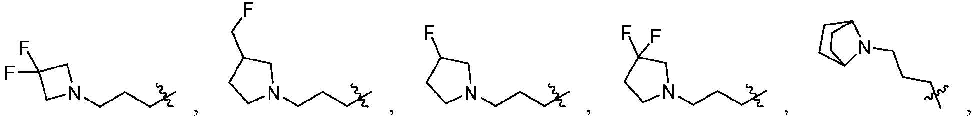

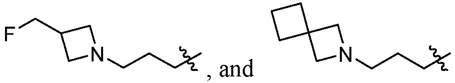

In some other embodiments, the structural fragment 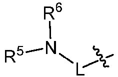 is selected from the group consisting of

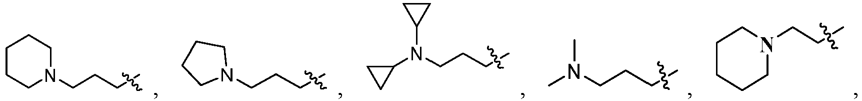

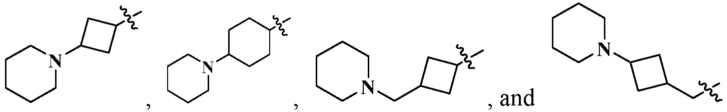

In some embodiments, the structural fragment 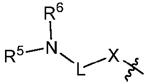 is selected from the group consisting 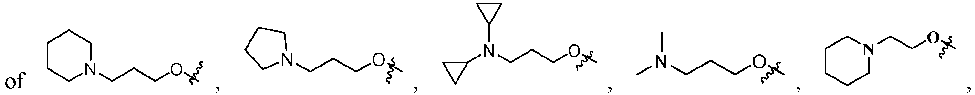 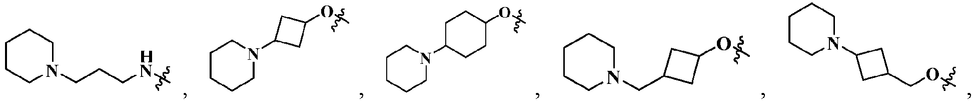 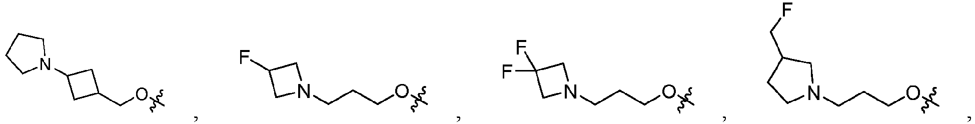 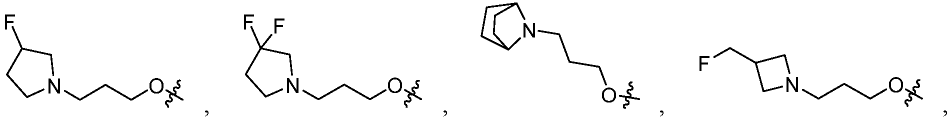 and

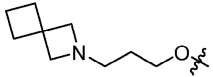

In some specific embodiments, the structural fragment 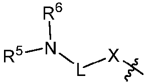 is selected from the group consisting of

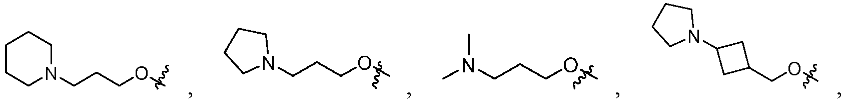

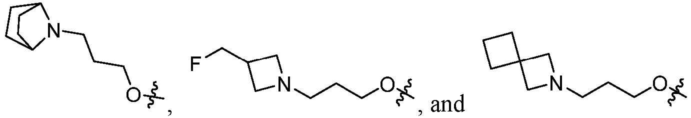

In some embodiments, the structural fragment 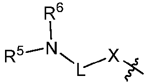 is selected from the group consisting of 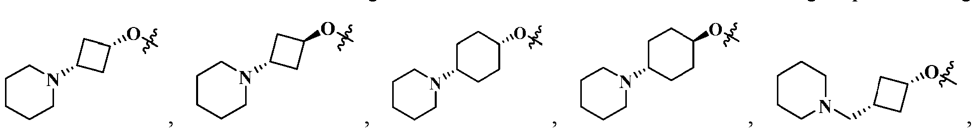 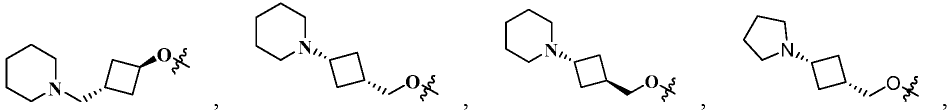 and

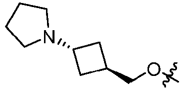

In some other embodiments, the structural fragment 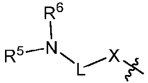 is selected from the group consisting of 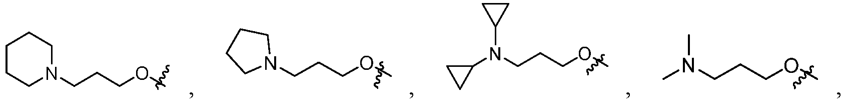 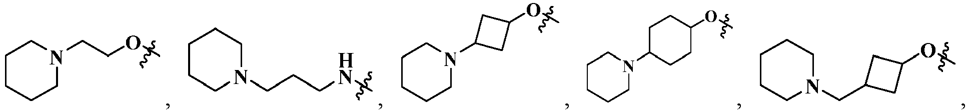 and

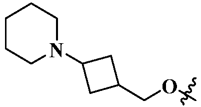

In some embodiments, the compound of formula **I-A** or the pharmaceutically acceptable salt thereof or the compound of formula **I** or the pharmaceutically acceptable salt thereof described herein is selected from a compound of formula **II** or a pharmaceutically acceptable salt thereof, 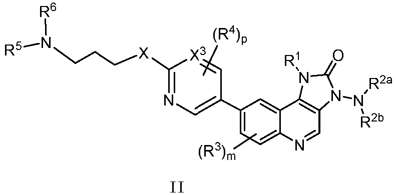 wherein, R¹, R^{2a}, R^{2b}, R³, R⁴, R⁵, R⁶, X, X³, m, or p is as defined above.

In some embodiments, the compound of formula **I-A** or the pharmaceutically acceptable salt thereof, the compound of formula **I** or the pharmaceutically acceptable salt thereof, or the compound of formula **II** or the pharmaceutically acceptable salt thereof described herein is selected from a compound of formula III or formula IV or a pharmaceutically acceptable salt thereof, 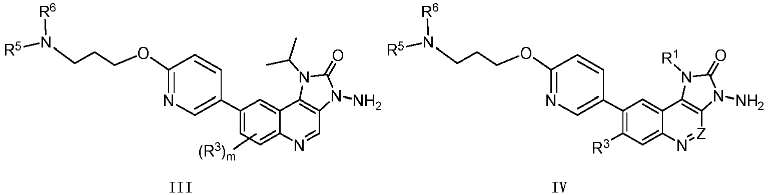 wherein, R¹, R³, R⁵, R⁶, m, or Z are as defined above.

In some embodiments, the present application encompasses the variables defined above and embodiments thereof, as well as any combination thereof.

The heteroatom in the heterocycloalkyl or heteroaryl described above is selected from the group consisting of nitrogen (NH or N), oxygen, sulfur (S), boron, Si, S(O), and S(O)₂, and the remaining ring atoms are selected from carbon. The heteroatom in the heterocycloalkyl or heteroaryl described above is selected from the group consisting of nitrogen (NH or N), oxygen, and sulfur (S), and the remaining ring atoms are selected from carbon. In some embodiments, the number of the heteroatom is selected from the group consisting of 1, 2, 3, and 4. In some embodiments, the number of the heteroatom is selected from the group consisting of 1, 2, and 3. In some embodiments, the number of the heteroatom is selected from the group consisting of 1 and 2.

The present application provides compounds, pharmaceutically acceptable salts thereof, or stereoisomers thereof as follows: 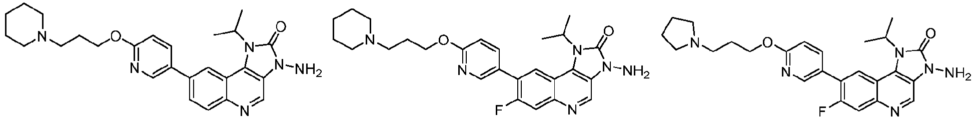 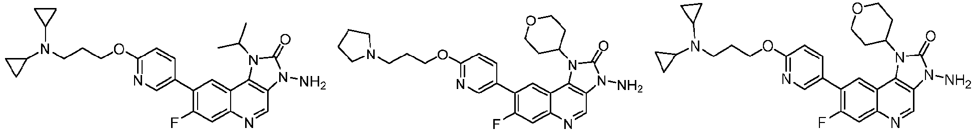 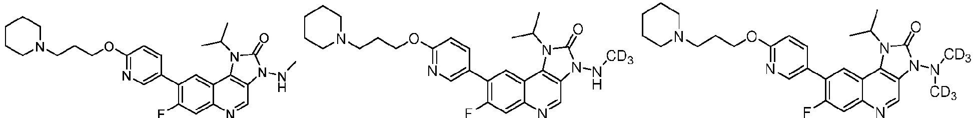 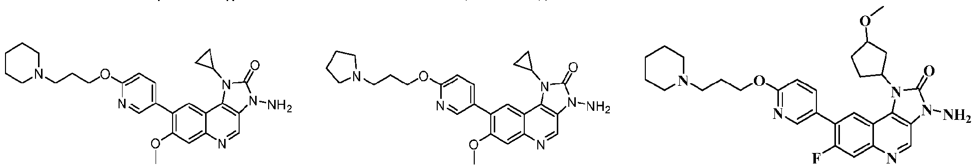 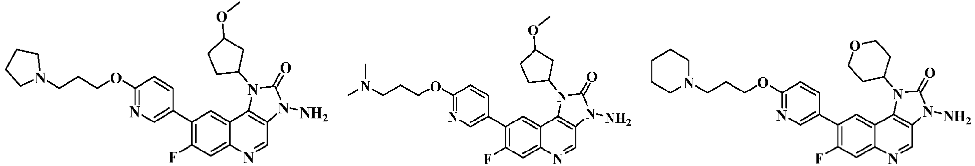 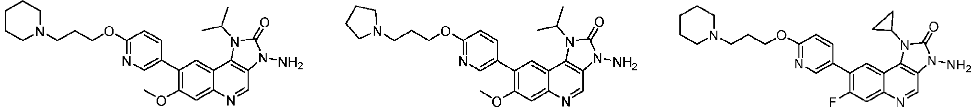 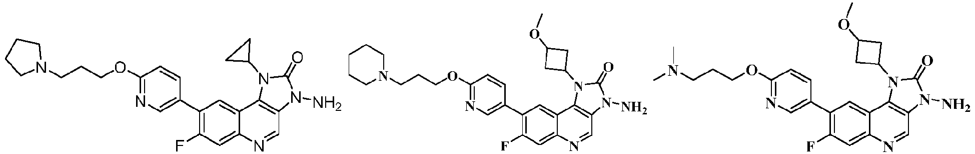 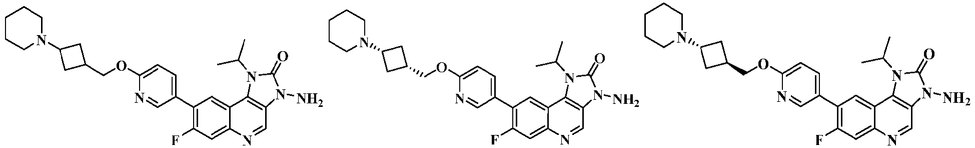 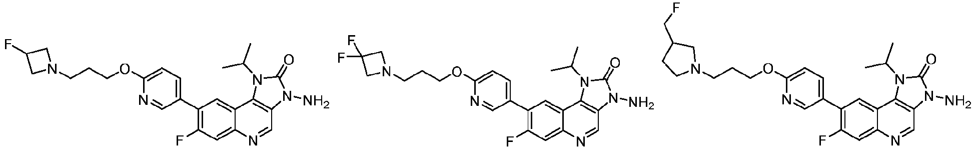 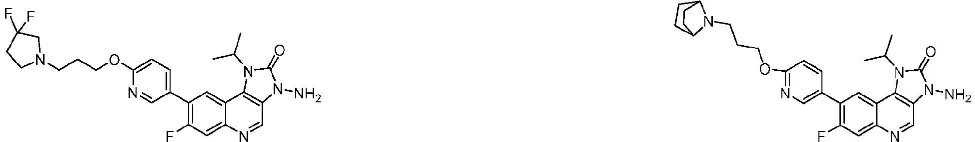 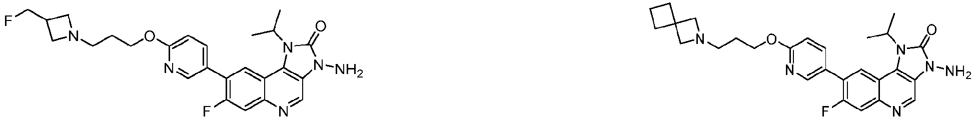 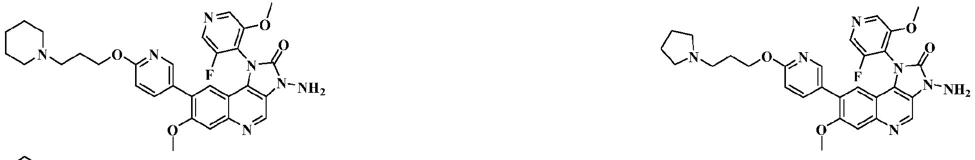 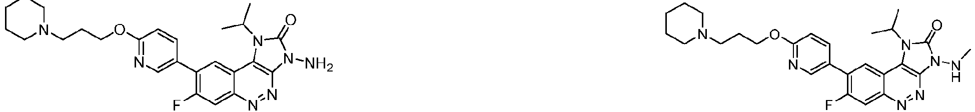 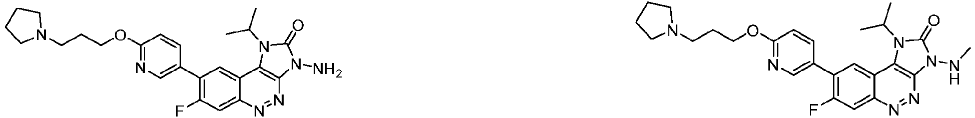 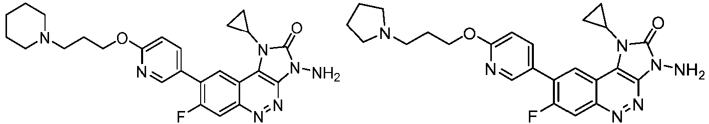 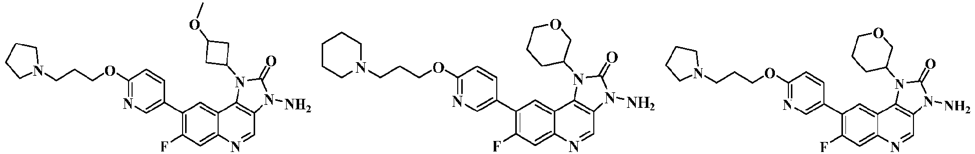 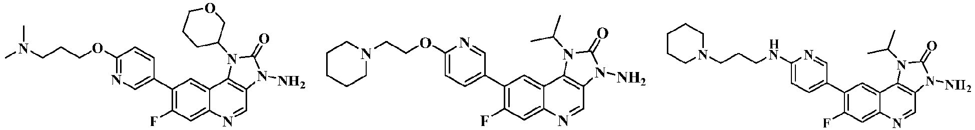 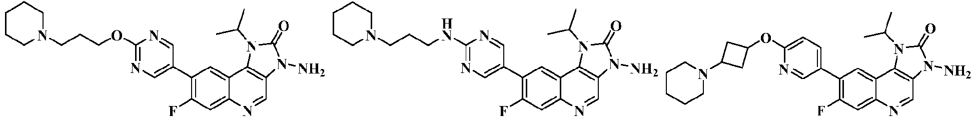 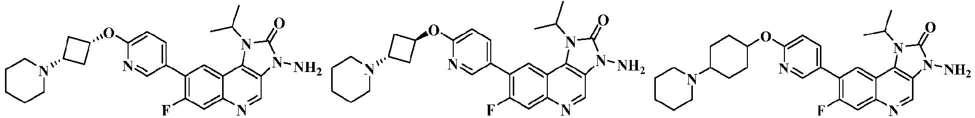 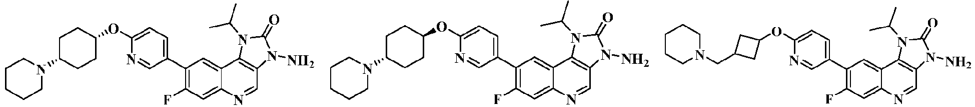 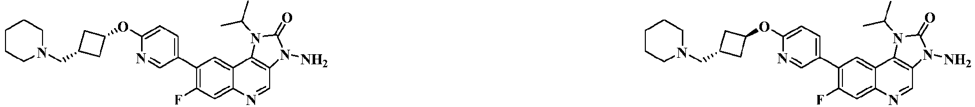 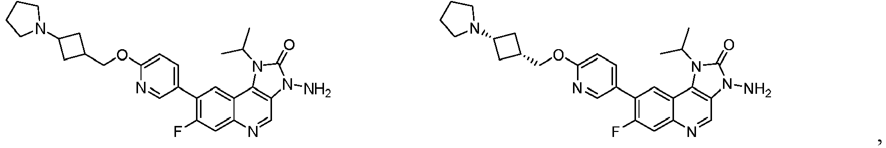 or

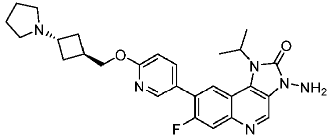

In another aspect, the present application further provides a pharmaceutical composition comprising the compound or the pharmaceutically acceptable salt thereof of the present application described above. In some embodiments, the pharmaceutical composition of the present application further comprises a pharmaceutically acceptable auxiliary material.

In another aspect, the present application further provides a method for treating various ATM-related diseases, which comprises administering to a mammal, preferably a human, in need of the treatment a therapeutically effective amount of the compound or the pharmaceutically acceptable salt thereof or the pharmaceutical composition thereof of the present application described above.

In another aspect, the present application further provides use of the compound or the pharmaceutically acceptable salt thereof or the pharmaceutical composition thereof of the present application described above in preparation of a medicament for treating various ATM-related diseases.

In another aspect, the present application further provides use of the compound or the pharmaceutically acceptable salt thereof or the pharmaceutical composition thereof of the present application described above for treating various ATM-related diseases.

In another aspect, the present application further provides the compound, the pharmaceutically acceptable salt thereof or the pharmaceutical composition thereof of the present application described above for use in treating various ATM-related diseases.

In some embodiments, the various ATM-related diseases are selected from the group consisting of tumors.

In some embodiments, the disease or tumor is selected from the group consisting of lung cancer and colon cancer.

### Technical Effects

The compounds of the present application have inhibitory activity against ATM kinase and exhibit selective inhibitory activity for ATM kinase relative to ATR and DNA-PK kinases. Meanwhile, the compounds of the present application have good inhibitory activity against CHK2 phosphorylation of NCI-H2228 cells, good *in vitro* and *in vivo* metabolism data, stable *in vitro* liver microsomal metabolism (species: human, monkey, dog, rat, and mouse), and low *in vitro* plasma protein binding rate; according to the *in vivo* drug metabolism data in mice, rats, or dogs, the compounds of the present application have high exposure in the brain and plasma of the mice and have high brain-to-blood ratio; according to *in vivo* drug effect study, the compounds of the present application can inhibit the growth of tumors.

### Definitions

Unless otherwise stated, the following terms used in the present application shall have the following meanings. A certain term, unless otherwise specifically defined, should not be considered uncertain or unclear, but construed according to its common meaning in the art. When referring to a trade name, it is intended to refer to its corresponding commercial product or its active ingredient.

When certain structural units or groups in the present application have a covalent bond that is not connected to a specific atom, it means that the covalent bond can be connected to any atom within that structural unit or group, as long as it adheres to the rules of valence bonding.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted and oxo is not possible on an aromatic group.

The term "optional" or "optionally" means that the subsequently described event or circumstance may, but does not necessarily, occur. The description includes instances where the event or circumstance occurs and instances where it does not. For example, ethyl "optionally" substituted with halogen means that the ethyl may be unsubstituted (CH₂CH₃), monosubstituted (for example, CH₂CH₂F), polysubstituted (for example, CHFCH₂F, CH₂CHF₂ and the like), or fully substituted (CF₂CF₃). It can be understood by those skilled in the art that for any groups comprising one or more substituents, no substitutions or substitution modes that are impossible to spatially exist and/or synthesize will be introduced.

"One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms. For example, C₁₋₃ means that the group may have 1 carbon atom, 2 carbon atoms, or 3 carbon atoms.

When any variable (e.g., R) occurs once or more times in the constitution or structure of a compound, the definition of the variable in each case is independent. Therefore, for example, if a group is substituted with 2 R, the definition of each R is independent.

When the number of connecting groups is 0, for example, -(CH₂)₀-, it means that the connecting group is a covalent bond.

When one of the variables is selected from a covalent bond, it means that the two groups connected to it are directly linked. For example, in A-L'-Z, where L' represents a covalent bond, it means that the structure is actually A-Z.

When a bond of a substituent is crosslinked to two atoms on a ring, the substituent can be bonded to any atoms on the ring. For example, structural unit 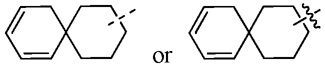 represents that substitution may occur at any one position of cyclohexyl or cyclohexadienyl. Specifically, for example, for 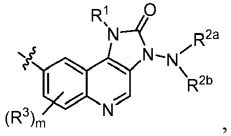 when m is 1, the position of R³ may be at the position illustrated in the following group: 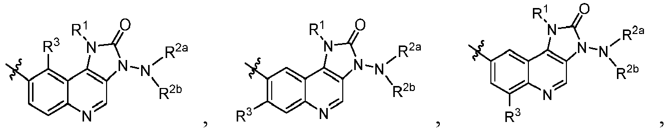 or

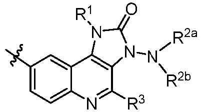

The term "halo-" or "halogen" refers to fluorine, chlorine, bromine, and iodine.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (for example, methyl, ethyl, n-propyl, isopropyl, *n*-butyl, isobutyl, *sec*-butyl, *tert*-butyl, n-pentyl, 1-methylbutyl, 2-methylbutyl, 3-methylbutyl, neopentyl, hexyl, 2-methylpentyl, and the like). The alkyl moieties (i.e., alkyl) of alkoxy, alkylamino, dialkylamino, alkylhydrazino, alkylsulfonyl, and alkylthio are similarly defined as above. As another example, the term "C₁₋₃ alkyl" refers to alkyl containing 1 to 3 carbon atoms (e.g., methyl, ethyl, propyl, and isopropyl).

The term "alkylene" refers to a divalent group formed by the removal of one hydrogen at any position of alkyl. For example, the term "C₁₋₆ alkyl" refers to alkylene containing 1 to 6 carbon atoms; the term "C₁₋₄ alkyl" refers to alkylene containing 1 to 4 carbon atoms, including but not limited to -CH₂-, -CH₂CH₂-, -CH₂CH₂CH₂-, or -CH₂CH₂CH₂CH₂-.

The term "alkoxy" refers to -O-alkyl.

The term "cycloalkyl" refers to a carbon ring group that is fully saturated and may exist as a monocyclic ring, a bridged ring, or a spiro ring; it may be a monovalent or a polyvalent, e.g., divalent, group, depending on its position in the compound. Unless otherwise specified, the carbon ring is typically a 3-15 membered ring, a 3-12 membered ring, a 3-10 membered ring, a 3-8 membered ring, a 4-6 membered ring, or a 5-8 membered ring. Non-limiting examples of cycloalkyl include, but are not limited to, cyclopropyl, cyclobutyl, cyclopentyl, cyclohexyl, norbornyl(bicyclo[2.2.1]heptyl), bicyclo[2.2.2]octyl, adamantyl, dicyclo[1.1.1]pentan-1-yl, and the like. For example, C₃₋₄ cycloalkyl includes cyclopropyl and cyclobutyl.

The term "heterocycloalkyl" refers to a cyclic group that is fully saturated and may exist as a monocyclic ring, a bridged ring (including a fused ring), or a spiro ring; it may be a monovalent or a polyvalent, e.g., divalent, group, depending on its position in the compound. Unless otherwise specified, the heterocycle is usually a 3-15 membered ring, a 3-12 membered, a 3-10 membered ring, a 3-9 membered ring, a 3-7 membered ring, a 4-6 membered ring, or a 5-6 membered ring containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiapyranyl, morpholinyl, piperazinyl, 1,4-oxathianyl, 1,4-dioxanyl, sulfomorpholinyl, 1,3-dithianyl, and 1,4-dithianyl. Examples of 7-membered heterocycloalkyl include, but are not limited to, azepanyl, oxepanyl, and thiepanyl. Monocyclic heterocycloalkyl having 5 or 6 ring atoms is preferred.

The term "aryl" refers to an all-carbon aromatic monocyclic or fused polycyclic group having a conjugated π-electron system. For example, aryl may have 6-20 carbon atoms, 6-14 carbon atoms, or 6-12 carbon atoms. Non-limiting examples of aryl include, but are not limited to, phenyl, naphthyl, anthryl, 1,2,3,4-tetrahydronaphthalene, and the like.

The term "heteroaryl" refers to a group having a monocyclic or fused polycyclic ring system that comprises at least one ring atom selected from the group consisting of N, O, and S, such as 1, 2, 3, or 4 ring atoms selected from the group consisting of N, O, and S, with the remaining ring atoms being C, and that has at least one aromatic ring. It may be a monovalent or a polyvalent, e.g., divalent, group, depending on its position in the compound. Preferably, heteroaryl has a single 5-8 membered or 5-6 membered ring, or has a plurality of fused rings comprising 6 to 14 ring atoms, in particular 6 to 10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, benzimidazolyl, imidazopyridinyl, indolyl, isoindolyl, and the like. For example, in the present application, 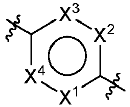 indicates that the ring system is heteroaryl.

The group or structural fragment, such as L and specific options thereof, in the present application, is correspondingly linked to the left side group and the right side group of the group or fragment in the general formula respectively in a left-to-right reading order. For example, when L is selected from 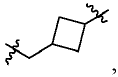 the left side of L is linked to the fragment 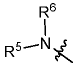 corresponding to the left side in the general formula and the right side is linked to the right side group X according to the left-to-right reading order, and the formed fragment is 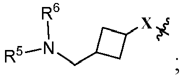 when L is selected from 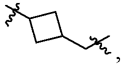 the left side of L is linked to the fragment 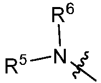 corresponding to the left side in the general formula and the right side of L is linked to the group X corresponding to the right side in the general formula according to the left-to-right reading order, and the formed fragment is

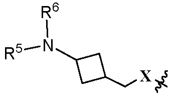

The other groups are as described above.

The term "treat", "treating", or "treatment" refers to administering the compound, the pharmaceutically acceptable salt thereof, the pharmaceutical composition thereof, or formulation described herein to ameliorate or eliminate a disease or one or more symptoms associated with the disease, and includes:
(i) inhibiting a disease or disease state, i.e., arresting its progression; and
(ii) alleviating a disease or disease state, i.e., causing its regression.

The term "prevent", "preventing", or "prevention" refers to administering the compound, the pharmaceutically acceptable salt thereof, the pharmaceutical composition thereof, or formulation described herein to prevent a disease or one or more symptoms associated with the disease, and includes: preventing the occurrence of the disease or disease state in a mammal, particularly when such a mammal is predisposed to the disease state but has not yet been diagnosed as having it.

The term "therapeutically effective amount" refers to an amount of the compound disclosed herein for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable auxiliary material. The pharmaceutical composition is intended to facilitate the administration of the compound of the present application to an organic entity.

The term "pharmaceutically acceptable auxiliary material" refers to those that do not have a significant irritating effect on an organic entity and do not impair the biological activity and properties of the active compound. Suitable auxiliary materials are well known to those skilled in the art, for example, carbohydrate, wax, water-soluble and/or water-swellable polymers, hydrophilic or hydrophobic material, gelatin, oil, solvent, or water.

The word "comprise" and variations thereof such as "comprises" or "comprising" will be understood in an open, non-exclusive sense, i.e., "including but not limited to".

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer is an imidazole moiety where a proton can transfer between two ring nitrogen atoms. A valence tautomer includes the interconversion via recombination of some bonding electrons.

The present application also comprises isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, and the like.

Certain isotopically labeled compounds of the present application (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dose) resulting from greater metabolic stability and hence may be preferred in some circumstances in which deuterium substitution may be partial or complete, wherein partial deuterium substitution refers to substitution of at least one hydrogen with at least one deuterium, and all such forms of the compounds are included within the scope of the present application.

The compound of the present application can be asymmetrical, for example, having one or more stereoisomers. Unless otherwise stated, all stereoisomers are included, for example, enantiomers and diastereoisomers. The compound with asymmetrical carbon atoms of the present application can be separated in an optically pure form or in a racemic form. The optically pure form can be separated from a racemic mixture or can be synthesized using a chiral raw material or a chiral reagent.

The pharmaceutical composition of the present application can be prepared by combining the compound of the present application with a suitable pharmaceutically acceptable auxiliary material, and can be formulated, for example, into a solid, semisolid, liquid or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present application can be manufactured using methods well known in the art, such as conventional mixing, dissolving, granulating, dragee-making, levigating, emulsifying, lyophilizing, and the like.

In some embodiments, the pharmaceutical composition is in an oral form. For oral administration, the pharmaceutical composition can be formulated by mixing the active compounds with pharmaceutically acceptable auxiliary materials well known in the art. These auxiliary materials enable the compounds of the present application to be formulated into tablets, pills, pastilles, dragees, capsules, liquids, gels, slurries, suspensions, and the like for oral administration to a patient. A solid oral composition can be prepared by conventional mixing, filling, or tableting. For example, it can be obtained by the following method: mixing the active compounds with solid auxiliary materials, optionally grinding the resulting mixture, adding additional suitable auxiliary materials if desired, and processing the mixture into granules to get the core parts of tablets or dragees. Suitable auxiliary materials include, but are not limited to: binders, diluents, disintegrants, lubricants, glidants, sweeteners, flavoring agents, or the like.

The pharmaceutical composition may also be suitable for parenteral administration, such as a sterile solution, a suspension, or a lyophilized product in a suitable unit dosage form.

Therapeutic dose of the compound of the present application may be determined by, for example, the specific use of a treatment, the route of administration of the compound, the health and condition of a patient, and the judgment of a prescribing physician. The proportion or concentration of the compound of the present application in a pharmaceutical composition may not be constant and depends on a variety of factors including dose, chemical properties (e.g., hydrophobicity), and route of administration. For example, the compound of the present application may be provided for parenteral administration by a physiological buffered aqueous solution containing about 0.1-10%w/v of the compound. Certain typical doses range from about 1 µg/kg body weight/day to about 1 g/kg body weight/day. In certain embodiments, the dose ranges from about 0.01 mg/kg body weight/day to about 100 mg/kg body weight/day. The dose is likely to depend on such variables as the type and degree of progression of the disease or disorder, the general health condition of a particular patient, the relative biological potency of the compound selected, the excipient formulation, and its route of administration. Effective doses can be extrapolated from dose-response curves derived from *in vitro* or animal model test systems.

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protective groups for reactive functional groups (e.g., amino in the present application). For example, reference may be made to Greene's Protective Groups in Organic Synthesis (4th Ed.) Hoboken, New Jersey: John Wiley & Sons, Inc.

In some embodiments, the compounds of general formulas of the present application may be prepared by those skilled in the art of organic synthesis using general or conventional methods in the art by the following routes: 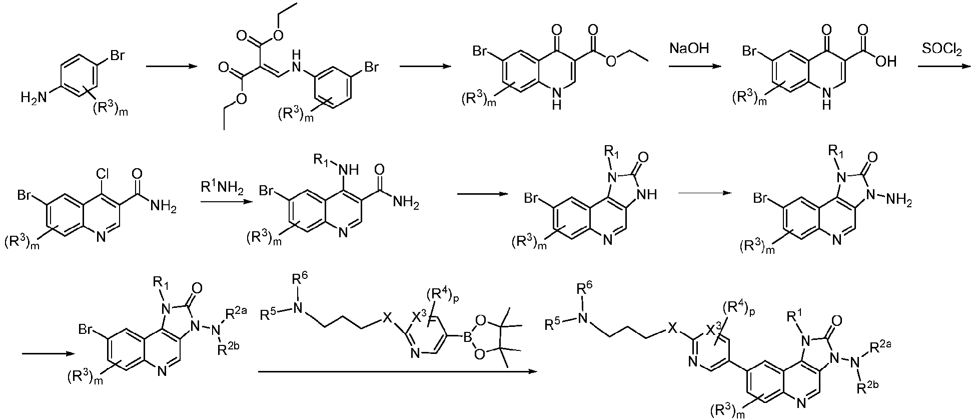 wherein, R¹, R^{2a}, R^{2b}, R³, R⁴, R⁵, R⁶, X, X³, m, and p are as described above.

### The following abbreviations are used in the present application:

DMF represents *N*,*N*-dimethylformamide; THF represents tetrahydrofuran; EtOH represents ethanol;

DIPEA represents *N,N*-diisopropylethylamine; DCM represents dichloromethane; EA represents ethyl acetate; MeOH represents methanol; DBU represents 1,8-diazabicyclo[5.4.0]undec-7-ene;

TBAB represents tetrabutylammonium bromide; DMA represents *N*,*N*-dimethylacetamide; CDI represents *N*,*N*-carbonyldiimidazole.

For clarity, the present application is further described with the following examples, which are, however, not intended to limit the scope of the present application. All reagents used in the present application are commercially available and can be used without further purification.

### DETAILED DESCRIPTION

### Preparation Example A-3

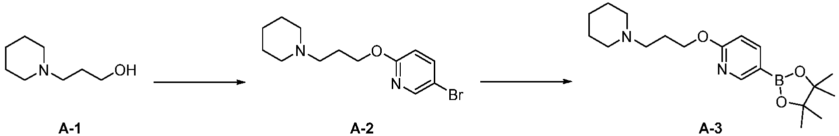

### Step A: synthesis of compound A-2

**A-1** (10.9 g) and THF (150 mL) were added successively to a single-necked flask, and then 60% (purity, by mass) sodium hydride (5.8 g) was added under N₂ atmosphere. The mixture was stirred for 10 min at 50 °C, and then cooled to room temperature. 5-bromo-2-fluoropyridine (10 g) was then added thereto, and the mixture was stirred at 50 °C for 2 h. After the reaction was completed, the reaction solution was poured slowly into an icy saturated ammonium chloride solution (100 mL) to quench the reaction. Dichloromethane (150 mL × 3) was added for extraction. The organic phases were combined, washed with saturated sodium chloride (150 mL), dried over anhydrous sodium sulfate, filtered under vacuum, and concentrated under reduced pressure to give **A-2** (20 g). MS (ESI+, [M+H]⁺) *m*/*z:* 298.97.

### Step B: synthesis of compound A-3

**A-2** (10.9 g), THF (250 mL), isopropanol pinacol borate (13.74 g) were added successively to a three-necked flask, and then the mixture was cooled to -78 °C under N₂ atmosphere. A 2.5 M solution of *n*-butyllithium in tetrahydrofuran (31 mL) was added dropwise and slowly. After the addition was completed, the mixture was stirred for 1 h, then allowed to return to room temperature, and stirred overnight. After the reaction was completed, the reaction solution was poured slowly into an icy saturated ammonium chloride solution (100 mL). EA (200 mL × 4) was added for extraction. The organic phase was washed with saturated sodium chloride (200 mL), dried over anhydrous sodium sulfate, filtered under vacuum, and concentrated under reduced pressure to remove the solvent, thus giving **A-3** (19 g). MS (ESI+, [M+H]⁺) *m*/*z:* 347.17.

### Preparation Example A-6

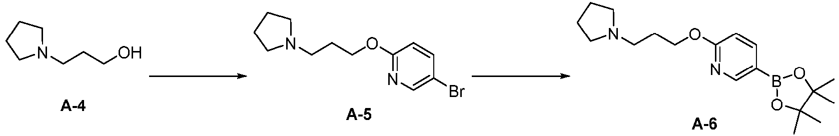

### Step A: synthesis of compound A-5

Referring to the synthesis of Preparation Example A-3, in the preparation step of A-2, A-1 was replaced with A-4 to give compound A-5. MS (ESI+, [M+H]⁺) *m*/*z:* 284.94.

### Step B: synthesis of compound A-6

Referring to the synthesis of Preparation Example A-3, in the preparation step of A-3, A-2 was replaced with A-5 to give compound A-6. MS (ESI+, [M+H]⁺) *m*/*z:* 333.09.

### Preparation Example A-9

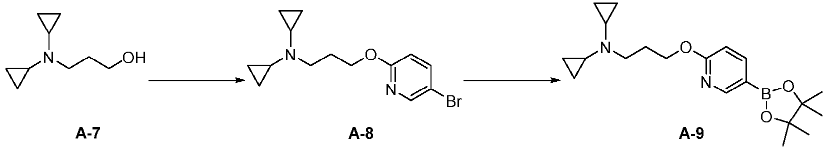

### Step A: synthesis of compound A-8

Referring to the synthesis of Preparation Example A-3, in the preparation step of A-2, A-1 was replaced with A-7 to give compound A-8. MS (ESI+, [M+H]⁺) *m*/*z:* 310.87.

### Step B: synthesis of compound A-9

Referring to the synthesis of Preparation Example A-3, in the preparation step of A-3, A-2 was replaced with A-8 to give compound A-9. MS (ESI+, [M+H]⁺) *m*/*z:* 359.12.

### Preparation Example A-12

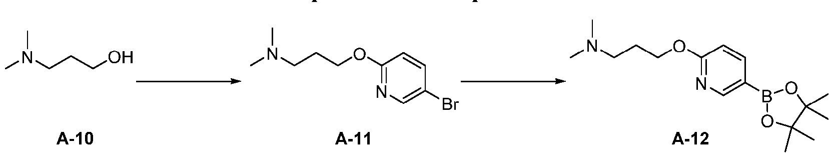

### Step A: synthesis of compound A-11

Referring to the synthesis of Preparation Example A-3, in the preparation step of A-2, A-1 was replaced with A-10 to give compound A-11. MS (ESI+, [M+H]⁺) *m*/*z:* 258.90.

### Step B: synthesis of compound A-12

Referring to the synthesis of Preparation Example A-3, in the preparation step of A-3, A-2 was replaced with A-11 to give compound A-12. MS (ESI+, [M+H]⁺) *m*/*z:* 307.23.

### Example 1

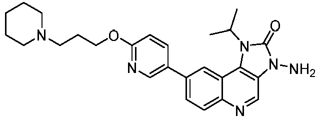

### Reaction process:

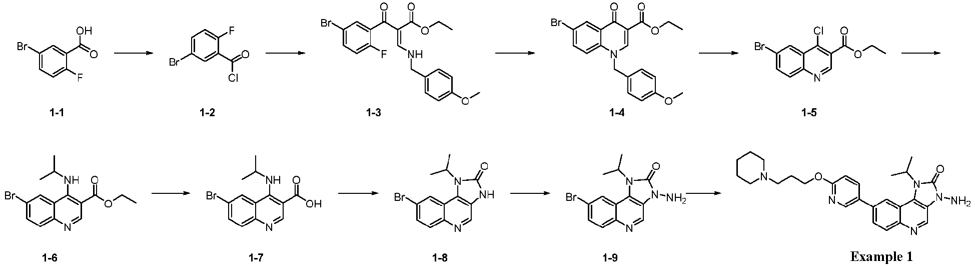

### Step A: synthesis of compound 1-2

**1-1** (10 g), dichloromethane (100 mL), and DMF (0.67 g) were added successively to a single-necked flask, and then oxalyl chloride (8.69 g) was added under an ice bath. The resulting mixture was stirred at 0 °C for 1 h. After the reaction was completed, the reaction solution was concentrated under reduced pressure to give intermediate **1-2** (10 g), which was used directly in the next step.

### Step B: synthesis of compound 1-3

**1-2** (10 g), toluene (100 mL), and DIPEA (8.16 mL) were added successively to a single-necked flask, and then ethyl (*E*)-3-(dimethylamino)acrylate (6.63 g) was added dropwise under N₂ atmosphere. The resulting solution was stirred at 70 °C for 17 h and then cooled to room temperature, and 4-methoxybenzylamine (5.84 g) was added. The mixture was stirred at room temperature for 3 h. After the reaction was completed, the reaction solution was diluted with DCM (300 mL) and washed successively with water (3 × 200 mL) and saturated brine (200 mL). The organic layer was dried over anhydrous sodium sulfate, filtered, and concentrated to give intermediate **1-3** (18 g). MS (ESI+, [M+H]⁺) *m*/*z:* 436.02.

### Step C: synthesis of compound 1-4

**1-3** (18 g) and acetone (200 mL) were added successively to a single-necked flask, and then DBU (6.28 g) was added at 10 °C. The mixture was stirred at room temperature for 16 h under N₂ atmosphere. After the reaction was completed, the reaction system was filtered, and the filter cake was collected, washed with petroleum ether (3 × 5 mL), and dried under vacuum and under reduced pressure to give intermediate **1-4** (8.0 g). MS (ESI+, [M+H]⁺) *m*/*z:* 416.06. ¹H NMR (500 MHz, DMSO-*d*₆) δ 8.92 (s, 1H), 8.30 (d, *J =* 2.4 Hz, 1H), 7.85 (dd, *J =* 9.1, 2.5 Hz, 1H), 7.67 (d, *J =* 9.1 Hz, 1H), 7.26 - 7.17 (m, 2H), 6.95 - 6.88 (m, 2H), 5.59 (s, 2H), 4.25 (q, *J =* 7.1 Hz, 2H), 3.71 (s, 3H), 1.29 (t, *J* = 7.1 Hz, 3H).

### Step D: synthesis of compound 1-5

**1-4** (8.3 g), thionyl chloride (47.4 g), and DMF (0.029 g) were added successively to a single-necked flask, and the mixture was stirred at 70 °C for 3 h under N₂ atmosphere. After the reaction was completed, the reaction solution was concentrated to dryness. The crude product was purified by slurrying with n-hexane (20 mL) and then filtered under vacuum to give intermediate **1-5** (5.3 g). MS (ESI+, [M+H]⁺) *m*/*z*: 313.85.

### Step E: synthesis of compound 1-6

**1-5** (2.5 g), DMA (30 mL), isopropylamine (0.705 g), and DIPEA (2.05 g) were added successively to a single-necked flask, and the mixture was stirred at 100 °C for 4 h under N₂ atmosphere. After the reaction was completed, the reaction solution was poured into ice water (150 mL). The filter cake was collected by filtration, washed with water (10 mL × 3), and dried under vacuum to give intermediate **1-6** (2.7 g). MS (ESI+, [M+H]⁺) *m*/*z:* 336.97.

### Step F: synthesis of compound 1-7

**1-6** (2.7 g), THF (30 mL), water (10 mL), and NaOH (1.6 g) were added successively to a single-necked flask, and the mixture was stirred at 60 °C for 5 h. After the reaction was completed, the reaction solution was concentrated, diluted with water (50 mL) and adjusted to pH 2-3 with 2 M hydrochloric acid. The filter cake was collected by filtration, washed with water (5 mL × 3), and dried under vacuum to give intermediate **1-7** (2.1 g.). MS (ESI+, [M+H]⁺) *m*/*z:* 308.88.

### Step G: synthesis of compound 1-8

**1-7** (1.6 g), DMF (15 mL), and triethylamine (1.571 g) were added successively to a single-necked flask, and the mixture was stirred at room temperature for 30 min under N₂ atmosphere. Diphenylphosphoryl azide (1.709 g) was then added, and the reaction solution was stirred at room temperature for 30 min and then stirred at 60 °C for 3 h. After the reaction was completed, the reaction solution was poured into ice water (100 mL) and filtered, and the filter cake was collected, washed with water (5 mL × 3), and dried under vacuum to give intermediate **1-8**(1.5 g). MS (ESI+, [M+H]⁺) *m*/*z*: 305.88.

### Step H: synthesis of compound 1-9

**1-8** (0.10 g), DCM (5 mL), water (2.5 mL), TBAB (10.53 mg), NaOH (21.6 mg), and 2,4-dinitrophenylhydroxylamine (98 mg) were added successively to a single-necked flask, and the mixture was stirred at room temperature for 24 h under nitrogen atmosphere. After the reaction was completed, the reaction solution was concentrated under reduced pressure to remove part of the solvent. The filter cake was collected by filtration, slurried with 5 mL of acetonitrile, and dried under vacuum to give intermediate **1-9** (95 mg). MS (ESI+, [M+H]⁺) *m*/*z:* 320.86.

### Step I: synthesis of Example 1

**1-9** (95 mg), 1,4-dioxane (5 mL), water (2 mL), **A-3** (166 mg), potassium carbonate (123 mg), 3-(di-*tert*-butylphosphino)propane-1-sulfonic acid (1.587 mg), and sodium tetrachloropalladate(II) (0.870 mg) were added successively to a single-necked flask, and the mixture was stirred at 80 °C for 1 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 1** (32.0 mg). MS (ESI+, [M+H]⁺) *m*/*z:* 461.27. ¹H NMR (500 MHz, DMSO-*d*6) δ 8.80 (s, 1H), 8.65 (d, *J =* 2.6 Hz, 1H), 8.39 (d, *J =* 2.0 Hz, 1H), 8.18 (dd, *J =* 8.6, 2.6 Hz, 1H), 8.13 (d, *J =* 8.8 Hz, 1H), 7.92 (dd, *J =* 8.8, 1.9 Hz, 1H), 6.97 (d, *J =* 8.6 Hz, 1H), 5.55 (s, 2H), 5.36 (p, *J =* 6.8 Hz, 1H), 4.35 (t, *J =* 6.6 Hz, 2H), 2.43 - 2.23 (m, 6H), 1.91 (q, *J =* 6.8 Hz, 2H), 1.68 (d, *J=* 6.7 Hz, 6H), 1.49 (p, *J=* 5.5 Hz, 4H), 1.38 (q, *J=* 5.9 Hz, 2H).

### Example 2

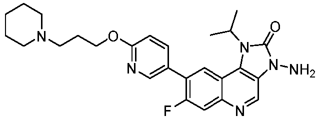

### Reaction process:

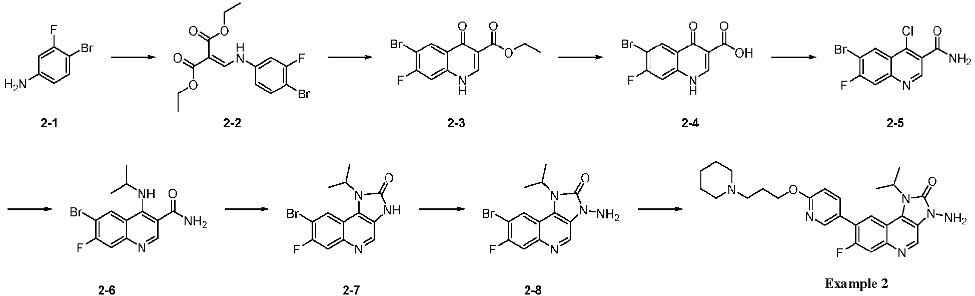

### Step A: synthesis of compound 2-2

**2-1** (50 g), EtOH (500 mL), and diethyl 1,3-2-(ethoxymethylene)malonate (62.6 g) were added successively to a three-necked flask, and the mixture was heated to 80 °C and left to react for 4 h under N₂ atmosphere. After the reaction was completed, the reaction solution was concentrated under reduced pressure to remove the solvent. After being left to stand, the reaction solution was filtered under vacuum, and the filter cake was washed with petroleum ether (20 mL × 5) and dried under reduced pressure to give intermediate **2-2** (90 g). MS (ESI+, [M+H]⁺) *m*/*z:* 360.00.

### Step B: synthesis of compound 2-3

**2-2** (90 g) and diphenyl ether (600 g) were added successively to a three-necked flask, and the mixture was heated to 240 °C and left to react for 8 h under N₂ atmosphere. After the reaction was completed, the reaction solution was cooled to room temperature and filtered, and the filter cake was washed with petroleum ether (20 mL × 5) and dried under reduced pressure to give intermediate **2-3** (40 g). MS (ESI+, [M+H]⁺) *m*/*z:* 313.82.

### Step C: synthesis of compound 2-4

**2-3** (40 g), EtOH (400 mL), water (80 mL), and NaOH (25.5 g) were added successively to a single-necked flask, and the mixture was stirred at 75 °C for 2 h under N₂ atmosphere. After the reaction was completed, the reaction solution was cooled under an ice bath, adjusted to pH 4-5 with 2 N hydrochloric acid, and filtered, and the filter cake was washed with water (20 mL × 3) and dried under vacuum to give intermediate **2-4** (35 g). MS (ESI+, [M+H]⁺) *m*/*z:* 285.82.

### Step D: synthesis of compound 2-5

**2-4** (31.6 g), thionyl chloride (120 mL), and DMF (0.16 g) were added successively to a single-necked flask, and the mixture was heated to reflux and left to react for 2 h under N₂ atmosphere. After the reaction was completed, the reaction solution was cooled and concentrated under reduced pressure, and the concentrate was added to a solution of ammonium hydroxide (63.8 mL) at 0 °C. The mixture was stirred vigorously at room temperature for 15 min and filtered, and the filter cake was washed with water (15 mL × 3) and dried under vacuum to give intermediate **2-5** (30 g). MS (ESI+, [M+H]⁺) *m*/*z:* 302.86.

### Step E: synthesis of compound 2-6

**2-5** (3.0 g), potassium carbonate (2.73 g), acetonitrile (40 mL), and isopropylamine (0.88 g) were added successively to a single-necked flask, and the mixture was stirred at 95 °C for 4 h under N₂ atmosphere. After the reaction was completed, the reaction solution was cooled, and 150 mL of water was added. The reaction solution was filtered, and the filter cake was washed with water (5 mL × 4) and dried under vacuum to give intermediate **2-6** (2.8 g). MS (ESI+, [M+H]⁺) *m*/*z:* 325.89

### Step F: synthesis of compound 2-7

**2-6** (2.8 g), DBU (2.61 g), and methanol (30 mL) were added successively to a single-necked flask, and then 1,3,5-trichloro-1,3,5-triazine-2,4,6-trione (1.00 g) was added at 5 °C. The mixture was stirred at room temperature for 1 h under N₂ atmosphere. After the reaction was completed, the reaction solution was filtered directly, and the filter cake was washed with water (10 mL × 2) and dried under vacuum to give intermediate **2-7** (2.4 g). MS (ESI+, [M+H]⁺) *m*/*z:* 323.86.

### Step G: synthesis of compound 2-8

**2-7** (2.4 g), DCM (30 mL), water (15 mL), TBAB (0.24 g), 2,4-dinitrophenylhydroxylamine (2.21 g), and NaOH (0.59 g) were added successively to a single-necked flask, and the mixture was stirred at room temperature for 24 h under N₂ atmosphere. After the reaction was completed, the reaction solution was concentrated to remove most of the solvent, and then filtered. The filter cake was slurried with 10 mL of acetonitrile and dried under vacuum to give intermediate **2-8** (1.8 g). MS (ESI+, [M+H]⁺) *m*/*z*: 338.88.

### Step H: synthesis of Example 2

**2-8** (150 mg), 1,4-dioxane (5 mL), water (2 mL), **A-3** (249 mg), potassium carbonate (183 mg), 3-(di-*tert*-butylphosphino)propane-1-sulfonic acid (2.37 mg), and sodium tetrachloropalladate(II) (1.30 mg) were added successively to a single-necked flask, and the mixture was stirred at 80 °C for 1 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was slurried with acetonitrile (5 mL) to give **Example 2** (70.0 mg). MS (ESI+, [M+H]⁺) *m*/*z:* 479.30. ¹H NMR (500 MHz, DMSO-d6) δ 8.82 (s, 1H), 8.53 - 8.47 (m, 1H), 8.32 (d, *J* = 8.2 Hz, 1H), 8.04 (dt, *J =* 8.7, 2.3 Hz, 1H), 7.91 (d, *J =* 12.0 Hz, 1H), 6.98 (d, *J =* 8.6 Hz, 1H), 5.55 (s, 2H), 5.35 - 5.23 (m, 1H), 4.36 (t, *J =* 6.6 Hz, 2H), 2.39 (t, *J =* 7.2 Hz, 2H), 2.33 (s, 4H), 1.96 - 1.85 (m, 2H), 1.64 (d, *J =* 6.7 Hz, 6H), 1.49 (p, *J =* 5.6 Hz, 4H), 1.38 (d, *J =* 6.1 Hz, 2H).

### Example 3

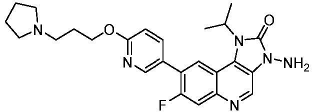

### Reaction process:

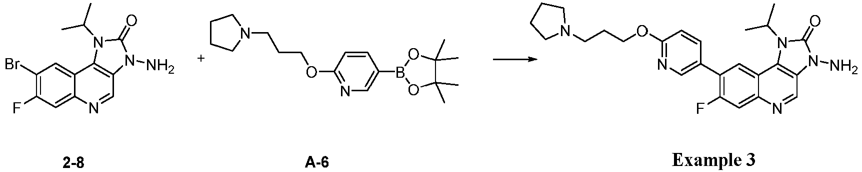

**2-8** (100 mg), 1,4-dioxane (5 mL), water (2 mL), **A-6** (196 mg), potassium carbonate (122 mg), 3-(di-*tert*-butylphosphino)propane-1-sulfonic acid (1.58 mg), and sodium tetrachloropalladate(II) (0.87 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was slurried with acetonitrile (5 mL) to give **Example 3** (25 mg). MS (ESI+, [M+H]⁺) *m*/*z:* 465.25, ¹H NMR (500 MHz, DMSO-d6) δ 8.82 (s, 1H), 8.52 - 8.48 (m, 1H), 8.32 (d, *J =* 8.2 Hz, 1H), 8.04 (dt, *J =* 8.6, 2.3 Hz, 1H), 7.91 (d, *J =* 12.0 Hz, 1H), 6.99 (d, *J =* 8.6 Hz, 1H), 5.55 (s, 2H), 5.29 (p, *J =* 6.8 Hz, 1H), 4.38 (t, *J =* 6.6 Hz, 2H), 2.55 (t, *J =* 7.1 Hz, 2H), 2.48 - 2.43 (m, 4H), 1.97 - 1.89 (m, 2H), 1.71 - 1.67 (m, 4H), 1.65 (d, *J =* 6.8 Hz, 6H).

### Example 4

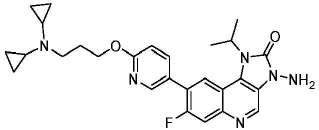

### Reaction process:

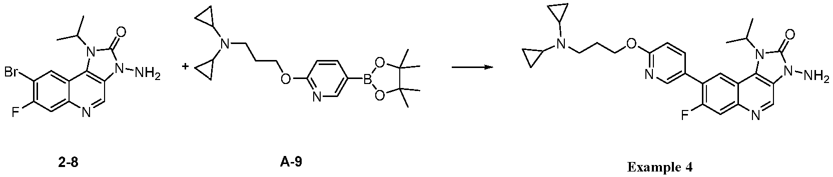

**2-8** (100 mg), 1,4-dioxane (5 mL), water (2 mL), **A-9** (211 mg), potassium carbonate (122 mg), 3-(di-*tert*-butylphosphino)propane-1-sulfonic acid (1.58 mg), and sodium tetrachloropalladate(II) (0.87 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 4** (51.6 mg). MS (ESI+, [M+H]⁺) *m*/*z:* 491.27, ¹H NMR (500 MHz, DMSO-*d*6) δ 8.82 (s, 1H), 8.50 (s, 1H), 8.32 (d, *J =* 8.1 Hz, 1H), 8.04 (dt, *J =* 8.8, 2.3 Hz, 1H), 7.91 (d, *J =* 12.0 Hz, 1H), 6.99 (d, *J =* 8.6 Hz, 1H), 5.55 (s, 2H), 5.35 - 5.21 (m, 1H), 4.34 (t, *J =* 6.5 Hz, 2H), 2.82 (t, *J =* 7.4 Hz, 2H), 2.03 (p, *J =* 6.8 Hz, 2H), 1.90 (tt, *J =* 6.8, 3.8 Hz, 2H), 1.64 (d, *J =* 6.7 Hz, 6H), 0.42 (dt, *J* = 6.3, 3.1 Hz, 4H), 0.32 (p, *J =* 4.2 Hz, 4H).

### Example 5

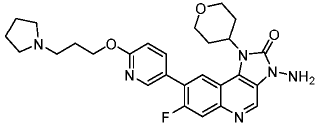

### Reaction process:

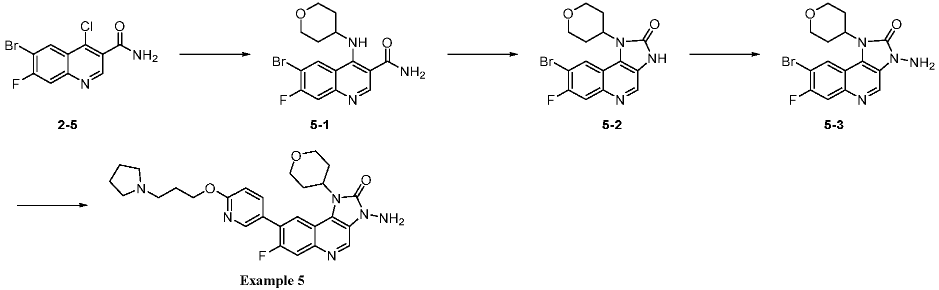

### Step A: synthesis of compound 5-1

**2-5** (1.0 g), DMF (10 mL), DIPEA (1.28 g), and 4-aminotetrahydropyran (0.40 g) were added successively to a single-necked flask, and the mixture was heated to 90 °C and left to react for 1 h under N₂ atmosphere. After the reaction was completed, the reaction solution was poured into 100 mL of water and filtered, and the filter cake was washed with water (10 mL × 2) and dried under vacuum to give intermediate **5-1** (1.1 g). MS (ESI+, [M+H]⁺) *m*/*z:* 368.95. ¹H NMR (500 MHz, DMSO-*d*₆) *δ* 8.71 (d, *J =* 7.5 Hz, 1H), 8.58 (s, 1H), 8.13 (s, 1H), 8.02 (d, *J =* 8.5 Hz, 1H), 7.71 (d, *J* = 10.0 Hz, 1H), 7.62 (s, 1H), 4.08 - 3.96 (m, 1H), 3.88 (d, *J =* 11.5 Hz, 2H), 3.36 (d, *J =* 1.3 Hz, 1H), 3.32 (s, 1H), 1.91 (d, *J =* 12.3 Hz, 2H), 1.60 (td, *J =* 14.2, 4.0 Hz, 2H).

### Step B: synthesis of compound 5-2

**5-1** (1.0 g), MeOH (30 mL), DBU (0.83 g), and 1,3,5-trichloro-1,3,5-triazine-2,4,6-trione (0.31 g) were added successively to a single-necked flask, and the suspension was stirred at room temperature for 1 h under N₂ atmosphere. After the reaction was completed, the reaction solution was filtered directly, and the filter cake was washed with water (10 mL × 2) and dried under vacuum to give intermediate **5-2** (0.85 g). MS (ESI+, [M+H]⁺) *m*/*z:* 365.92. ¹H NMR (500 MHz, DMSO-*d*₆) *δ* 8.67 (s, 1H), 8.54 (d, *J =* 7.3 Hz, 1H), 7.89 (d, *J =* 10.2 Hz, 1H), 4.98 - 4.83 (m, 1H), 4.03 (dd, *J =* 11.3, 4.2 Hz, 2H), 3.58 (t, *J =* 11.3 Hz, 3H), 2.76 - 2.62 (m, 2H), 1.86 - 1.77 (m, 2H).

### Step C: synthesis of compound 5-3

**5-2** (0.65 g), THF (10 mL), sodium hydride (60% purity, by mass, 0.11 g), and 2,4-dinitrophenylhydroxylamine (0.42 g) were added successively to a single-necked flask, and the mixture was stirred at room temperature for 24 h under N₂ atmosphere. After the reaction was completed, 50 mL of water was added, and EA (50 mL × 3) was added for extraction. The organic phases were combined, washed with saturated brine (50 mL), dried over anhydrous sodium sulfate, filtered, and concentrated under reduced pressure to dryness, and the resulting crude product was subjected to silica gel column chromatography (eluent: DCM/MeOH = 25/1) to give intermediate **5-3** (0.45 g). MS (ESI+, [M+H]⁺) *m*/*z:* 381.95.

### Step D: synthesis of Example 5

**5-3** (200 mg), 1,4-dioxane (20 mL), **A-6** (227 mg), potassium carbonate (218 mg) dissolved in 0.5 mL of water, 3-(di-tert-butylphosphino)propane-1-sulfonic acid (2.8 mg) dissolved in 0.5 mL of water, and sodium tetrachloropalladate(II) (1.5 mg) were added successively to a single-necked flask, and the mixture was heated to 80 °C and left to react for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was subjected to silica gel column chromatography (eluent: DCM/MeOH = 10/1) to give **Example 5** (44 mg). MS (ESI+, [M+H]⁺) *m*/*z:* 507.28. ¹H NMR (500 MHz, DMSO-*d*₆) *δ* 8.84 (d, *J =* 1.7 Hz, 1H), 8.54 (t, *J =* 2.5 Hz, 1H), 8.34 (d, *J =* 8.1 Hz, 1H), 8.10 - 8.07 (m, 1H), 7.93 (dd, *J =* 12.1, 1.6 Hz, 1H), 7.00 (d, *J =* 8.2 Hz, 1H), 5.58 (s, 2H), 5.09 (ddt, *J =* 11.8, 8.2, 4.2 Hz, 1H), 4.40 - 4.37 (m, 2H), 4.03 (dd, *J=* 11.4, 4.6 Hz, 2H), 3.54 (t, *J =* 11.9 Hz, 2H), 2.74 - 2.66 (m, 2H), 2.54 (t, *J =* 7.2 Hz, 2H), 2.46 - 2.43 (m, 4H), 1.96 - 1.89 (m, 4H), 1.70 - 1.67 (m, 4H).

### Example 6

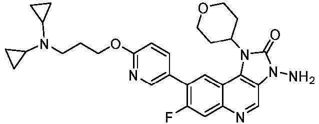

### Reaction process:

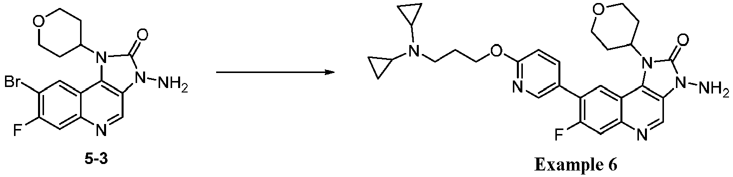

**5-3** (200 mg), 1,4-dioxane (20 mL), **A-9** (244 mg), potassium carbonate (218 mg) dissolved in 0.5 mL of water, 3-(di-tert-butylphosphino)propane-1-sulfonic acid (2.8 mg) dissolved in 0.5 mL of water, and sodium tetrachloropalladate(II) (1.5 mg) were added successively to a single-necked flask, and the mixture was heated to 80 °C and left to react for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was subjected to silica gel column chromatography (eluent: DCM/MeOH = 10/1) to give **Example 6** (94 mg). MS (ESI+, [M+H]⁺) *m*/*z:* 533.34. ¹H NMR (500 MHz, DMSO-*d*₆) *δ* 8.83 (s, 1H), 8.54 (s, 1H), 8.34 (d, *J =* 7.9 Hz, 1H), 8.08 (d, *J =* 7.0 Hz, 1H), 7.93 (d, *J =* 12.1 Hz, 1H), 7.00 (d, *J =* 8.6 Hz, 1H), 5.58 (s, 2H), 5.09 (t, *J =* 11.6 Hz, 1H), 4.38 (t, *J =* 6.5 Hz, 2H), 4.10 - 4.00 (m, 2H), 3.54 (t, *J =* 11.8 Hz, 2H), 2.70 (dt, *J =* 20.1, 10.0 Hz, 2H), 2.01 (qd, *J =* 11.9, 4.4 Hz, 2H), 2.08 - 2.01 (m, 4H), 1.89 (tt, *J =* 6.8, 3.8 Hz, 2H), 0.42 (dd, *J =* 6.6, 2.2Hz, 4H), 0.32 (dd, *J =* 3.7, 2.3Hz, 4H).

### Example 7

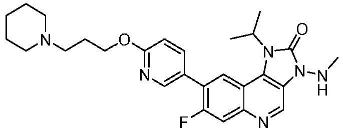

### Reaction process:

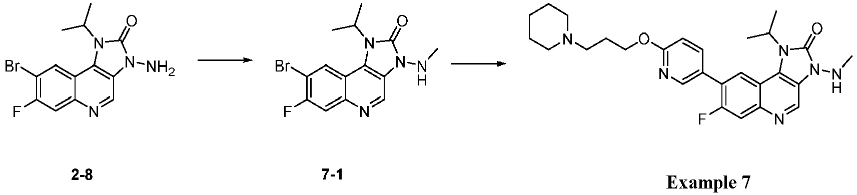

### Step A: synthesis of compound 7-1

**2-8** (100 mg), DMF (5 mL), NaOH (35.4 mg), and CH₃I (62.8 mg) were added successively to a single-necked flask, and the mixture was left to react at room temperature for 14 h under N₂ atmosphere. After the reaction was completed, 50 mL of water was added to the system. The mixture was filtered, and the filter cake was washed with water (5 mL × 2) and dried under reduced pressure to give intermediate **7-1** (100 mg). MS (ESI+, [M+H]⁺) *m*/*z:* 352.92.

### Step B: synthesis of Example 7

**7-1** (100 mg), 1,4-dioxane (5 mL), water (2 mL), **A-3** (159 mg), potassium carbonate (117 mg), 3-(di-*tert*-butylphosphino)propane-1-sulfonic acid (1.52 mg), and sodium tetrachloropalladate(II) (0.83 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 7** (24 mg). MS (ESI+, [M+H]⁺) *m*/*z:* 493.27. ¹H NMR (500 MHz, DMSO-*d*6) δ 8.81 (s, 1H), 8.50 (t, *J =* 1.8 Hz, 1H), 8.32 (d, *J =* 8.1 Hz, 1H), 8.04 (dt, *J =* 8.6, 2.3 Hz, 1H), 7.92 (d, *J =* 11.9 Hz, 1H), 6.98 (d, *J =* 8.6 Hz, 1H), 6.27 (d, *J =* 5.6 Hz, 1H), 5.28 (p, *J =* 6.8 Hz, 1H), 4.36 (t, *J =* 6.6 Hz, 2H), 2.77 (d, *J =* 5.4 Hz, 3H), 2.39 (t, *J =* 7.2 Hz, 2H), 2.38 - 2.28 (m, 4H), 1.95 - 1.86 (m, 2H), 1.65 (d, *J =* 6.7 Hz, 6H), 1.54 - 1.45 (m, 4H), 1.42 - 1.35 (m, 2H).

### Example 8

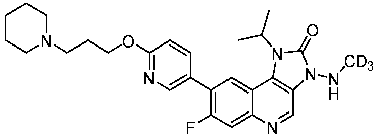

### Reaction process:

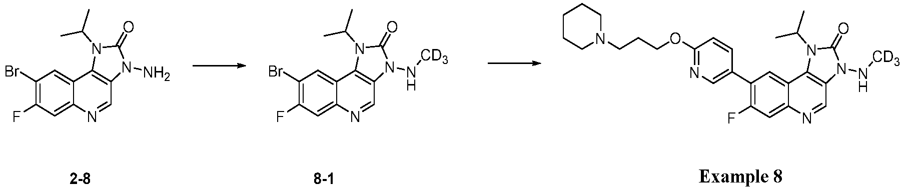

### Step A: synthesis of compound 8-1

**2-8** (50 mg), DMF (5 mL), NaOH (17.7 mg), and CD₃I (31.2 mg) were added successively to a single-necked flask, and the mixture was left to react at room temperature for 14 h under N₂ atmosphere. After the reaction was completed, 50 mL of water was added. The reaction solution was filtered, and the filter cake was washed with water (5 mL × 2) and dried under reduced pressure to give intermediate **8-1** (45 mg). MS (ESI+, [M+H]⁺) *m*/*z:* 356.01.

### Step B: synthesis of Example 8

**8-1** (45 mg), 1,4-dioxane (5 mL), water (2 mL), **A-3** (71.1 mg), potassium carbonate (52.4 mg), 3-(di-*tert*-butylphosphino)propane-1-sulfonic acid (0.68 mg), and sodium tetrachloropalladate(II) (0.37 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 8** (15 mg). MS (ESI+, [M+H]⁺) *m*/*z:* 496.29. ¹H NMR (500 MHz, DMSO-*d*6) δ 8.82 (s, 1H), 8.58 - 8.50 (m, 1H), 8.32 (d, *J =* 8.1 Hz, 1H), 8.09 (dt, *J =* 8.6, 2.2 Hz, 1H), 7.93 (d, *J =* 11.9 Hz, 1H), 7.01 (d, *J =* 8.6 Hz, 1H), 6.25 (s, 1H), 5.28 (h, *J =* 6.7 Hz, 1H), 4.43 (t, *J =* 6.1 Hz, 2H), 3.47 (d, *J =* 11.9 Hz, 2H), 3.24 - 3.11 (m, 2H), 2.96 - 2.79 (m, 2H), 2.31 - 2.17 (m, 2H), 1.86 - 1.74 (m, 4H), 1.74 - 1.68 (m, 2H), 1.65 (d, *J =* 6.7 Hz, 6H).

### Example 9

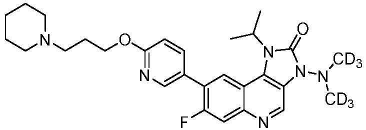

### Reaction process:

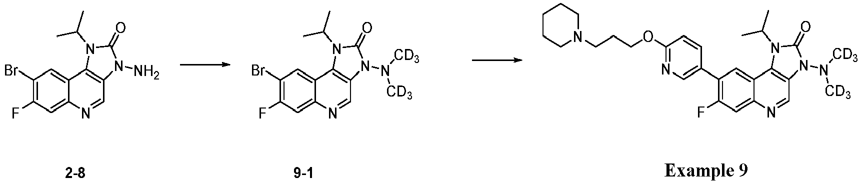

### Step A: synthesis of compound 9-1

**2-8** (100 mg), DMF (5 mL), and sodium hydride (35.4 mg, 60% purity, by mass) were added successively to a single-necked flask, and the mixture was stirred at room temperature for 10 min under N₂ atmosphere. CD₃I (107 mg) was then added, and the mixture was left to react at room temperature for 14 h. After the reaction was completed, 50 mL of ice water was added to quench the reaction. The reaction solution was filtered, and the filter cake was washed with water (5 mL × 2) and dried under reduced pressure to give intermediate **9-1** (70 mg). MS (ESI+, [M+H]⁺) m/z: 372.99.

### Step B: synthesis of Example 9

**9-1** (70 mg), 1,4-dioxane (5 mL), water (2 mL), **A-3** (106 mg), potassium carbonate (78 mg), 3-(di-*tert*-butylphosphino)propane-1-sulfonic acid (1.0 mg), and sodium tetrachloropalladate(II) (0.55 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 9** (14.4 mg). MS (ESI+, [M+H]⁺) *m*/*z:* 513.33. ¹H NMR (500 MHz, DMSO-*d*6) δ 8.80 (s, 1H), 8.52 (t, *J =* 1.9 Hz, 1H), 8.30 (d, *J =* 8.2 Hz, 1H), 8.08 (dt, *J =* 8.7, 2.2 Hz, 1H), 7.94 (d, *J =* 11.9 Hz, 1H), 7.01 (d, *J =* 8.6 Hz, 1H), 5.28 - 5.18 (m, 1H), 4.42 (t, *J =* 6.2 Hz, 2H), 3.53 - 3.39 (m, 2H), 3.24 - 3.07 (m, 2H), 2.96 - 2.75 (m, 2H), 2.27 - 2.13 (m, 2H), 1.86 - 1.69 (m, 6H), 1.64 (d, *J =* 6.8 Hz, 6H).

### Example 10

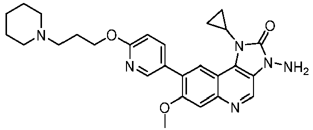

### Reaction process:

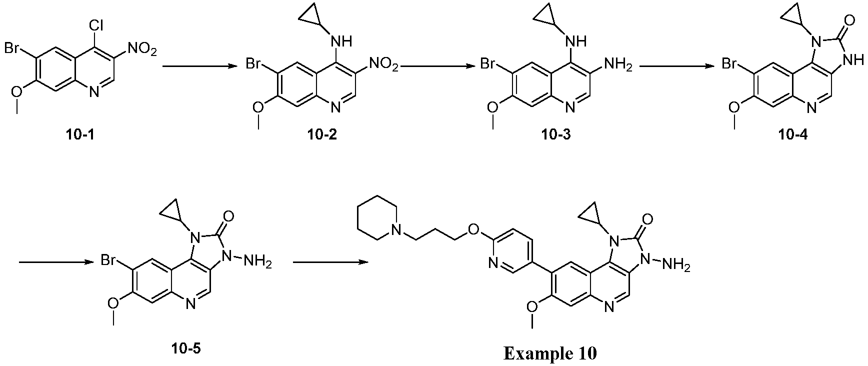

### Step A: synthesis of compound 10-2

Cyclopropylamine (0.27 g) and THF (10 mL) were added successively to a single-necked flask, and then sodium hydride (0.38 g, 60% purity, by mass) was added at 0 °C under N₂ atmosphere. The mixture was stirred for 10 min, and then 10-1 (1.0 g) was added. The mixture was stirred at room temperature for 1 h. After the reaction was completed, the reaction solution was poured into 50 mL of ice water, stirred for 30 min, and filtered, and the filter cake was collected, washed with water (5 mL × 3), and then washed with petroleum ether (5 mL × 4) to give intermediate 10-2 (1.06 g). MS (ESI+, [M+H]⁺) *m*/*z:* 337.84. ¹H NMR (500 MHz, DMSO-*d*6) δ 9.08 (s, 1H), 8.99 (s, 1H), 8.95 (s, 1H), 7.38 (s, 1H), 4.00 (s, 3H), 3.02 (s, 1H), 0.84 - 0.80 (m, 2H), 0.68 - 0.65 (m, 2H).

### Step B: synthesis of compound 10-3

**10-2** (0.27 g), MeOH (60 mL), a solution of ammonium chloride (1.5 g) in water (15 mL), and iron powder (0.8 g) were added successively to a single-necked flask, and the mixture was heated to 65 °C and left to react for 1 h under N₂ atmosphere. After the reaction was completed, the reaction solution was filtered, and the filter cake was washed with dichloromethane (10 mL × 3). The filtrate was concentrated under reduced pressure to dryness, and the resulting crude product was slurried with water (50 mL) and filtered to give intermediate **10-3** (883 mg). MS (ESI+, [M+H]⁺) *m*/*z*: 307.83.

### Step C: synthesis of compound 10-4

**10-3** (0.78 g), THF (20 mL), *N,N*-diisopropylethylamine (1.6 g), and *N,N*'-carbonyldiimidazole (2 g) were added successively to a single-necked flask, and the mixture was stirred at room temperature for 4 h under N₂ atmosphere. After the reaction was completed, the reaction solution was poured into 100 mL of ice water, stirred for 30 min, and filtered, and the filter cake was collected, washed with water (5 mL × 3), and then washed with petroleum ether (5 mL × 4) to give intermediate **10-4** (784 mg). MS (ESI+, [M+H]⁺) *m*/*z:* 333.82.

### Step D: synthesis of compound 10-5

**10-4** (0.68 g), DCM (20 mL), 2,4-dinitrophenylhydroxylamine (0.6 g), tetrabutylammonium bromide (65 mg), and a solution of sodium hydroxide (160 mg) in water (10 mL) were added successively to a single-necked flask, and the mixture was stirred at room temperature overnight. After the reaction was completed, the reaction solution was directly concentrated to remove the organic phase, and acetonitrile (10 mL) was added. The mixture was stirred for 5 min and filtered, and the filter cake was collected and washed with water (5 mL × 3) and acetonitrile (5 mL × 3) successively to give intermediate **10-5** (515 mg). MS (ESI+, [M+H]⁺) *m*/*z:* 349.02.

### Step E: synthesis of Example 10

**10-5** (100 mg), 1,4-dioxane (6 mL), **A-3** (143 mg), a solution of potassium carbonate (119 mg) in water (0.5 mL), a solution of 3-(di-*tert*-butylphosphino)propane-1-sulfonic acid (7.69 mg) in water (0.5 mL), and sodium tetrachloropalladate(II) (4.21 mg) were added successively to a single-necked flask, and the mixture was heated to 80 °C and stirred for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was purified by silica gel column chromatography to give **Example 10** (101 mg). MS (ESI+, [M+H]⁺) *m*/*z:* 489.31. ¹H NMR (500 MHz, DMSO-*d*₆) δ 8.69 (s, 1H), 8.62 (s, 1H), 8.41 (d, *J =* 2.4 Hz, 1H), 7.97 (dd, *J =* 8.6, 2.5 Hz, 1H), 7.55 (s, 1H), 6.91 (d, *J =* 8.6 Hz, 1H), 5.47 (s, 2H), 4.33 (t, *J =* 6.6 Hz, 2H), 3.93 (s, 3H), 3.49 (tt, *J =* 7.0, 3.7 Hz, 1H), 2.39 (t, *J =* 7.2 Hz, 2H), 2.33 (s, 4H), 1.89 (p, *J =* 6.8 Hz, 2H), 1.49 (p, *J =* 5.6 Hz, 4H), 1.40 - 1.38 (m, 2H), 1.23 - 1.19 (m, 2H), 1.12 - 1.09 (m, 2H).

### Example 11

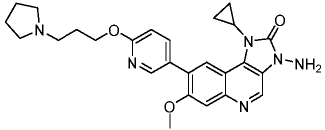

### Reaction process:

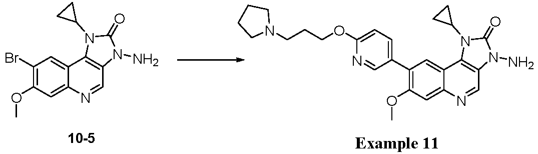

### Step A: synthesis of Example 11

**10-5** (100 mg), 1,4-dioxane (6 mL), **A-6** (143 mg), a solution of potassium carbonate (119 mg) in water (0.5 mL), a solution of 3-(di-*tert*-butylphosphino)propane-1-sulfonic acid (7.69 mg) in water (0.5 mL), and sodium tetrachloropalladate(II) (4.21 mg) were added successively to a single-necked flask, and the mixture was heated to 80 °C and stirred for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was purified by silica gel column chromatography to give **Example 11** (74 mg). MS (ESI+, [M+H]⁺) *m*/*z:* 475.32. ¹H NMR (500 MHz, DMSO-*d*₆) δ 8.69 (s, 1H), 8.61 (s, 1H), 8.40 (d, *J =* 2.5 Hz, 1H), 7.97 (dd, *J =* 8.6, 2.5 Hz, 1H), 7.55 (s, 1H), 6.91 (d, *J =* 8.6 Hz, 1H), 5.47 (s, 2H), 4.35 (t, *J =* 6.6 Hz, 2H), 3.93 (s, 3H), 3.48 (tt, *J =* 6.9, 3.7 Hz, 1H), 2.53 (t, *J =* 7.2 Hz, 2H), 2.46 - 2.42 (m, 4H), 1.92 (p, *J* = 6.8 Hz, 2H), 1.71 - 1.66 (m, 4H), 1.23 - 1.19 (m, 2H), 1.12 - 1.09 (m, 2H).

### Example 12

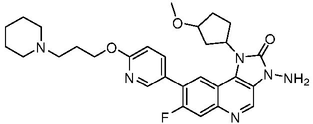

### Reaction process:

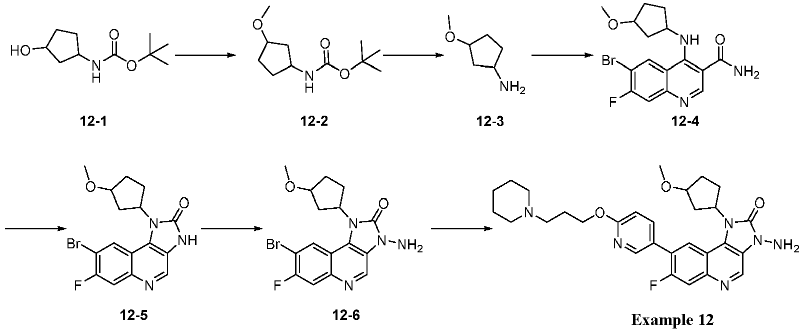

### Step A: synthesis of compound 12-2

**12-1** (0.27 g) and DMF (30 mL) were added successively to a two-necked flask, and then 60% sodium hydride (0.3 g) was added at 0 °C under N₂ atmosphere. The mixture was stirred for 30 min, and then iodomethane (1.0 g) was added. The mixture was stirred at room temperature for 3 h. After the reaction was completed, the reaction solution was poured slowly into 50 mL of ice water and stirred for 10 min, and ethyl acetate (50 mL × 3) was added for extraction. The organic phases were combined, washed with saturated brine (50 mL), dried over anhydrous sodium sulfate, filtered, and concentrated under reduced pressure to dryness to give intermediate **12-2** (1 g).

### Step B: synthesis of compound 12-3

**12-2** (1 g) and 4 M hydrogen chloride-dioxane solution (10 mL) were added successively to a single-necked flask, and the mixture was stirred at room temperature for 1 h. After the reaction was completed, the reaction solution was concentrated under reduced pressure to dryness to give intermediate **12-3** (690 mg).

### Step C: synthesis of compound 12-4

**2-5** (1 g), *N,N*-diisopropylethylamine (2.4 g), and **12-3** (0.5 g) were added successively to a single-necked flask, and the mixture was heated to 95 °C and stirred for 2 h under N₂ atmosphere. After the reaction was completed, the reaction solution was poured into 100 mL of water, stirred for 5 min, and filtered, and the filter cake was collected, washed with water (5 mL × 3), and then washed with petroleum ether (5 mL × 4) to give intermediate **12-4** (889 mg). MS (ESI+, [M+H]⁺) *m*/*z*: 381.94.

### Step D: synthesis of compound 12-5

**12-4** (600 mg), DBU (478 mg), and methanol (12 mL) were added successively to a single-necked flask, and then 1,3,5-trichloro-1,3,5-triazine-2,4,6-trione (182 mg) was added at 5 °C. The mixture was stirred at room temperature for 1 h under N₂ atmosphere. After the reaction was completed, the reaction solution was filtered directly, and the filter cake was washed with water (10 mL × 2) and dried under vacuum to give intermediate **12-5** (589 mg). MS (ESI+, [M+H]⁺) *m*/*z:* 379.93.

### Step E: synthesis of compound 12-6

**12-5** (515 mg), DCM (20 mL), 2,4-dinitrophenylhydroxylamine (405 mg), tetrabutylammonium bromide (43 mg), and a solution of sodium hydroxide (108 mg) in water (10 mL) were added successively to a single-necked flask, and the mixture was stirred at room temperature overnight. After the reaction was completed, the reaction solution was concentrated to remove the organic phase, and acetonitrile (10 mL) was added. The mixture was stirred at room temperature for 10 min and filtered, and the filter cake was collected and washed with water (5 mL × 3) and then with acetonitrile (5 mL × 3) to give intermediate **12-6** (268 mg). MS (ESI+, [M+H]⁺) *m*/*z:* 394.97.

### Step F: synthesis of Example 12

**12-6** (88 mg), 1,4-dioxane (6 mL), **A-3** (115 mg), a solution of potassium carbonate (92 mg) in water (0.5 mL), a solution of 3-(di-*tert*-butylphosphino)propane-1-sulfonic acid (5.97 mg) in water (0.5 mL), and sodium tetrachloropalladate(II) (3.28 mg) were added successively to a single-necked flask, and the mixture was heated to 80 °C and stirred for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was subjected to silica gel column chromatography (eluent: DCM/MeOH = 10/1) to give **Example 12** (86 mg). MS (ESI+, [M+H]⁺) *m*/*z:* 535.33. ¹H NMR (500 MHz, DMSO-*d*₆) δ 8.85 (s, 1H), 8.47 - 8.46 (m, 1H), 8.41 (d, *J =* 8.2 Hz, 1H), 8.03 - 8.00 (m, 1H), 7.91 (d, *J =* 11.9 Hz, 1H), 6.97 (d, *J =* 8.6 Hz, 1H), 5.59 (s, 2H), 5.33 (p, *J =* 9.3 Hz, 1H), 4.36 (t, *J =* 6.6 Hz, 2H), 3.91 (p, *J =* 6.0 Hz, 1H), 3.05 (s, 3H), 2.48 - 2.28 (m, 9H), 2.05 - 1.98 (m, 1H), 1.97 - 1.88 (m, 4H), 1.49 (p, *J =* 5.6 Hz, 4H), 1.38 (q, *J =* 5.9 Hz, 2H).

### Example 13

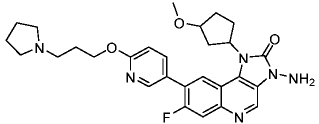

### Reaction process:

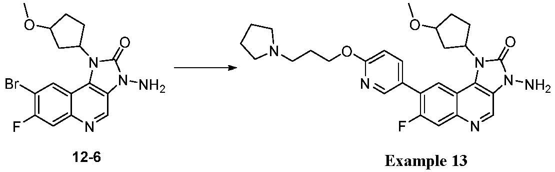

### Step A: synthesis of Example 13

**12-6** (88 mg), 1,4-dioxane (6 mL), **A-6** (111 mg), a solution of potassium carbonate (92 mg) in water (0.5 mL), a solution of 3-(di-*tert*-butylphosphino)propane-1-sulfonic acid (5.97 mg) in water (0.5 mL), and sodium tetrachloropalladate(II) (3.28 mg) were added successively to a single-necked flask, and the mixture was heated to 80 °C and stirred for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was subjected to silica gel column chromatography (eluent: DCM/MeOH = 10/1) to give **Example 13** (84 mg). MS (ESI+, [M+H]⁺) *m*/*z*: 521.25. ¹H NMR (500 MHz, DMSO-*d*₆) δ 8.85 (s, 1H), 8.46 (t, *J=* 1.7 Hz, 1H), 8.41 (d, *J =* 8.1 Hz, 1H), 8.01 (dt, *J* = 8.7, 2.3 Hz, 1H), 7.91 (d, *J =* 12.0 Hz, 1H), 6.97 (d, *J =* 8.5 Hz, 1H), 5.59 (s, 2H), 5.33 (p, *J =* 9.3 Hz, 1H), 4.38 (t, *J =* 6.6 Hz, 2H), 3.91 (p, *J =* 6.0 Hz, 1H), 3.05 (s, 3H), 2.54 (t, *J =* 7.2 Hz, 2H), 2.48 - 2.39 (m, 6H), 2.37 - 2.29 (m, 1H), 2.05 - 1.98 (m, 1H), 1.97 - 1.90 (m, 4H), 1.72 - 1.66 (m, 4H).

### Example 14

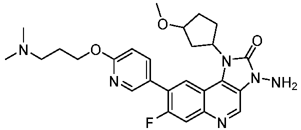

### Reaction process:

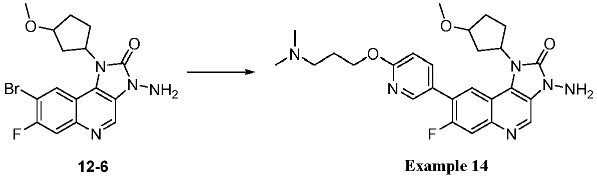

### Step A: synthesis of Example 14

**12-6** (88 mg), 1,4-dioxane (6 mL), **A-12** (111 mg), a solution of potassium carbonate (92 mg) in water (0.5 mL), a solution of 3-(di-*tert*-butylphosphino)propane-1-sulfonic acid (5.97 mg) in water (0.5 mL), and sodium tetrachloropalladate(II) (3.28 mg) were added successively to a single-necked flask, and the mixture was heated to 80 °C and stirred for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was purified by silica gel column chromatography to give **Example 14** (91 mg). MS (ESI+, [M+H]⁺) *m*/*z:* 495.33. ¹H NMR (500 MHz, DMSO-*d*₆) δ 8.85 (s, 1H), 8.46 (d, *J =* 2.3 Hz, 1H), 8.42 (dd, *J =* 8.4, 2.7 Hz, 1H), 8.01 (dt, *J =* 8.6, 2.3 Hz, 1H), 7.91 (dd, *J =* 11.9, 2.7 Hz, 1H), 6.97 (dd, *J =* 8.6, 2.5 Hz, 1H), 5.59 (s, 2H), 5.33 (p, *J =* 9.4 Hz, 1H), 4.37 (td, *J =* 6.6, 2.4 Hz, 2H), 3.91 (p, *J =* 5.9 Hz, 1H), 3.05 (s, 3H), 2.48 - 2.41 (m, 2H), 2.39 - 2.35 (m, 2H), 2.34 - 2.28 (m, 1H), 2.15 (s, 6H), 2.05 - 2.00 (m, 1H), 1.98 - 1.94 (m, 2H), 1.92 - 1.86 (m, 2H).

### Example 15

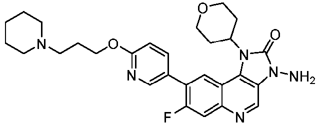

### Reaction process:

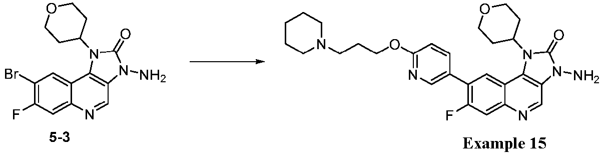

### Step A: synthesis of Example 15

**5-3** (130 mg), 1,4-dioxane (10 mL), **A-3** (177 mg), a solution of potassium carbonate (141 mg) in water (0.5 mL), a solution of 3-(di-*tert*-butylphosphino)propane-1-sulfonic acid (9.15 mg) in water (0.5 mL), and sodium tetrachloropalladate(II) (5.02 mg) were added successively to a single-necked flask, and the mixture was heated to 80 °C and stirred for 1 h under N₂ atmosphere. After the reaction was completed, 50 mL of water was added, and dichloromethane (50 mL × 3) was added for extraction. The organic phases were combined, washed with saturated brine (50 mL), dried over anhydrous sodium sulfate, filtered, and concentrated under reduced pressure to dryness, and the resulting crude product was purified by silica gel column chromatography to give **Example 15** (158 mg). MS (ESI+, [M+H]⁺) *m*/*z:* 521.29. ¹H NMR (500 MHz, DMSO-*d*₆) δ 8.83 (s, 1H), 8.54 (t, *J =* 2.0 Hz, 1H), 8.33 (d, *J =* 8.2 Hz, 1H), 8.08 (dt, *J =* 8.6, 2.2 Hz, 1H), 7.93 (d, *J =* 12.1 Hz, 1H), 7.00 (d, *J =* 8.6 Hz, 1H), 5.58 (s, 2H), 5.09 (tt, *J =* 11.8, 4.3 Hz, 1H), 4.36 (t, *J =* 6.6 Hz, 2H), 4.03 (dd, *J =* 11.3, 4.6 Hz, 2H), 3.54 (td, *J =* 12.0, 1.9 Hz, 2H), 2.70 (qd, *J =* 12.5, 4.7 Hz, 2H), 2.41 - 2.34 (m, 6H), 1.93 - 1.89 (m, 4H), 1.52 - 1.47 (m, 4H), 1.40 - 1.37 (m, 2H).

### Example 16

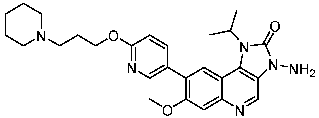

### Reaction process:

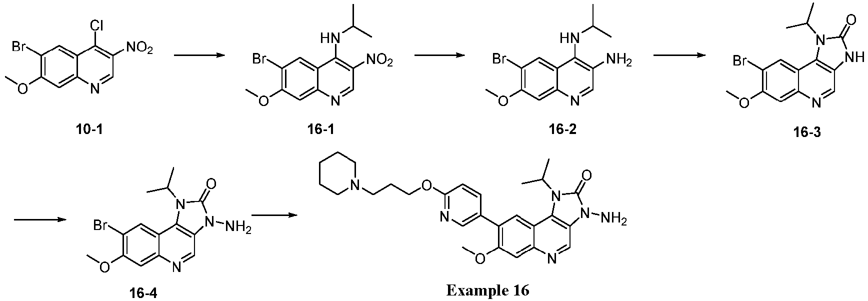

### Step A: synthesis of compound 16-1

Isopropylamine (167.4 mg) and THF (15 mL) were added successively to a single-necked flask, and then sodium hydride (228 mg) was added at 0 °C under nitrogen atmosphere. The mixture was stirred for 5 min, and then **10-1** (600 mg) was added. The mixture was stirred at room temperature for 15 min. After the reaction was completed, water (50 mL) was added, and the reaction solution was filtered. The filter cake was collected and washed with water (5 mL × 3) and petroleum ether (5 mL × 2) successively to give intermediate **16-1** (500 mg). MS (ESI+, [M+H]⁺) *m*/*z:* 339.92.

### Step B: synthesis of compound 16-2

**16-1** (500 mg), DCM (20 mL), acetic acid (10 mL), and iron powder (410 mg) were added successively to a single-necked flask, and the mixture was left to react at room temperature for 1 h under nitrogen atmosphere. After the reaction was completed, the reaction solution was filtered. The filtrate was concentrated to dryness, and the residue was adjusted to pH 8-9 with a saturated sodium bicarbonate solution, followed by extraction with DCM (20 mL × 5). The organic phases were combined, washed with saturated brine (20 mL), dried over anhydrous sodium sulfate, and filtered, and the filtrate was concentrated to give intermediate **16-2** (400 mg). MS (ESI+, [M+H]⁺) *m*/*z*: 309.90.

### Step C: synthesis of compound 16-3

**16-2** (0.3 g), THF (10 mL), DIPEA (0.625 g), and CDI (0.784 g) were added successively to a single-necked flask, and the mixture was left to react at room temperature for 12 h under nitrogen atmosphere. After the reaction was completed, ice water (100 mL) was added, and the reaction solution was filtered. The filter cake was collected and washed with water (5 mL × 3) and petroleum ether (5 mL × 2) successively to give intermediate **16-3** (0.23 g). MS (ESI+, [M+H]⁺) *m*/*z*: 335.83.

### Step D: synthesis of compound 16-4

**16-3** (180 mg), DCM (5 mL), water (2 mL), TBAB (17.26 mg), 2,4-dinitrophenylhydroxylamine (160 mg), and NaOH (0.59 mg) were added successively to a single-necked flask, and the mixture was stirred at room temperature for 24 h under N₂ atmosphere. After the reaction was completed, the reaction solution was concentrated to remove most of the solvent, and then filtered. The filter cake was slurried with acetonitrile (10 mL) and dried under vacuum to give intermediate **16-4** (118 mg). MS (ESI+, [M+H]⁺) *m*/*z*: 350.88.

### Step E: synthesis of Example 16

**16-4** (50 mg), 1,4-dioxane (5 mL), water (2 mL), **A-3** (64.1 mg), potassium carbonate (59 mg), 3-(di-*tert*-butylphosphino)propane-1-sulfonic acid (1.0 mg), and sodium tetrachloropalladate(II) (0.5 mg) were added successively to a single-necked flask, and the mixture was stirred at 80 °C for 1 h under nitrogen atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by column chromatography to give **Example 16** (38.6 mg). MS (ESI+, [M+H]⁺) *m*/*z:* 491.28. ¹H NMR (500 MHz, DMSO-d6) *δ* 8.72 (s, 1H), 8.39 (d, *J =* 2.4 Hz, 1H), 8.11 (s, 1H), 7.96 (dd, *J =* 8.5, 2.6 Hz, 1H), 7.57 (s, 1H), 6.91 (d, *J =* 8.5 Hz, 1H), 5.50 (s, 2H), 5.22 (p, *J* = 6.7 Hz, 1H), 4.34 (t, *J =* 6.6 Hz, 2H), 3.92 (s, 3H), 2.39 (t, *J =* 7.2 Hz, 2H), 2.36 - 2.32 (m, 4H), 1.90 (p, *J =* 6.7 Hz, 2H), 1.62 (d, *J =* 6.7 Hz, 6H), 1.53 - 1.46 (m, 4H), 1.41 - 1.33 (m, 2H).

### Example 17

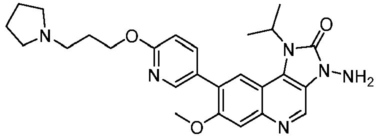

### Reaction process:

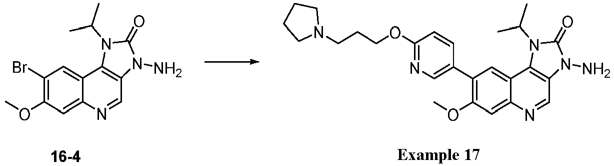

**16-4** (50 mg), 1,4-dioxane (5 mL), water (2 mL), **A-6** (82 mg), potassium carbonate (59 mg), 3-(di-*tert*-butylphosphino)propane-1-sulfonic acid (1.0 mg), and sodium tetrachloropalladate(II) (0.5 mg) were added successively to a single-necked flask, and the mixture was stirred at 80 °C for 1 h under nitrogen atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by column chromatography to give **Example 17** (10.3 mg). MS (ESI+, [M+H]⁺) *m*/*z:* 477.26. ¹H NMR (500 MHz, DMSO-*d*6) *δ* 8.72 (s, 1H), 8.39 (d, *J =* 2.6 Hz, 1H), 8.11 (s, 1H), 7.96 (dd, *J =* 8.5, 2.6 Hz, 1H), 7.57 (s, 1H), 6.91 (d, *J =* 8.5 Hz, 1H), 5.50 (s, 2H), 5.27 - 5.15 (m, 1H), 4.36 (t, *J =* 6.6 Hz, 2H), 3.92 (s, 3H), 2.65 - 2.55 (m, 4H), 2.04 - 1.86 (m, 2H), 1.77 - 1.66 (m, 4H), 1.62 (d, *J =* 6.9 Hz, 6H), 1.28 - 1.22 (m, 2H).

### Example 18

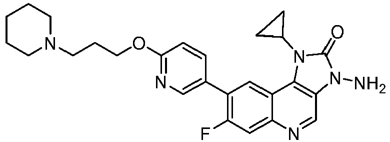

### Reaction process:

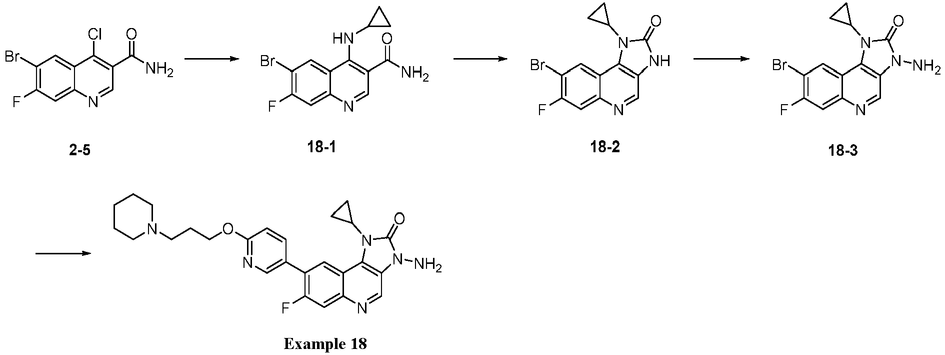

### Step A: synthesis of compound 18-1

**2-5** (1.0 g), potassium carbonate (1.37 g), acetonitrile (40 mL), and cyclopropylamine (0.28 g) were added successively to a single-necked flask, and the mixture was stirred at 95 °C for 4 h under N₂ atmosphere. After the reaction was completed, the reaction solution was cooled, and 150 mL of water was added. The reaction solution was filtered, and the filter cake was washed with water (5 mL × 4) and dried under vacuum to give intermediate **18-1** (985 mg). MS (ESI+, [M+H]⁺) *m*/*z:* 323.85

### Step B: synthesis of compound 18-2

**18-1** (750 mg), iodobenzene diacetate (745 mg), acetonitrile (10 mL), ethyl acetate (10 mL), water (10 mL), and KOH (260 mg) were added successively to a single-necked flask, and the mixture was stirred at room temperature overnight. After the reaction was completed, the reaction solution was filtered directly, and the filter cake was washed with water (10 mL × 2) and dried under vacuum to give intermediate **18-2** (700 mg). MS (ESI+, [M+H]⁺) *m*/*z:* 321.92.

### Step C: synthesis of compound 18-3

**18-2** (700 mg), DCM (20 mL), water (10 mL), TBAB (70 mg), 2,4-dinitrophenylhydroxylamine (650 mg), and NaOH (175 mg) were added successively to a single-necked flask, and the mixture was stirred at room temperature overnight. After the reaction was completed, the reaction solution was concentrated to remove most of the solvent, and then filtered. The filter cake was slurried with 5 mL of acetonitrile and dried under vacuum to give intermediate **18-3** (350 mg). MS (ESI+, [M+H]⁺) *m*/*z:* 336.98.

### Step D: synthesis of Example 18

**18-3** (150 mg), 1,4-dioxane (5 mL), water (2 mL), **A-3** (249 mg), potassium carbonate (183 mg), 3-(di-*tert*-butylphosphino)propane-1-sulfonic acid (2.37 mg), and sodium tetrachloropalladate(II) (1.30 mg) were added successively to a single-necked flask, and the mixture was stirred at 80 °C for 1 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was slurried with acetonitrile (5 mL) to give **Example 18** (80 mg). MS (ESI+, [M+H]⁺) *m*/*z:* 477.27. ¹H NMR (500 MHz, DMSO-*d*₆) δ 8.91 - 8.77 (m, 2H), 8.61 (s, 1H), 8.13 - 7.99 (m, 1H), 7.89 (d, *J =* 12.3 Hz, 1H), 6.99 (d, *J =* 8.6 Hz, 1H), 5.53 (s, 2H), 4.36 (t, *J =* 6.6 Hz, 2H), 3.69 - 3.43 (m, 1H), 2.50 - 2.28 (m, 6H), 1.99 - 1.78 (m, 2H), 1.58 - 1.45 (m, 4H), 1.45 - 1.33 (m, 2H), 1.33 - 1.21 (m, 2H), 1.17 -1.10 (m, 2H).

### Example 19

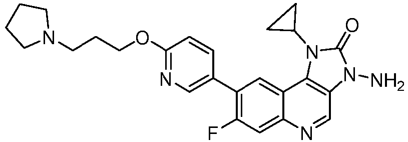

### Reaction process:

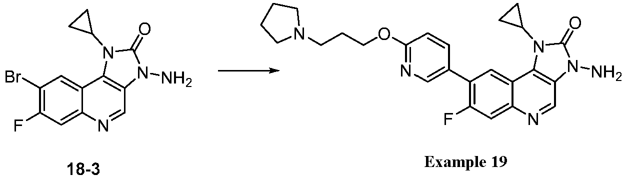

**18-3** (150 mg), 1,4-dioxane (5 mL), water (2 mL), **A-6** (249 mg), potassium carbonate (183 mg), 3-(di-*tert*-butylphosphino)propane-1-sulfonic acid (2.37 mg), and sodium tetrachloropalladate(II) (1.30 mg) were added successively to a single-necked flask, and the mixture was stirred at 80 °C for 1 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was slurried with acetonitrile (5 mL) to give **Example 19** (60 mg). MS (ESI+, [M+H]⁺) *m*/*z:* 463.37. ¹H NMR (500 MHz, DMSO-*d*₆) δ 9.00 - 8.75 (m, 2H), 8.50 (s, 1H), 8.05 (dt, *J* = 8.6, 2.2 Hz, 1H), 7.89 (d, *J =* 12.3 Hz, 1H), 6.99 (d, *J =* 8.5 Hz, 1H), 5.53 (s, 2H), 4.37 (t, *J =* 6.6 Hz, 2H), 3.59 - 3.51 (m, 1H), 2.55 (t, *J =* 7.2 Hz, 2H), 2.49 - 2.38 (m, 4H), 1.99 - 1.80 (m, 2H), 1.82 - 1.64 (m, 4H), 1.30 - 1.20 (m, 2H), 1.17 - 1.09 (m, 2H).

### Example 20

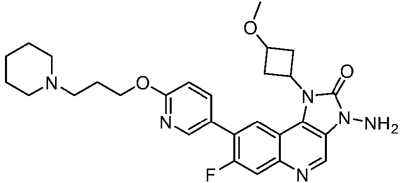

### Reaction process:

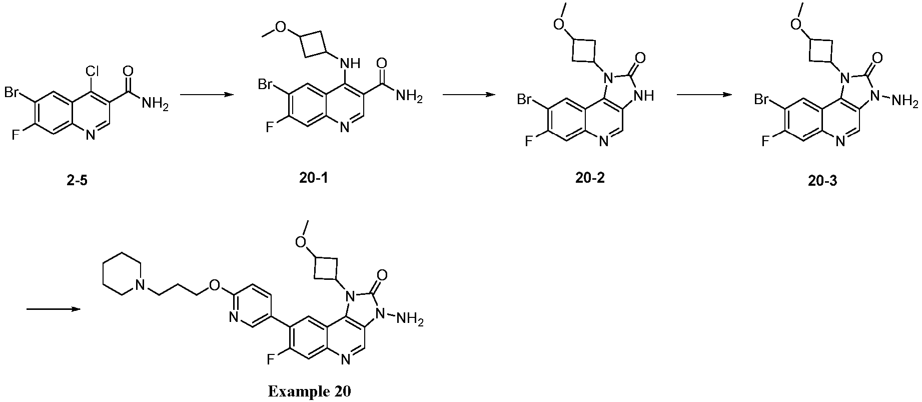

### Step A: synthesis of compound 20-1

**2-5** (1.0 g), potassium carbonate (1.37 g), acetonitrile (40 mL), and 3-methoxycyclobutylamine hydrochloride (0.46 g) were added successively to a single-necked flask, and the mixture was stirred at 95 °C for 4 h under N₂ atmosphere. After the reaction was completed, the reaction solution was cooled, and 150 mL of water was added. The reaction solution was filtered, and the filter cake was washed with water (5 mL × 4) and dried under vacuum to give intermediate **20-1** (1.06 g). MS (ESI+, [M+H]⁺) *m*/*z*: 367.95.

### Step B: synthesis of compound 20-2

**20-1** (500 mg), DBU (413 mg), and methanol (30 mL) were added successively to a single-necked flask, and then 1,3,5-trichloro-1,3,5-triazine-2,4,6-trione (158 mg) was added at 5 °C. The mixture was stirred at room temperature for 1 h under N₂ atmosphere. After the reaction was completed, the reaction solution was filtered directly, and the filter cake was washed with water (10 mL × 2) and dried under vacuum to give intermediate **20-2** (350 mg). MS (ESI+, [M+H]⁺) *m*/*z:* 366.94.

### Step C: synthesis of compound 20-3

**20-2** (350 mg), DCM (20 mL), water (10 mL), TBAB (31 mg), 2,4-dinitrophenylhydroxylamine (285 mg), and NaOH (76 mg) were added successively to a single-necked flask, and the mixture was stirred at room temperature overnight. After the reaction was completed, the reaction solution was concentrated to remove most of the solvent, and then filtered. The filter cake was slurried with 5 mL of acetonitrile and dried under vacuum to give intermediate **20-3** (240 mg). MS (ESI+, [M+H]⁺) *m*/*z*: 380.97.

### Step D: synthesis of Example 20

**20-3** (150 mg), 1,4-dioxane (5 mL), water (2 mL), **A-3** (249 mg), potassium carbonate (183 mg), 3-(di-*tert*-butylphosphino)propane-1-sulfonic acid (2.37 mg), and sodium tetrachloropalladate(II) (1.30 mg) were added successively to a single-necked flask, and the mixture was stirred at 80 °C for 1 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was slurried with acetonitrile (5 mL) to give **Example 20** (80 mg). MS (ESI+, [M+H]⁺) *m*/*z:* 521.31. ¹H NMR (500 MHz, DMSO-*d*₆) δ 8.82 (s, 1H), 8.56 - 8.47 (m, 1H), 8.37 (d, *J* = 8.1 Hz, 1H), 8.09 - 7.99 (m, 1H), 7.90 (d, *J =* 12.0 Hz, 1H), 6.98 (d, *J =* 8.5 Hz, 1H), 5.55 (s, 2H), 5.21 - 5.02 (m, 1H), 4.36 (t, *J=* 6.6 Hz, 2H), 3.90 - 3.76 (m, 1H), 3.18 (s, 3H), 3.05 - 2.93 (m, 2H), 2.86 - 2.74 (m, 2H), 2.43 - 2.22 (m, 6H), 1.97 - 1.86 (m, 2H), 1.54 - 1.45 (m, 4H), 1.46 - 1.33 (m, 2H).

### Example 21

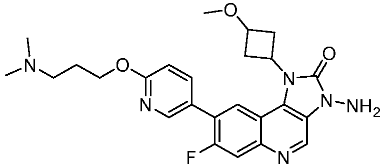

### Reaction process:

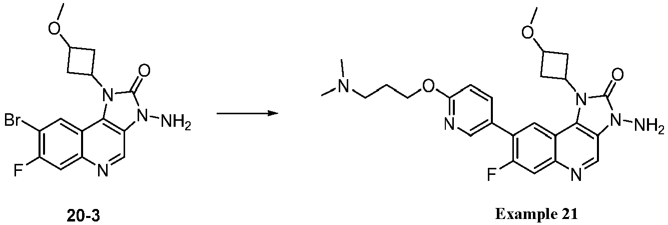

**20-3** (150 mg), 1,4-dioxane (5 mL), water (2 mL), **A-12** (249 mg), potassium carbonate (183 mg), 3-(di-*tert*-butylphosphino)propane-1-sulfonic acid (2.37 mg), and sodium tetrachloropalladate(II) (1.30 mg) were added successively to a single-necked flask, and the mixture was stirred at 80 °C for 1 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was slurried with acetonitrile (5 mL) to give **Example 21** (95 mg). MS (ESI+, [M+H]⁺) *m*/*z:* 481.31. ¹H NMR (500 MHz, DMSO-*d*₆) δ 8.91 - 8.77 (m, 1H), 8.51 (s, 1H), 8.38 (s, 1H), 8.15 - 8.00 (m, 1H), 8.00 - 7.86 (m, 1H), 6.99 (s, 1H), 5.55 (s, 2H), 5.10 (s, 1H), 4.49 - 4.30 (m, 2H), 3.90 - 3.75 (m, 1H), 3.18 (s, 3H), 3.09 - 2.93 (m, 2H), 2.90 - 2.75 (m, 2H), 2.42 - 2.35 (m, 2H), 2.16 (s, 6H), 2.01 - 1.86 (m, 2H).

### Example 22

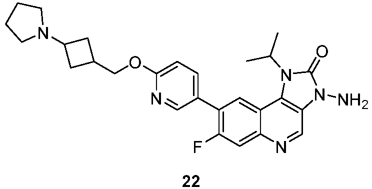

### Reaction process:

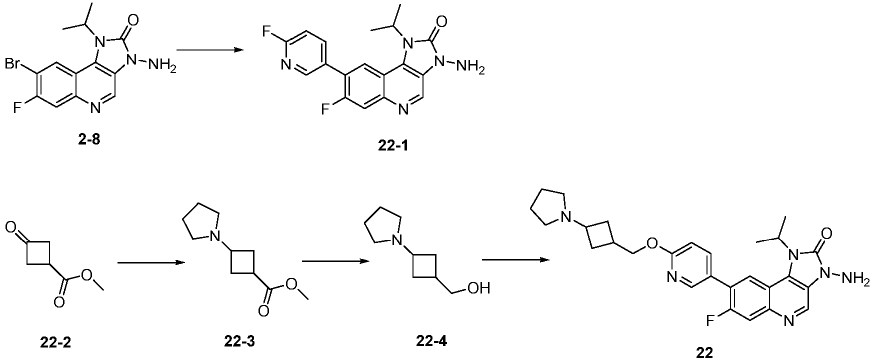

### Step A: synthesis of compound 22-1

**2-8** (100 mg), 1,4-dioxane (3 mL), 2-fluoropyrimidine-5-boronic acid pinacol ester (100 mg), potassium carbonate (122 mg) dissolved in 0.5 mL of water, 3-(di-*tert*-butylphosphino)propane-1-sulfonic acid (3.96 mg) dissolved in 0.5 mL of water, and sodium tetrachloropalladate(II) (2.17 mg) were added successively to a single-necked flask, and the mixture was heated to 80 °C and stirred for 1 h under N₂ atmosphere. After the reaction was completed, 10 mL of water was added, and dichloromethane was added for extraction. The organic phases were combined, washed with saturated brine, dried over anhydrous sodium sulfate, filtered, and concentrated under reduced pressure to dryness, and the resulting crude product was subjected to silica gel column chromatography (eluent: DCM/MeOH) to give intermediate **22-1** (105 mg). MS (ESI+, [M+H]⁺) *m*/*z:* 356.05. ¹H NMR (500 MHz, DMSO-*d*₆) δ 8.84 (s, 1H), 8.60 (t, *J =* 1.7 Hz, 1H), 8.39 (d, *J =* 8.1 Hz, 1H), 8.35 (tt, *J =* 8.1, 2.1 Hz, 1H), 7.96 (d, *J =* 12.0 Hz, 1H), 7.40 (dd, *J =* 8.5, 2.8 Hz, 1H), 5.56 (s, 2H), 5.31 (p, *J =* 6.7 Hz, 1H), 1.64 (d, *J =* 6.7 Hz, 6H).

### Step B: synthesis of compound 22-3

**22-2** (1 g), ethanol (100 mL), pyrrolidine (555 mg), and 10% (purity, by mass) Pd/C (0.831 g) were added successively to a single-necked flask, and the mixture was stirred at room temperature overnight under H₂ atmosphere. After the reaction was completed, the reaction solution was filtered through diatomite under vacuum, and the filtrate was concentrated under reduced pressure to dryness to give intermediate **22-3** (1.4 g).

### Step C: synthesis of compound 22-4

**22-3** (200 mg) and tetrahydrofuran (5 mL) were added successively to a single-necked flask, and then a 2.5 M lithium aluminum hydride-tetrahydrofuran solution (1.31 mL) was added at 0 °C. The mixture was stirred at room temperature for 1 h under N₂ atmosphere. After the reaction was completed, the reaction solution was poured into 2 mL of water, stirred for 5 min, and filtered through diatomite under vacuum, and the filtrate was concentrated under reduced pressure to dryness to give intermediate **22-4** (150 mg).

### Step D: synthesis of Example 22

**22-4** (131 mg) and tetrahydrofuran (5 mL) were added successively to a single-necked flask, and then 60wt% sodium hydride (56 mg) was added at 0 °C under N₂ atmosphere. The mixture was heated to 50 °C and stirred for 20 min, and then **22-1** (100 mg) was added. The mixture was stirred for 2 h. After the reaction was completed, the reaction solution was poured slowly into 10 mL of ice water and stirred for 10 min, and dichloromethane was added for extraction. The organic phases were combined, washed with saturated brine, dried over anhydrous sodium sulfate, filtered, and concentrated under reduced pressure to dryness, and the resulting crude product was subjected to silica gel column chromatography (eluent: DCM/MeOH) to give **Example 22** (66 mg). MS (ESI+, [M+H]⁺) *m*/*z:* 491.25. ¹H NMR (500 MHz, DMSO-*d*₆) δ 8.82 (s, 1H), 8.49 (s, 1H), 8.32 (d, *J =* 8.1 Hz, 1H), 8.04 (dt, *J =* 8.6, 2.3 Hz, 1H), 7.91 (d, *J =* 12.0 Hz, 1H), 6.98 (d, *J =* 8.6 Hz, 1H), 5.55 (s, 2H), 5.33 - 5.25 (m, 1H), 4.30 (d, *J =* 6.5 Hz, 2H), 2.80 (p, *J =* 7.8 Hz, 1H), 2.44 (q, *J =* 8.0 Hz, 1H), 2.37 (d, *J =* 5.8 Hz, 4H), 2.18 - 2.13 (m, 2H), 1.75 - 1.71 (m, 2H), 1.69 - 1.66 (m, 4H), 1.64 (d, *J =* 6.7 Hz, 6H).

### Example 23

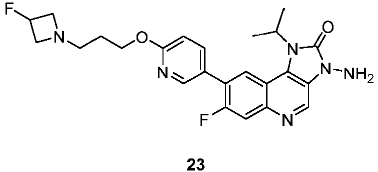

### Reaction process:

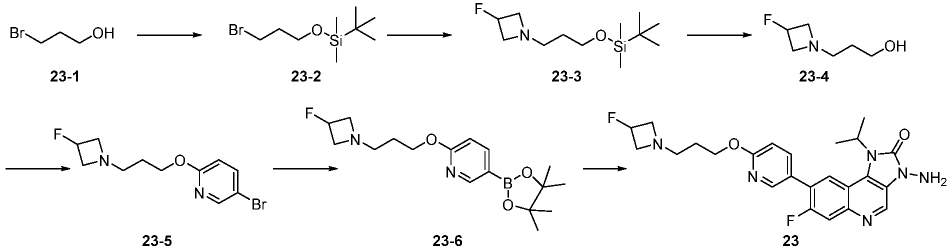

### Step A: synthesis of compound 23-2

3-Bromopropanol (20 g), DCM (150 mL), and imidazole (17.14 g) were added successively to a single-necked flask, and then tert-butyldimethylsilyl chloride (21.69 g) was added at 0 °C under N₂ atmosphere. The mixture was stirred for 15 min, and then the mixture was stirred at room temperature for 1 h. After the reaction was completed, 50 mL of DCM and 50 mL of saturated brine were added to the reaction solution. After liquid separation, the aqueous phase was extracted with 40 mL of DCM, and the organic phases were combined, dried over anhydrous sodium sulfate, and filtered under vacuum, and the solvent was removed by evaporation, thus giving intermediate **23-2** (36 g). GC-MS (EI, M⁺) *m*/*z:* 253. Step B: synthesis of compound **23-3 23-2** (8 g), acetonitrile (40 mL), 3-fluoroazetidine hydrochloride (3.6 g), potassium carbonate (13.1 g), and potassium iodide (2.62 g) were added successively to a single-necked flask, and the mixture was heated to 60 °C and left to react for 2 h under N₂ atmosphere. After the reaction was completed, 100 mL of water was added, and EA was added for extraction. The organic phases were combined, washed with saturated brine, dried over anhydrous sodium sulfate, filtered, and concentrated under reduced pressure to dryness to give intermediate **23-3** (7.0 g). GC-MS (EI, M⁺) *m*/*z:* 247.

### Step C: synthesis of compound 23-4

**23-3** (0.78 g), dioxane (20 mL), and a solution of hydrochloric acid in dioxane (4 M, 27 mL) were added successively to a single-necked flask, and the mixture was stirred at room temperature for 2 h. After the reaction was completed, the reaction solution was concentrated to remove the solvent, and 200 mL of EA and 5 g of Na₂CO₃ were added. The mixture was stirred at room temperature for 30 min and then filtered under vacuum, and the filtrate was concentrated to remove the solvent, thus giving intermediate **23-4** (3.2 g). GC-MS (EI, M⁺) *m*/*z:* 133.

### Step D: synthesis of compound 23-5

Referring to the synthesis of Preparation Example **A-3,** in the preparation step of **A-2, A-1** was replaced with **23-4** to give compound **23-5.** MS (ESI+, [M+H]⁺) *m*/*z:* 288.87.

### Step E: synthesis of compound 23-6

Referring to the synthesis of Preparation Example **A-3,** in the preparation step of **A-3, A-2** was replaced with **23-5** to give compound **23-6.** MS (ESI+, [M+H]⁺) *m*/*z:* 337.00.

### Step F: synthesis of Example 23

**2-8** (100 mg), 1,4-dioxane (6 mL), **23-6** (mg), potassium carbonate (61 mg) dissolved in 0.5 mL of water, 3-(di-*tert*-butylphosphino)propane-1-sulfonic acid (4 mg) dissolved in 0.5 mL of water, and sodium tetrachloropalladate(II) (2 mg) were added successively to a single-necked flask, and the mixture was heated to 80 °C and stirred for 1 h under N₂ atmosphere. After the reaction was completed, 50 mL of water was added, and dichloromethane was added for extraction. The organic phases were combined, washed with saturated brine, dried over anhydrous sodium sulfate, filtered, and concentrated under reduced pressure to dryness, and the resulting crude product was purified by silica gel column chromatography to give **Example 23** (60 mg). MS (ESI+, [M+H]⁺) *m*/*z*: 469.17. ¹H NMR (500 MHz, DMSO-*d*₆) δ 8.82 (s, 1H), 8.68 - 8.45 (m, 1H), 8.32 (d, *J =* 8.0 Hz, 1H), 8.04 (dt, *J* = 8.5, 2.4 Hz, 1H), 7.91 (d, *J =* 11.9 Hz, 1H), 6.98 (d, *J =* 8.6 Hz, 1H), 5.55 (s, 2H), 5.30 (h, *J =* 6.6 Hz, 1H), 5.15 (dp, *J =* 57.8, 5.1 Hz, 1H), 4.35 (t, *J =* 6.6 Hz, 2H), 3.76 - 3.51 (m, 2H), 3.12 - 3.00 (m, 2H), 2.58 (t, *J =* 6.9 Hz, 2H), 1.78 (p, *J =* 6.7 Hz, 2H), 1.65 (d, *J =* 6.6 Hz, 6H).

### Example 24

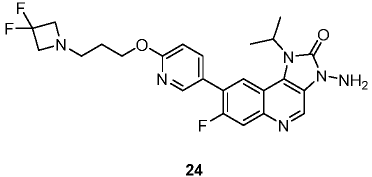

### Reaction process:

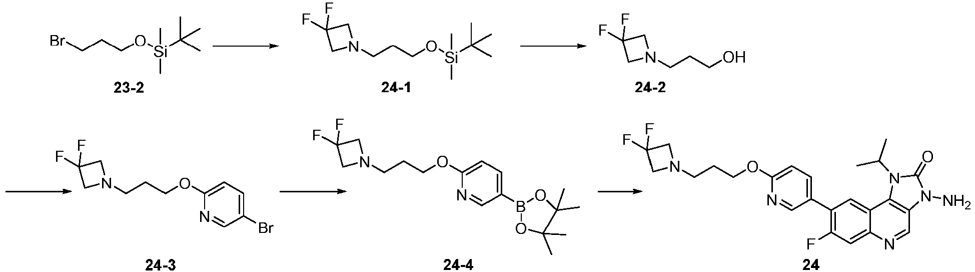

### Step A: synthesis of compound 24-1

Referring to the synthesis of Example **23,** in the preparation step of **23-3,** 3-fluoroazetidine hydrochloride was replaced with 3,3-difluoroazetidine hydrochloride to give compound **24-1.** GC-MS (EI, M⁺) *m*/*z:* 265*.*

### Step B: synthesis of compound 24-2

Referring to the synthesis of Example **23,** in the preparation step of **23-4, 23-3** was replaced with **24-1** to give compound **24-2.** GC-MS (EI, M⁺) *m*/*z:* 151.

### Step C: synthesis of compound 24-3

Referring to the synthesis of Example **23,** in the preparation step of **23-5, 23-4** was replaced with **24-2** to give compound **24-3.** MS (ESI+, [M+H]⁺) *m*/*z:* 306.87.

### Step D: synthesis of compound 24-4

Referring to the synthesis of Example **23,** in the preparation step of **23-6, 23-5** was replaced with **24-3** to give compound **24-4.** MS (ESI+, [M+H]⁺) *m*/*z:* 355.01.

### Step E: synthesis of Example 24

**2-8** (100 mg), 1,4-dioxane (6 mL), **24-4** (157 mg), potassium carbonate (119 mg) dissolved in 0.5 mL of water, 3-(di-*tert*-butylphosphino)propane-1-sulfonic acid (7.69 mg) dissolved in 0.5 mL of water, and sodium tetrachloropalladate(II) (4.21 mg) were added successively to a single-necked flask, and the mixture was heated to 80 °C and stirred for 1 h under N₂ atmosphere. After the reaction was completed, 50 mL of water was added, and dichloromethane was added for extraction. The organic phases were combined, washed with saturated brine, dried over anhydrous sodium sulfate, filtered, and concentrated under reduced pressure to dryness, and the resulting crude product was purified by silica gel column chromatography to give **Example 24** (60 mg). MS (ESI+, [M+H]⁺) *m*/*z*: 487.15. ¹H NMR (500 MHz, DMSO-*d*₆) δ 8.82 (s, 1H), 8.50 (t, *J =* 1.8 Hz, 1H), 8.32 (d, *J =* 8.2 Hz, 1H), 8.05 (dt, *J =* 8.6, 2.3 Hz, 1H), 7.91 (d, *J =* 12.1 Hz, 1H), 6.99 (d, *J =* 8.6 Hz, 1H), 5.55 (s, 2H), 5.28 (h, *J =* 6.7 Hz, 1H), 4.37 (t, *J =* 6.5 Hz, 2H), 3.58 (t, *J =* 12.5 Hz, 4H), 2.68 (dd, *J =* 7.6, 6.0 Hz, 2H), 1.81 (p, *J =* 6.7 Hz, 2H), 1.65 (d, *J =* 6.7 Hz, 6H).

### Example 25

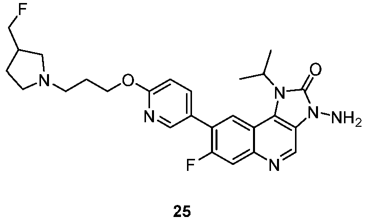

### Reaction process:

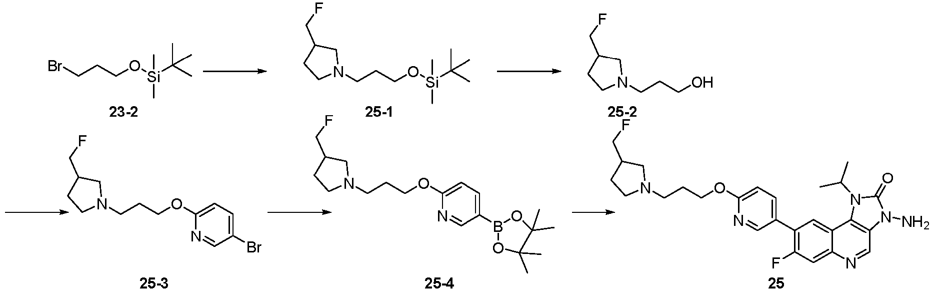

### Step A: synthesis of compound 25-1

Referring to the synthesis of Example **23,** in the preparation step of **23-3,** 3-fluoroazetidine hydrochloride was replaced with 3-(fluoromethyl)pyrrolidine to give compound **25-1.** GC-MS (EI, M⁺) *m*/*z*: 275.

### Step B: synthesis of compound 25-2

Referring to the synthesis of Example **23,** in the preparation step of **23-4, 23-3** was replaced with **25-1** to give compound **25-2.** GC-MS (EI, M⁺) *m*/*z:* 161.

### Step C: synthesis of compound 25-3

Referring to the synthesis of Example **23,** in the preparation step of **23-5, 23-4** was replaced with **25-2** to give compound **25-3.** MS (ESI+, [M+H]⁺) *m*/*z:* 316.91.

### Step D: synthesis of compound 25-4

Referring to the synthesis of Example **23,** in the preparation step of **23-6, 23-5** was replaced with **25-3** to give compound **25-4.** MS (ESI+, [M+H]⁺) *m*/*z:* 365.13.

### Step E: synthesis of Example 25

**2-8** (100 mg), 1,4-dioxane (6 mL), **25-4** (157 mg), potassium carbonate (119 mg) dissolved in 0.5 mL of water, 3-(di-*tert*-butylphosphino)propane-1-sulfonic acid (7.69 mg) dissolved in 0.5 mL of water, and sodium tetrachloropalladate(II) (4.21 mg) were added successively to a single-necked flask, and the mixture was heated to 80 °C and stirred for 1 h under N₂ atmosphere. After the reaction was completed, 50 mL of water was added, and dichloromethane was added for extraction. The organic phases were combined, washed with saturated brine, dried over anhydrous sodium sulfate, filtered, and concentrated under reduced pressure to dryness, and the resulting crude product was purified by silica gel column chromatography to give **Example 25** (40 mg). MS (ESI+, [M+H]⁺) *m*/*z*: 497.24. ¹H NMR (500 MHz, Chloroform-*d*) *δ* 8.94 (s, 1H), 8.41 (d, *J =* 2.4 Hz, 1H), 8.18 (d, *J =* 7.9 Hz, 1H), 7.93 - 7.84 (m, 2H), 6.89 (d, *J =* 8.6 Hz, 1H), 5.31 - 5.13 (m, 1H), 4.50 (s, 2H), 4.44 (t, *J =* 6.4 Hz, 2H), 4.40 - 4.37 (m, 1H), 4.31 - 4.28 (m, 1H), 2.80 - 2.51 (m, 6H), 2.47 (dd, *J=* 9.3, 5.4 Hz, 1H), 2.09 - 2.01 (m, 2H), 1.97 (ddd, *J =* 13.2, 9.7, 6.6 Hz, 1H), 1.76 (d, *J =* 6.9 Hz, 6H), 1.57 - 1.46 (m, 1H).

### Example 26

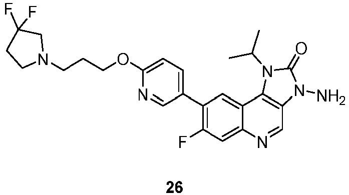

### Reaction process:

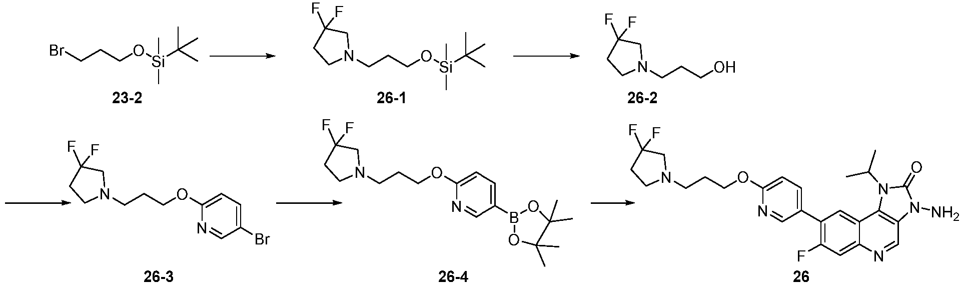

### Step A: synthesis of compound 26-1

Referring to the synthesis of Example **23,** in the preparation step of **23-3,** 3-fluoroazetidine hydrochloride was replaced with 3,3-difluoropyrrolidine hydrochloride to give compound **26-1.** GC-MS (EI, M⁺) *m*/*z:* 279.

### Step B: synthesis of compound 26-2

Referring to the synthesis of Example **23,** in the preparation step of **23-4, 23-3** was replaced with **26-1** to give compound **26-2.** GC-MS (EI, M⁺) *m*/*z:* 165.

### Step C: synthesis of compound 26-3

Referring to the synthesis of Example **23,** in the preparation step of **23-5, 23-4** was replaced with **26-2** to give compound **26-3.** MS (ESI+, [M+H]⁺) *m*/*z:* 320.86.

### Step D: synthesis of compound 26-4

Referring to the synthesis of Example **23,** in the preparation step of **23-6, 23-5** was replaced with **26-3** to give compound **26-4.** MS (ESI+, [M+H]⁺) *m*/*z:* 369.02.

### Step E: synthesis of Example 26

**2-8** (100 mg), 1,4-dioxane (6 mL), **26-4** (157 mg), potassium carbonate (119 mg) dissolved in 0.5 mL of water, 3-(di-*tert*-butylphosphino)propane-1-sulfonic acid (7.69 mg) dissolved in 0.5 mL of water, and sodium tetrachloropalladate(II) (4.21 mg) were added successively to a single-necked flask, and the mixture was heated to 80 °C and stirred for 1 h under N₂ atmosphere. After the reaction was completed, 50 mL of water was added, and dichloromethane was added for extraction. The organic phases were combined, washed with saturated brine, dried over anhydrous sodium sulfate, filtered, and concentrated under reduced pressure to dryness, and the resulting crude product was purified by silica gel column chromatography to give **Example 26** (40 mg). MS (ESI+, [M+H]⁺) *m*/*z*: 501.15. ¹H NMR (500 MHz, Chloroform-d) *δ* 8.94 (s, 1H), 8.41 (d, *J =* 2.4 Hz, 1H), 8.18 (d, *J =* 7.9 Hz, 1H), 8.03 - 7.85 (m, 2H), 6.89 (d, *J =* 8.6 Hz, 1H), 5.29 - 5.08 (m, 1H), 4.50 (s, 2H), 4.45 (t, *J =* 6.4 Hz, 2H), 2.94 (t, *J =* 13.3 Hz, 2H), 2.78 (t, *J =* 6.9 Hz, 2H), 2.67 (t, *J =* 7.3 Hz, 2H), 2.29 (tt, *J =* 14.6, 6.9 Hz, 2H), 2.02 (p, *J =* 6.6 Hz, 2H), 1.76 (d, *J =* 7.0 Hz, 6H).

### Example 27

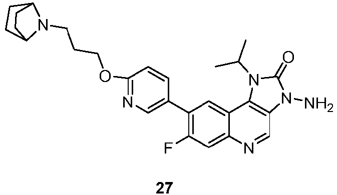

### Reaction process:

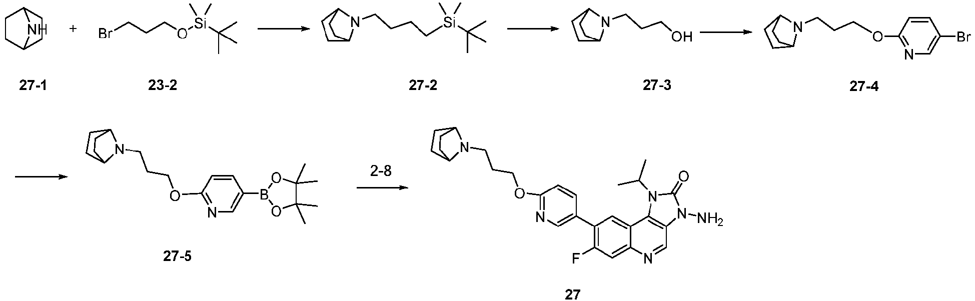

### Step A: synthesis of compound 27-2

**27-1** (1.0 g), **23-2** (1.0 g), potassium carbonate (1.6 g), potassium iodide (0.33 g), and acetonitrile (15 mL) were added successively to a single-necked flask, and the mixture was heated to 60 °C and left to react. After the reaction was completed, 50 mL of water was added, and dichloromethane was added for extraction. The organic phase was washed with a saturated sodium chloride solution, dried over anhydrous sodium sulfate, and filtered, and the filtrate was concentrated to give intermediate **27-2** (1.0 g), which was directly used in the next step without purification. MS (ESI+, [M+H]⁺) *m*/*z*: 270.23.

### Step B: synthesis of compound 27-3

**27-2** (1.0 g) and a solution of hydrochloric acid in ethyl acetate (1 M, 20 mL) were added successively to a single-necked flask, and the mixture was left to react at room temperature. After the reaction was completed, the reaction system was directly concentrated to give intermediate **27-3** (0.5 g). MS (ESI+, [M+H]⁺) *m*/*z*: 156.14.

### Step C: synthesis of compound 27-4

**27-3** (0.5 g), THF (15 mL), and 5-bromo-2-fluoropyridine (0.68 g) were added successively to a single-necked flask, and then sodium hydride (60wt%, 0.39 g) was added under an ice bath. The mixture was left to react at room temperature. After the reaction was completed, an icy saturated ammonium chloride solution was added to the system, and DCM was added for extraction. The organic phases were combined, washed with saturated brine, dried over anhydrous sodium sulfate, and filtered, and the filtrate was concentrated to dryness. The resulting crude product was purified by silica gel column chromatography (DCM/MeOH) to give intermediate **27-4** (326 mg). MS (ESI+, [M+H]⁺) *m*/*z*: 310.91.

### Step D: synthesis of compound 27-5

**27-4** (326 mg), THF (10 mL) and isopropanol pinacol borate (253 mg) were added successively to a single-necked flask, and the mixture was cooled to -78 °C under nitrogen atmosphere. A solution of *n*-butyllithium in *n*-hexane (2.5 M, 1.25 mL) was then added dropwise. After the dropwise addition was completed, the mixture was left to react for 1 h with the temperature maintained at this temperature, and then the mixture was allowed to return to room temperature and left to react. After the reaction was completed, saturated ammonium chloride was added to the system under an ice bath to quench the reaction. 50 mL of water was added for dilution, and EA was added for extraction. The organic phases were combined, washed with saturated brine, dried over anhydrous sodium sulfate, and filtered, and the filtrate was concentrated to give intermediate **27-5** (330 mg). MS (ESI+, [M+H]⁺) *m*/*z*: 359.13.

### Step E: synthesis of Example 27

**27-5** (330 mg), 1,4-dioxane (10 mL), water (2.5 mL), intermediate **2-8** (150 mg), potassium carbonate (183 mg), 3-(di-*tert*-butylphosphino)propane-1-sulfonic acid (2.4 mg), and sodium tetrachloropalladate(II) (1.3 mg) were added successively to a single-necked flask, and the mixture was left to react at 80 °C under nitrogen atmosphere. After the reaction was completed, 20 mL of water was added, and dichloromethane was added for extraction. The organic phases were combined, washed with saturated brine, dried over anhydrous sodium sulfate, filtered, and concentrated under reduced pressure to dryness, and the resulting crude product was subjected to silica gel column chromatography (DCM/MeOH) to give **Example 27** (120 mg). MS (ESI+, [M+H]⁺) *m*/*z:* 491.26. ¹H NMR (500 MHz, DMSO-d6) δ 8.82 (s, 1H), 8.54 - 8.49 (m, 1H), 8.32 (d, J = 8.2 Hz, 1H), 8.07 (dt, J = 8.5, 2.3 Hz, 1H), 7.92 (d, J = 12.1 Hz, 1H), 7.00 (d, J = 8.5 Hz, 1H), 5.56 (s, 2H), 5.29 (p, J = 6.8 Hz, 1H), 4.42 (t, J = 6.3 Hz, 2H), 4.10 - 3.54 (m, 2H), 3.07 - 2.68 (m, 2H), 2.18 - 1.99 (m, 2H), 1.92 - 1.85 (m, 4H), 1.65 (d, J = 6.7 Hz, 6H), 1.56 - 1.44 (m, 4H).

### Example 28

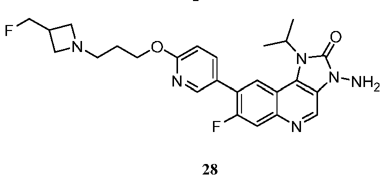

### Reaction process:

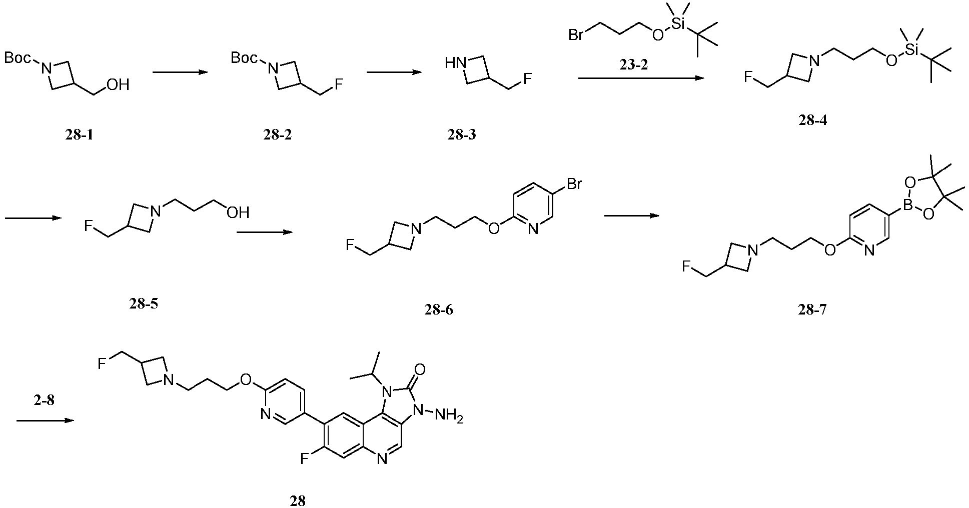

### Step A: synthesis of compound 28-2

**28-1** (5.0 g) and DCM (50 mL) were added successively to a single-necked flask, and then diethylaminosulfur trifluoride (8.61 g) was added dropwise and slowly under an ice bath. After the dropwise addition was completed, the mixture was left to react at room temperature. After the reaction was completed, saturated sodium bicarbonate was added dropwise and slowly to quench the reaction. 200 mL of water was added, and DCM was added for extraction. The organic phase was washed with saturated brine, dried over anhydrous sodium sulfate, and filtered, and the filtrate was concentrated to give intermediate **28-2** (5.0 g), which was directly used in the next step without purification. GC-MS (EI, M⁺) *m*/*z:* 189.

### Step B: synthesis of compound 28-3

**28-2** (5.0 g) and a solution of hydrochloric acid in 1,4-dioxane (4 M, 50 mL) were added successively to a single-necked flask, and the mixture was left to react at room temperature. After the reaction was completed, the system was directly concentrated to give intermediate **28-3** (5.0 g). GC-MS (EI, M⁺) *m*/*z:* 89.

### Step C: synthesis of compound 28-4

**28-3** (5.0 g), **23-2** (10.0 g), potassium carbonate (16.37 g), potassium iodide (3.28 g), and acetonitrile (150 mL) were added successively to a single-necked flask, and the mixture was heated to 60 °C and left to react. After the reaction was completed, 500 mL of water was added, and dichloromethane was added for extraction. The organic phase was washed with a saturated sodium chloride solution, dried over anhydrous sodium sulfate, and filtered, and the filtrate was concentrated to give intermediate **28-4** (5.0 g), which was directly used in the next step without purification. GC-MS (EI, M⁺) *m*/*z:* 261.

### Step D: synthesis of compound 28-5

**28-4** (5.0 g) and a solution of hydrochloric acid in 1,4-dioxane (4 M, 50 mL) were added successively to a single-necked flask, and the mixture was left to react at room temperature. After the reaction was completed, the system was directly concentrated to give intermediate **28-5** (3.0 g). GC-MS (EI, M⁺) *m*/*z:* 147.

### Step E: synthesis of compound 28-6

**28-5** (3.0 g), THF (100 mL), and 5-bromo-2-fluoropyridine (2.43 g) were added successively to a single-necked flask, and then sodium hydride (60wt%, 1.38 g) was added under an ice bath. The mixture was left to react at room temperature. After the reaction was completed, an icy saturated ammonium chloride solution (500 mL) was added to the reaction system, and DCM was added for extraction. The organic phases were combined, washed with saturated brine, dried over anhydrous sodium sulfate, and filtered, and the filtrate was concentrated to dryness. The resulting crude product was purified by silica gel column chromatography (DCM/MeOH) to give intermediate **28-6** (0.4 g). MS (ESI+, [M+H]⁺) *m*/*z:* 302.89.

### Step F: synthesis of compound 28-7

**28-6** (0.4 g), THF (10 mL), and isopropanol pinacol borate (319 mg) were added successively to a single-necked flask, and the mixture was cooled to -78 °C under nitrogen atmosphere. A solution of *n*-butyllithium in *n*-hexane (2.5 M, 0.8 mL) was then added dropwise. After the dropwise addition was completed, the mixture was left to react for 1 h with the temperature maintained at this temperature, and then the mixture was allowed to return to room temperature and left to react. After the reaction was completed, saturated ammonium chloride was added to the system under an ice bath to quench the reaction. 50 mL of water was added for dilution, and EA was added for extraction. The organic phases were combined, washed with saturated brine, dried over anhydrous sodium sulfate, and filtered, and the filtrate was concentrated to give intermediate **28-7** (400 mg). MS (ESI+, [M+H]⁺) *m*/*z:* 351.10.

### Step G: synthesis of Example 28

**28-7** (232 mg), 1,4-dioxane (10 mL), water (2.5 mL), intermediate **2-8** (150 mg), potassium carbonate (183 mg), 3-(di-*tert*-butylphosphino)propane-1-sulfonic acid (2.4 mg), and sodium tetrachloropalladate(II) (1.3 mg) were added successively to a single-necked flask, and the mixture was left to react at 80 °C under nitrogen atmosphere. After the reaction was completed, 20 mL of water was added, and dichloromethane was added for extraction. The organic phases were combined, washed with saturated brine, dried over anhydrous sodium sulfate, filtered, and concentrated under reduced pressure to dryness, and the resulting crude product was subjected to silica gel column chromatography (DCM/MeOH) to give **Example 28** (90 mg). MS (ESI+, [M+H]+) *m*/*z:* 483.17. ¹H NMR (500 MHz, DMSO-*d*6) δ 8.82 (s, 1H), 8.50 (s, 1H), 8.32 (d, J = 8.1 Hz, 1H), 8.04 (dt, J = 8.5, 2.3 Hz, 1H), 7.91 (d, J = 11.9 Hz, 1H), 6.98 (d, J = 8.5 Hz, 1H), 5.55 (s, 2H), 5.34 - 5.24 (m, 1H), 4.51 (dd, J = 47.6, 6.1 Hz, 2H), 4.34 (t, J = 6.6 Hz, 2H), 3.29 (d, J = 7.3 Hz, 2H), 2.96 (t, J = 6.8 Hz, 2H), 2.78 - 2.67 (m, 1H), 2.54 (d, J = 7.0 Hz, 2H), 1.80 - 1.70 (m, 2H), 1.64 (d, J = 6.7 Hz, 6H).

### Example 29

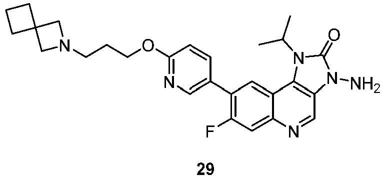

### Reaction process:

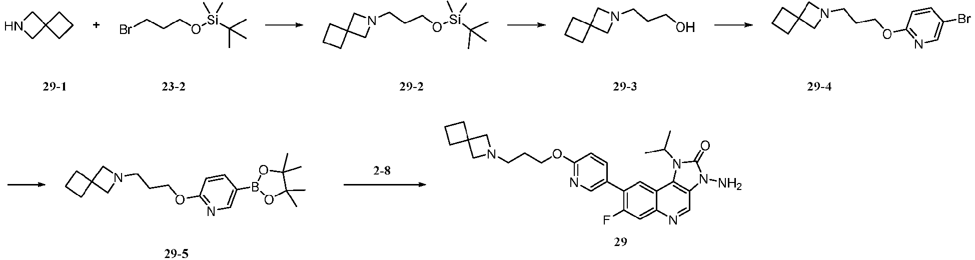

### Step A: synthesis of compound 29-2

**29-1** (0.20 g), **23-2** (0.38 g), potassium carbonate (0.62 g), potassium iodide (0.13 g), and acetonitrile (15 mL) were added successively to a single-necked flask, and the mixture was heated to 60 °C and left to react. After the reaction was completed, 50 mL of water was added, and dichloromethane was added for extraction. The organic phase was washed with a saturated sodium chloride solution, dried over anhydrous sodium sulfate, and filtered, and the filtrate was concentrated to give intermediate **29-2** (0.15 g), which was directly used in the next step without purification. GC-MS (EI, M⁺) *m*/*z:* 269.

### Step B: synthesis of compound 29-3

**29-2** (0.15 g) and a solution of hydrochloric acid in ethyl acetate (1 M, 10 mL) were added successively to a single-necked flask, and the mixture was left to react at room temperature. After the reaction was completed, the system was directly concentrated to give intermediate **29-3** (0.12 g). GC-MS (EI, M⁺) *m*/*z:* 155.

### Step C: synthesis of compound 29-4

**29-3** (0.12 g), THF (10 mL), and 5-bromo-2-fluoropyridine (0.14 g) were added successively to a single-necked flask, and then sodium hydride (60wt%, 0.08 g) was added under an ice bath. The mixture was left to react at room temperature. After the reaction was completed, an icy saturated ammonium chloride solution was added to the system, and DCM was added for extraction. The organic phases were combined, washed with saturated brine, dried over anhydrous sodium sulfate, and filtered, and the filtrate was concentrated to dryness. The resulting crude product was purified by silica gel column chromatography (DCM/MeOH) to give intermediate **29-4** (120 mg). MS (ESI+, [M+H]⁺) *m*/*z*: 310.92.

### Step D: synthesis of compound 29-5

**29-4** (120 mg), THF (10 mL), and isopropanol pinacol borate (93 mg) were added successively to a single-necked flask, and the mixture was cooled to -78 °C under nitrogen atmosphere. A solution of *n*-butyllithium in *n*-hexane (2.5 M, 0.23 mL) was then added dropwise. After the dropwise addition was completed, the mixture was left to react for 1 h with the temperature maintained at this temperature, and then the mixture was allowed to return to room temperature and left to react. After the reaction was completed, saturated ammonium chloride was added to the system under an ice bath to quench the reaction. 50 mL of water was added for dilution, and EA was added for extraction. The organic phases were combined, washed with saturated brine, dried over anhydrous sodium sulfate, and filtered, and the filtrate was concentrated to give intermediate **29-5** (130 mg). MS (ESI+, [M+H]⁺) *m*/*z*: 359.12.

### Step E: synthesis of Example 29

**29-5** (130 mg), 1,4-dioxane (10 mL), water (2.5 mL), intermediate **2-8**(150 mg), potassium carbonate (122 mg), 3-(di-*tert*-butylphosphino)propane-1-sulfonic acid (1.58 mg), and sodium tetrachloropalladate(II) (0.87 mg) were added successively to a single-necked flask, and the mixture was left to react at 80 °C under nitrogen atmosphere. After the reaction was completed, 20 mL of water was added, and dichloromethane was added for extraction. The organic phases were combined, washed with saturated brine, dried over anhydrous sodium sulfate, filtered, and concentrated under reduced pressure to dryness, and the resulting crude product was subjected to silica gel column chromatography (DCM/MeOH) to give **Example 29** (55 mg). MS (ESI+, [M+H]⁺) *m*/*z:* 491.26. ¹H NMR (500 MHz, DMSO-d6) δ 8.82 (s, 1H), 8.52 - 8.44 (m, 1H), 8.32 (d, J = 8.2 Hz, 1H), 8.04 (dt, J = 8.5, 2.3 Hz, 1H), 7.91 (d, J = 12.1 Hz, 1H), 6.97 (d, J = 8.5 Hz, 1H), 5.55 (s, 2H), 5.34 - 5.21 (m, 1H), 4.32 (t, J = 6.6 Hz, 2H), 3.07 (s, 4H), 2.44 (t, J = 7.0 Hz, 2H), 2.02 (t, J = 7.6 Hz, 4H), 1.79 - 1.68 (m, 4H), 1.64 (d, J = 6.9 Hz, 6H).

### Example 30

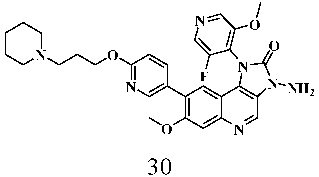

### Reaction process:

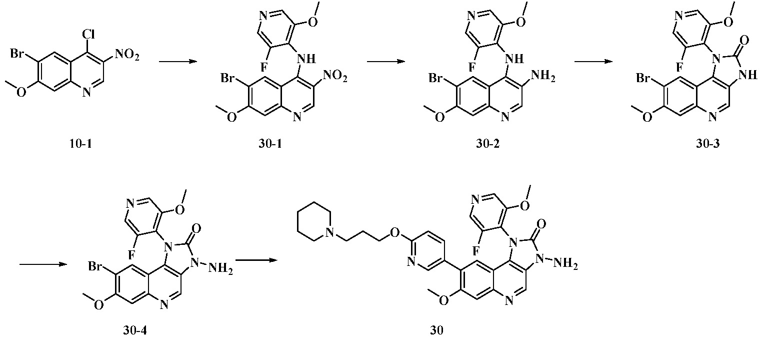

### Step A: synthesis of compound 30-1

3-Fluoro-5-methoxypyridin-4-amine (1.34 g) and DMF (20 mL) were added successively to a single-necked flask, and then 60wt% sodium hydride (1.26 g) was added slowly at 0 °C under nitrogen atmosphere. After the mixture was stirred for 10 min, **10-1** (2.0 g) was added. The mixture was left to react at room temperature for 20 min. After the reaction was completed, ice water (100 mL) was added, and the mixture was stirred for 10 min and filtered. The filter cake was collected and washed with water (5 mL × 3) and petroleum ether (5 mL × 2) successively to give intermediate **30-1** (2.5 g). MS (ESI+, [M+H]⁺) *m*/*z:* 422.95.

### Step B: synthesis of compound 30-2

**30-1** (2.5 g), DCM (50 mL), acetic acid (20 mL), and iron powder (1.65 g) were added successively to a single-necked flask, and the mixture was left to react at room temperature for 1 h under nitrogen atmosphere. After the reaction was completed, the reaction solution was filtered. The filtrate was concentrated to dryness, and the residue was adjusted to pH 8-9 with a saturated sodium bicarbonate solution (25 mL), followed by extraction with DCM (40 mL × 5). The organic phases were combined, washed with saturated brine (50 mL), dried over anhydrous sodium sulfate, and filtered, and the filtrate was concentrated to give intermediate **30-2** (1.8 g). MS (ESI+, [M+H]⁺) *m*/*z:* 392.93.

### Step C: synthesis of compound 30-3

**30-2** (1.8 g), THF (40 mL), DIPEA (2.86 g), and CDI (2.28 g) were added successively to a single-necked flask, and the mixture was left to react at room temperature for 4 h under nitrogen atmosphere. After the reaction was completed, ice water (200 mL) was added to the reaction solution, and the reaction solution was filtered. The filter cake was collected, washed with water (10 mL × 3) and petroleum ether (10 mL × 2) successively, and dried under reduced pressure to give intermediate **30-3** (1.2 g). MS (ESI+, [M+H]⁺) *m*/*z:* 419.06.

### Step D: synthesis of compound 30-4

**30-3** (1.0 g), DCM (30 mL), water (15 mL), TBAB (77 mg), 2,4-dinitrophenylhydroxylamine (712 mg), and NaOH (191 mg) were added successively to a single-necked flask, and the mixture was stirred at room temperature for 24 h under N₂ atmosphere. After the reaction was completed, the reaction solution was concentrated to remove the organic solvent, and then filtered. The filter cake was slurried with a mixed solvent of acetonitrile:water (1: 1, 10 mL) and dried under vacuum to give intermediate **30-4** (0.8 g). MS (ESI+, [M+H]⁺) *m*/*z:* 433.95.

### Step E: synthesis of Example 30

**30-4** (100 mg), 1,4-dioxane (5 mL), water (2 mL), **A-3** (130 mg), potassium carbonate (95 mg), 3-(di-*tert*-butylphosphino)propane-1-sulfonic acid (1.24 mg), and sodium tetrachloropalladate(II) (0.68 mg) were added successively to a single-necked flask, and the mixture was stirred at 80 °C for 1 h under nitrogen atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by silica gel column chromatography to give **Example 30** (40.0 mg). HRMS (ESI+, [M+H]⁺) *m*/*z:* 574.2573. ¹H NMR (500 MHz, DMSO-d6) *δ* 8.86 (s, 1H), 8.69 (s, 1H), 8.65 (s, 1H), 7.99 (d, J = 2.6 Hz, 1H), 7.66 (dd, J = 8.5, 2.6 Hz, 1H), 7.58 (s, 1H), 6.93 (s, 1H), 6.81 (d, J = 8.7 Hz, 1H), 5.80 (s, 2H), 4.28 (t, J = 6.6 Hz, 2H), 3.91 (s, 3H), 3.87 (s, 3H), 2.45 - 2.28 (m, 6H), 1.92 - 1.84 (m, 2H), 1.55 - 1.47 (m, 4H), 1.43 - 1.36 (m, 2H).

### Example 31

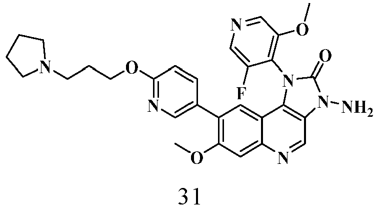

### Reaction process:

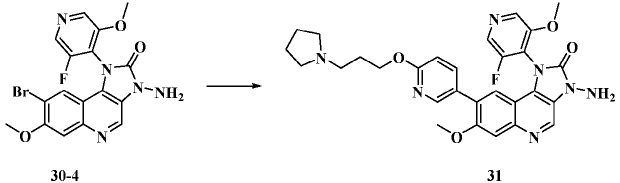

**30-4** (100 mg), 1,4-dioxane (5 mL), water (2 mL), **A-6** (133 mg), potassium carbonate (95 mg), 3-(di-*tert*-butylphosphino)propane-1-sulfonic acid (1.24 mg), and sodium tetrachloropalladate(II) (0.68 mg) were added successively to a single-necked flask, and the mixture was stirred at 80 °C for 1 h under nitrogen atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by silica gel column chromatography to give **Example 31** (37.0 mg). HRMS (ESI+, [M+H]⁺) *m*/*z:* 560.2412.

¹ H NMR (500 MHz, DMSO-d6) δ 8.86 (s, 1H), 8.69 (s, 1H), 8.65 (s, 1H), 7.99 (d, J = 2.4 Hz, 1H), 7.66 (dd, J = 8.6, 2.5 Hz, 1H), 7.58 (s, 1H), 6.93 (s, 1H), 6.82 (d, J = 8.5 Hz, 1H), 5.80 (s, 2H), 4.30 (t, J = 6.6 Hz, 2H), 3.91 (s, 3H), 3.87 (s, 3H), 2.55 - 2.51 (m, 2H), 2.48 - 2.45 (m, 2H), 1.92 - 1.86 (m, 2H), 1.72 - 1.66 (m, 4H).

### Example 32

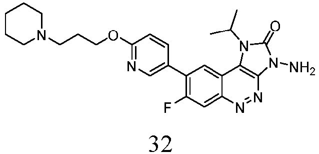

### Reaction process:

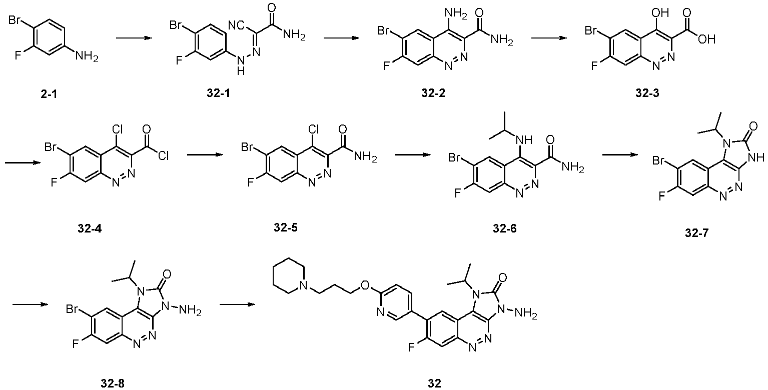

### Step A: synthesis of compound 32-1

**2-1** (3.0 g), water (50 mL), concentrated hydrochloric acid (26.3 mL), and acetic acid (18.08 mL) were added successively to a three-necked flask, and then a solution of sodium nitrite (1.20 g) in water (30 mL) was added dropwise at 0 °C. After the dropwise addition was completed, the mixture was left to react for 2 h with the temperature maintained at 0 °C. Then, sodium acetate (10.36 g) was added in portions with the temperature maintained at this temperature. After the addition was completed, the mixture was stirred for 30 min with the temperature maintained at this temperature. The system was marked as solution a. 2-Cyanoacetamide (1.6 g), ethanol (50 mL), and water (200 mL) were added to a single-necked flask, and then sodium acetate (51.8 g) was added with the temperature maintained at 0 °C. After complete dissolving, the mixture was left to react for 30 min with the temperature maintained at this temperature to obtain solution b. The solution a was added dropwise to the solution b at 0 °C. After the dropwise addition was completed, the mixture was left to react for 2 h with the temperature maintained at this temperature. After the reaction was completed, the system was left to stand and then filtered under vacuum, and the filter cake was washed with water (10 mL × 5) and dried under reduced pressure to give intermediate **32-1** (4.3 g).

### Step B: synthesis of compound 32-2

**32-1** (4.0 g), orthodichlorobenzene (50 mL), and aluminum trichloride (5.61 g) were added successively to a three-necked flask, and the mixture was heated to 120 °C and left to react for 24 h under N₂ atmosphere. After the reaction was completed, the reaction solution was cooled, and 300 mL of water was added under an ice bath to quench the reaction. The reaction solution was stirred for 30 min and filtered, and the filter cake was washed with 100 mL of water, then washed with 20 mL of acetonitrile, then washed with 10 mL of petroleum ether, and dried to give intermediate **32-2** (4.0 g). MS (ESI+, [M+H]⁺) *m*/*z:* 284.78.

### Step C: synthesis of compound 32-3

**32-2** (4.0 g), DMSO (20 mL), and 4 M sulfuric acid (20 mL) were added successively to a single-necked flask, and the mixture was left to react at 130 °C for 30 h under N₂ atmosphere. After the reaction was completed, the reaction solution was cooled under an ice bath, and water (150 mL) was added. The reaction solution was filtered, and the filter cake was washed with water (10 mL × 3) and dried under vacuum to give intermediate **32-3** (3.0 g). MS (ESI-, [M+H]⁻) *m*/*z:* 284.94.

### Step D: synthesis of compound 32-4

**32-3** (3.0 g), thionyl chloride (30 mL), and DMF (0.06 g) were added successively to a single-necked flask, and the mixture was heated to reflux and left to react for 2 h under N₂ atmosphere. After the reaction was completed, the reaction solution was cooled and concentrated under reduced pressure, and 100 mL of toluene was added. The mixture was subjected to ultrasonic treatment and concentrated again, and the steps were repeated twice to give intermediate **32-4** (3.5 g), which was directly used in the next step.

### Step E: synthesis of compound 32-5

**32-4** (3.5 g) and acetonitrile (50 mL) were added successively to a single-necked flask, followed by ultrasonic dispersion. 25% ammonium hydroxide solution (10 mL) was then added dropwise at 0 °C. After the dropwise addition was completed, the mixture was stirred vigorously for 30 min and filtered, and the filter cake was washed with water (5 mL × 3) and dried under vacuum to give intermediate **32-5** (2.1 g).

### Step F: synthesis of compound 32-6

**32-5** (2.1 g), DIPEA (1.783 g), acetonitrile (30 mL), and isopropylamine (0.408 g) were added successively to a single-necked flask, and the mixture was stirred at 80 °C for 5 h under N₂ atmosphere. After the reaction was completed, the reaction solution was cooled, and 100 mL of water was added. The reaction solution was filtered, and the filter cake was washed with water (5 mL × 4) and dried under vacuum to give intermediate **32-6** (2.26 g). MS (ESI+, [M+H]⁺) *m*/*z:* 326.87.

### Step G: synthesis of compound 32-7

**32-6** (2.26 g), DBU (2.103 g), and methanol (20 mL) were added successively to a single-necked flask, and then 1,3,5-trichloro-1,3,5-triazine-2,4,6-trione (0.803 g) was added at 5 °C. The mixture was stirred at room temperature for 1 h under N₂ atmosphere. After the reaction was completed, 50 mL of water was added, and the reaction solution was stirred for 10 min and filtered. The filter cake was washed with water (5 mL × 3) and dried under vacuum to give intermediate **32-7** (2.0 g). MS (ESI+, [M+H]⁺) *m*/*z*: 324.83.

### Step H: synthesis of compound 32-8

**32-7** (1.3 g), DCM (40 mL), water (20 mL), TBAB (198 mg), 2,4-dinitrophenylhydroxylamine (0.919 g), and NaOH (345 mg) were added successively to a single-necked flask, and the mixture was left to react at room temperature for 24 h under N₂ atmosphere. After the reaction was completed, 100 mL of water was added, and DCM (20 mL × 5) was added for extraction. The organic phases were combined, washed with saturated brine, dried over anhydrous sodium sulfate, filtered, and concentrated under reduced pressure, and the resulting crude product was purified by silica gel column chromatography to give intermediate **32-8** (580 mg). MS (ESI+, [M+H]⁺) m/z: 339.89.

### Step I: synthesis of Example 32

**32-8** (80 mg), 1,4-dioxane (5 mL), water (2 mL), **A-3** (163 mg), potassium carbonate (98 mg), 3-(di-*tert*-butylphosphino)propane-1-sulfonic acid (1.26 mg), and sodium tetrachloropalladate(II) (0.69 mg) were added successively to a single-necked flask, and the mixture was stirred at 80 °C for 1 h under N₂ atmosphere. After the reaction was completed, the reaction solution was concentrated, and the resulting residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 32** (40.0 mg). HRMS (ESI+, [M+H]⁺) *m*/*z:* 480.2517. ¹H NMR (500 MHz, DMSO-d6) δ 8.53 (t, *J* = 2.0 Hz, 1H), 8.35 (d, *J* = 7.8 Hz, 1H), 8.21 (d, *J* = 11.4 Hz, 1H), 8.07 (dt, *J* = 8.5, 2.3 Hz, 1H), 7.00 (d, *J* = 8.7 Hz, 1H), 5.59 (s, 2H), 5.26 (p, *J* = 6.8 Hz, 1H), 4.37 (t, *J* = 6.6 Hz, 2H), 2.39 (t, *J* = 7.2 Hz, 2H), 2.34 - 2.31 (m, 4H), 1.91 (p, *J* = 6.7 Hz, 2H), 1.64 (d, *J =* 6.7 Hz, 6H), 1.52 - 1.46 (m, 4H), 1.40 - 1.35 (m, 2H).

### Example 33

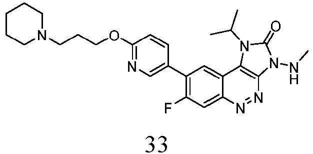

### Reaction process:

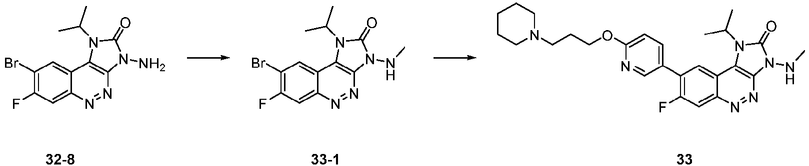

### Step A: synthesis of compound 33-1

**32-8** (0.36 mg), THF (5 mL), NaOH (200 mg), and CH₃I (0.9 g) were added successively to a single-necked flask, and the mixture was left to react at room temperature for 1 h under N₂ atmosphere. After the reaction was completed, 50 mL of water was added to the system, and EA (15 mL × 3) was added for extraction. The organic phase was washed with saturated brine, dried over anhydrous sodium sulfate, filtered, and concentrated, and the resulting crude product was purified by silica gel column chromatography to give intermediate **33-1** (120 mg). MS (ESI+, [M+H]⁺) m/z: 353.96.

### Step B: synthesis of Example 33

**33-1** (65 mg), 1,4-dioxane (5 mL), water (2 mL), **A-3** (127 mg), potassium carbonate (76 mg), 3-(di-*tert*-butylphosphino)propane-1-sulfonic acid (1.00 mg), and sodium tetrachloropalladate(II) (0.5 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 33** (29 mg). HRMS (ESI+, [M+H]⁺) *m*/*z:* 494.2683. ¹ H NMR (500 MHz, DMSO-d6) δ 8.53 (t, *J* = 2.1 Hz, 1H), 8.35 (d, *J* = 7.8 Hz, 1H), 8.21 (d, *J* = 11.4 Hz, 1H), 8.07 (dt, *J* = 8.5, 2.4 Hz, 1H), 7.00 (d, *J =* 8.5 Hz, 1H), 6.31 (q, *J =* 5.6 Hz, 1H), 5.30 - 5.20 (m, 1H), 4.37 (t, *J =* 6.6 Hz, 2H), 2.83 (d, *J* = 5.5 Hz, 3H), 2.39 (t, *J* = 7.2 Hz, 2H), 2.36 - 2.30 (m, 4H), 1.94 - 1.87 (m, 2H), 1.64 (d, *J* = 6.9 Hz, 6H), 1.52 - 1.47 (m, 4H), 1.42 - 1.34 (m, 2H).

### Example 34

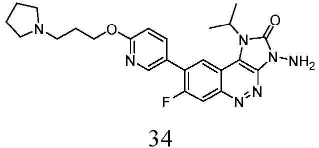

### Reaction process:

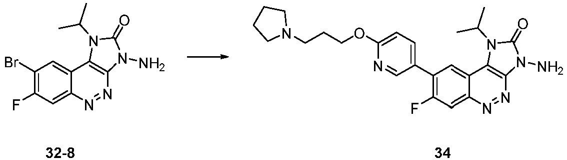

### Step A: synthesis of Example 34

**32-8** (65 mg), 1,4-dioxane (5 mL), water (2 mL), **A-6** (127 mg), potassium carbonate (76 mg), 3-(di-*tert*-butylphosphino)propane-1-sulfonic acid (1.00 mg), and sodium tetrachloropalladate(II) (0.5 mg) were added successively to a single-necked flask, and the mixture was stirred at 80 °C for 1 h under N₂ atmosphere. The reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution:acetonitrile = 60:40) to give **Example 34** (49 mg). HRMS (ESI+, [M+H]⁺) *m*/*z:* 466.2368. ¹ 1H NMR (500 MHz, DMSO-d6) δ 8.56 - 8.50 (m, 1H), 8.35 (d, *J* = 7.8 Hz, 1H), 8.20 (d, *J =* 11.4 Hz, 1H), 8.07 (dt, *J* = 8.5, 2.4 Hz, 1H), 7.00 (d, *J =* 8.7 Hz, 1H), 5.58 (s, 2H), 5.26 (p, *J =* 6.8 Hz, 1H), 4.39 (t, *J =* 6.6 Hz, 2H), 2.54 (t, *J* = 7.2 Hz, 2H), 2.44 (dq, *J* = 7.6, 3.8 Hz, 4H), 1.94 (q, *J* = 6.9 Hz, 2H), 1.71 - 1.67 (m, 4H), 1.64 (d, J = 6.7 Hz, 6H).

### Example 35

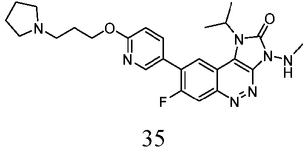

### Reaction process:

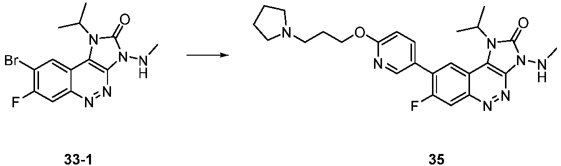

### Step A: synthesis of Example 35

**33-1** (55 mg), 1,4-dioxane (5 mL), water (2 mL), **A-6** (103 mg), potassium carbonate (65 mg), 3-(di-*tert*-butylphosphino)propane-1-sulfonic acid (0.83 mg), and sodium tetrachloropalladate(II) (0.45 mg) were added successively to a single-necked flask, and the mixture was left to react at 80 °C for 1 h under N₂ atmosphere. After the reaction was completed, the reaction solution was concentrated, and the resulting residue was dissolved in DCM (50 mL). The resulting solution was washed with saturated brine (2 × 10 mL), dried over anhydrous sodium sulfate, filtered, and concentrated to give the crude product, which was purified by preparative high-pressure chromatography (column: Polar RP (10 µm, 21.2 × 250 mm); mobile phase: 10 mM ammonium acetate-0.1% aqueous acetic acid solution: acetonitrile = 60:40) to give **Example 35** (6 mg). HRMS (ESI+, [M+H]⁺) *m*/*z:* 480.2521. ¹H NMR (500 MHz, DMSO-d6) δ 8.53 (t, J = 2.0 Hz, 1H), 8.35 (d, *J* = 7.8 Hz, 1H), 8.21 (d, *J* = 11.4 Hz, 1H), 8.07 (dt, *J* = 8.7, 2.4 Hz, 1H), 7.00 (d, *J* = 8.8 Hz, 1H), 6.31 (q, *J* = 5.6 Hz, 1H), 5.31 - 5.17 (m, 1H), 4.39 (t, *J* = 6.6 Hz, 2H), 2.83 (d, *J* = 5.5 Hz, 3H), 2.54 (t, *J* = 7.2 Hz, 2H), 2.48 - 2.40 (m, 4H), 1.93 (p, *J* = 6.8 Hz, 2H), 1.71 - 1.67 (m, 4H), 1.64 (d, *J* = 6.7 Hz, 6H).

### Example 36

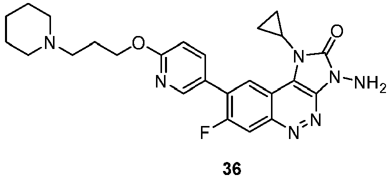

### Reaction process:

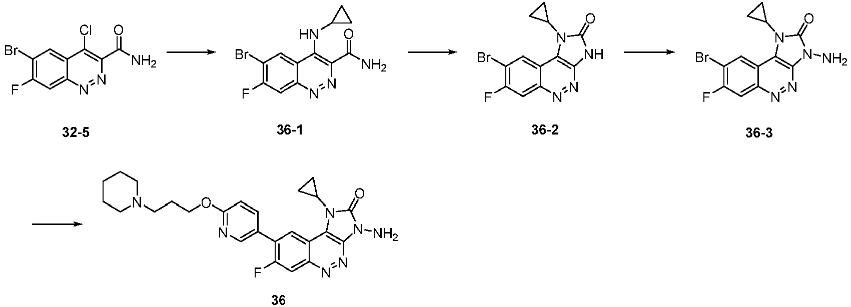

### Step A: synthesis of compound 36-1

**32-5** (5.0 g), DIPEA (6.37 g), acetonitrile (100 mL), and cyclopropylamine (1.88 g) were added successively to a single-necked flask, and the mixture was left to react at 95 °C for 4 h under N₂ atmosphere. After the reaction was completed, the reaction solution was cooled, and 150 mL of water was added. The reaction solution was filtered, and the filter cake was washed with water (30 mL × 4) and dried under vacuum to give intermediate **36-1** (4.1 g). MS (ESI+, [M+H]⁺) *m*/*z:* 324.87.

### Step B: synthesis of compound 36-2

**36-1** (4.1 g), iodobenzene diacetate (4.06 g), acetonitrile (30 mL), ethyl acetate (30 mL), water (30 mL), and KOH (1.4 g) were added successively to a single-necked flask, and the mixture was stirred at room temperature overnight. After the reaction was completed, the reaction solution was filtered directly, and the filter cake was washed with water (10 mL × 2) and dried under vacuum to give intermediate **36-2** (2.8 g). MS (ESI+, [M+H]⁺) *m*/*z:* 322.85.

### Step C: synthesis of compound 36-3

**36-2** (2.0 g), DCM (50 mL), water (20 mL), TBAB (200 mg), 2,4-dinitrophenylhydroxylamine (1.8 g), and NaOH (500 mg) were added successively to a single-necked flask, and the mixture was stirred at room temperature overnight. After the reaction was completed, the reaction solution was concentrated to remove the organic solvent, and then filtered. The filter cake was slurried with 10 mL of acetonitrile and dried under vacuum to give intermediate **36-3** (800 mg). MS (ESI+, [M+H]⁺) *m*/*z:* 337.88.

### Step D: synthesis of Example 36

**36-3** (100 mg), 1,4-dioxane (5 mL), water (2 mL), **A-3** (205 mg), potassium carbonate (123 mg), 3-(di-*tert*-butylphosphino)propane-1-sulfonic acid (7.9 mg), and sodium tetrachloropalladate(II) (4.4 mg) were added successively to a single-necked flask, and the mixture was left to react at 80 °C for 2 h under N₂ atmosphere. After the reaction was completed, the reaction solution was concentrated, and the residue was dissolved in DCM (50 mL). The resulting solution was washed with saturated brine (10 mL × 2), dried over anhydrous sodium sulfate, filtered, and concentrated to give the crude product, which was purified by silica gel column chromatography to give **Example 36** (30 mg). MS (ESI+, [M+H]⁺) *m*/*z:* 478.29. ¹H NMR (500 MHz, Chloroform-*d*) δ 8.59 (d, *J =* 7.7 Hz, 1H), 8.44 (s, 1H), 8.15 (d, *J* = 11.2 Hz, 1H), 7.90 (d, *J* = 8.6 Hz, 1H), 6.89 (d, *J* = 8.6 Hz, 1H), 4.74 (s, 2H), 4.42 (t, *J* = 6.4 Hz, 2H), 3.40 - 3.26 (m, 1H), 2.54 - 2.40 (m, 6H), 2.07 - 2.02 (m, 2H), 1.63 - 1.58 (m, 4H), 1.51 - 1.41 (m, 4H), 1.32 - 1.29 (m, 2H).

### Example 37

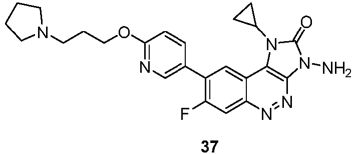

### Reaction process:

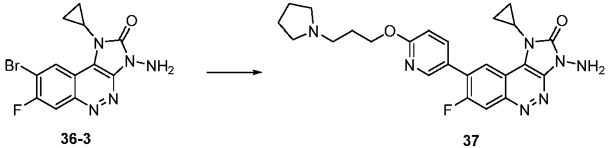

**36-3** (100 mg), 1,4-dioxane (5 mL), water (2 mL), **A-6** (197 mg), potassium carbonate (123 mg), 3-(di-*tert*-butylphosphino)propane-1-sulfonic acid (7.9 mg), and sodium tetrachloropalladate(II) (4.4 mg) were added successively to a single-necked flask, and the mixture was stirred at 80 °C for 2 h under N₂ atmosphere. After the reaction was completed, the reaction solution was concentrated, and the residue was dissolved in DCM (50 mL). The resulting solution was washed with saturated brine (2 × 10 mL), dried over anhydrous sodium sulfate, filtered, and concentrated to give the crude product, which was purified by silica gel column chromatography to give **Example 37** (35 mg). MS (ESI+, [M+H]⁺) *m*/*z:* 464.27. ¹H NMR (500 MHz, Chloroform-*d*) δ 8.59 (d, *J=* 7.7 Hz, 1H), 8.44 (s, 1H), 8.15 (d, *J* = 11.2 Hz, 1H), 7.91 (d, *J* = 8.7 Hz, 1H), 6.90 (d, *J* = 8.6 Hz, 1H), 4.73 (s, 2H), 4.44 (t, *J* = 6.5 Hz, 2H), 3.39 - 3.31 (m, 1H), 2.68 - 2.63 (m, 2H), 2.59 - 2.52 (m, 4H), 2.10 - 2.02 (m, 2H), 1.82 - 1.79 (m, 4H), 1.48 - 1.38 (m, 2H), 1.32 - 1.29 (m, 2H).

### Test Example 1: In Vitro Inhibitory Activity Against Kinase

### 1.1 Determination of inhibitory activity against ATM kinase

ATM kinase solution (concentration: 25 ng/µL) was added to assay wells at 6 µL/well, and different compounds dissolved in DMSO were added to the assay wells separately using a nanoliter pipettor, such that the final concentrations of the compounds were 10000 nM to 2.44 nM. In this experiment, the wells were set in duplicate, and a control well was set. After incubation of the above system for 30 min at room temperature, ATP (100 µM) was mixed with p53 substrate (50 nM) at a ratio of 1:1, and the mixture was added at 4 µL/well. The resulting mixture was left to react at room temperature for 2 h, and then 5 µL of EDTA was added to terminate the reaction, followed by addition of 5 µL of detection antibodies, Mab Anti-phospho p53 and Mab Anti GST-d2 (manufacturer: perkinelmer). The mixture was incubated at room temperature for 1 h. The plate was read using a PerkinElmer Envision multifunctional microplate reader (excitation: 320 nm, emission: 620 nm/665 nm), and IC₅₀ was calculated by four-parameter fitting.

### 1.2 Determination of inhibitory activity against ATR (Ataxia telangiectasia and Rad3-related) kinase

ATR/ATPIP (ATR-interacting protein) kinase solution (concentration: 50 ng/µL) was added to assay wells at 6 µL/well, and different compounds dissolved in DMSO were added to the assay wells separately using a nanoliter pipettor, such that the final concentrations of the compounds were 10000 nM to 2.44 nM. In this experiment, the wells were set in duplicate, and a control well was set. After incubation of the above system for 30 min at room temperature, ATP (5 µM) was mixed with p53 substrate (50 nM) at a ratio of 1:1, and the mixture was added at 4 µL/well. The resulting mixture was left to react at room temperature for 2 h, and then 5 µL of EDTA was added to terminate the reaction, followed by addition of 5 µL of detection antibodies, Mab Anti-phospho p53 and Mab Anti GST-d2 (manufacturer: perkinelmer). The mixture was incubated at room temperature for 1 h. The plate was read using a PerkinElmer Envision multifunctional microplate reader (excitation: 320 nm, emission: 620 nm/665 nm), and IC₅₀ was calculated by four-parameter fitting.

### 1.3 Determination of inhibitory activity against DNA-PK (DNA-dependent protein kinase)

DNA-PK kinase solution (concentration: 0.15 ng/µL) was added to assay wells at 6 µL/well, and different compounds dissolved in DMSO were added to the assay wells separately using a nanoliter pipettor, such that the final concentrations of the compounds were 10000 nM to 2.44 nM. In this experiment, the wells were set in duplicate, and a control well was set. After incubation of the above system for 30 min at room temperature, ATP (100 µM) was mixed with p53 substrate (50 nM) at a ratio of 1:1, and the mixture was added at 4 µL/well. The resulting mixture was left to react at room temperature for 2 h, and then 5 µL of EDTA was added to terminate the reaction, followed by addition of 5 µL of detection antibodies, Mab Anti-phospho p53 and Mab Anti GST-d2 (manufacturer: perkinelmer). The mixture was incubated at room temperature for 1 h. The plate was read using a PerkinElmer Envision multifunctional microplate reader (excitation: 320 nm, emission: 620 nm/665 nm), and IC₅₀ was calculated by four-parameter fitting.

The test results are shown in Table 1.

**Table 1**

| Example | | ATM (IC₅₀ nM) | ATR (IC₅₀ nM) | | DNA-PK (IC₅₀ nM) |
|---|---|---|---|---|---|
| | 1 | A | | 289 | >1000 |
| | 2 | A | | 821 | >1000 |
| | 3 | A | | >1000 | >1000 |
| | 4 | A | | >1000 | >1000 |
| | 5 | A | | >1000 | >1000 |
| | 6 | A | | >1000 | >1000 |
| | 7 | A | | >1000 | >1000 |
| | 8 | A | | >1000 | >1000 |
| | 10 | A | | >1000 | >1000 |
| | 11 | A | | >1000 | >1000 |
| | 12 | A | | >1000 | >1000 |
| | 13 | A | | >1000 | >1000 |
| | 14 | A | | >1000 | >1000 |
| | 15 | A | | >1000 | >1000 |
| | 16 | A | | >1000 | >1000 |
| | 17 | A | | >1000 | >1000 |
| | 18 | A | | >1000 | >1000 |
| | 19 | A | | >1000 | >1000 |
| | 20 | A | | >1000 | >1000 |
| | 21 | A | | >1000 | >1000 |
| | 22 | A | | | >1000 |
| | 23 | A | | | >1000 |
| | 24 | A | | | >1000 |
| | 25 | A | | | >1000 |
| | 26 | A | | | >1000 |
| | 27 | A | | | >1000 |
| | 28 | A | | | >1000 |
| | 29 | A | | | >1000 |
| | 30 | A | | 8087 | >10000 |
| | 31 | A | | 8721 | >10000 |
| | 33 | A | | >10000 | >10000 |
| | 34 | A | | >10000 | >10000 |
| | 35 | A | | >10000 | >10000 |
| | 36 | A | | >10000 | >10000 |
| | 37 | A | | >10000 | >10000 |

wherein, A represents that IC₅₀ < 50 nM.

The test results show that the compounds of the present application have good inhibitory activity against ATM kinase and exhibit selective inhibitory activity for ATM relative to ATR and/or DNA-PK.

### Test Example 2: Determination of In Vitro Inhibitory Activity Against Cells

### 2.1 Inhibitory activity against CHK2 phosphorylation of NCI-H2228 cells

NCI-H2228 cells in a good growth state were collected, added to a centrifuge tube, adjusted to a cell density of 1.25 × 10⁶ cells/mL, and seeded in a 384-well plate (8 µL/well). The cells were equilibrated in a cell incubator for 1.5 h, and then compounds were added using a nanoliter pipettor, such that the final concentrations of the compounds were 200 nM to 0.049 nM. In this experiment, the wells were set in duplicate, and a control well was set. After the mixture was further cultured for 1 h in the cell incubator, an assay was performed using a p-CHK2 (Thr68) assay kit (manufacturer: perkinelmer) and the Envision microplate reader AlphaLISA program. A four-parameter analysis was performed, a dose-response curve was fit, and IC₅₀ was calculated. The results are shown in Table 2.

**Table 2**

| Example | NCI-H2228 (IC₅₀ nM) |
|---|---|
| 2 | A |
| 3 | A |
| 5 | A |
| 7 | A |
| 11 | A |
| 13 | A |
| 14 | A |
| 17 | A |
| 18 | A |
| 19 | A |
| 20 | A |
| 21 | A |
| 22 | A |
| 23 | A |
| 24 | A |
| 25 | A |
| 26 | A |
| 27 | A |
| 28 | A |
| 29 | A |
| 31 | A |
| 32 | A |

wherein, A represents that IC₅₀ < 50 nM.

The test results show that the compounds of the present application have good inhibitory activity against CHK2 phosphorylation of NCI-H2228 cells.

### Test Example 3: In Vitro Pharmacokinetics

### 3.1 In vitro liver microsomal metabolic stability

Liver microsome incubation samples were prepared by mixing a PBS buffer (pH 7.4), a liver microsome solution (0.5 mg/mL; species: human, monkey, dog, rat, and mouse), a test compound, and an NADPH + MgCl₂ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The results are shown in Table 3 below.

**Table 3**

| **Example** | Human liver microsome | **Example** | Human liver microsome | **Example** | Human liver microsome |
|---|---|---|---|---|---|
| | Residual rate at 60 min% | | Residual rate at 60 min% | | Residual rate at 60 min% |
| 2 | 64.43 | 15 | 58.36 | 27 | 81.31 |
| 3 | 86.32 | 16 | 72.38 | 28 | 65.62 |
| 5 | 90.63 | 17 | 85.15 | 29 | 64.94 |
| 11 | 58.59 | 19 | 77.01 | 32 | 95.84 |
| 13 | 55.95 | 21 | 79.62 | | |
| 14 | 66.23 | 22 | 73.33 | | |

The results show that the test compounds of the present application exhibit stable *in vitro* metabolism (e.g., large residual content at 60 min).

### 3.2 In vitro plasma protein binding rate

Plasma samples were prepared by mixing blank plasma (mouse, rat, dog, and human) with a test compound solution (3 µM). High throughput equilibrium dialysis device (RED) samples were prepared as red chamber plasma samples and white chamber PBS buffer samples, which were incubated at 37 °C and 100 rpm for 4 h. An acetonitrile solution containing an internal standard was added to the plasma samples and buffer samples, and supernatants were prepared by protein precipitation, diluted, and then assayed by LC/MS/MS.

The results show that the test compounds of the present application have low *in vitro* plasma protein binding rate.

### Test Example 4: In Vivo Pharmacokinetics

### 4.1 Mouse pharmacokinetics

ICR mice weighing 21-23 g were randomized into groups of 9 in each group after 3-5 days of acclimatization and then intragastrically given the solution of Example 1 at a dose of 10 mg/kg. Plasma samples to be tested were prepared by taking blood from the orbit at time points of 0 min, 15 min, 30 min, 1 h, 2 h, 4 h, 6 h, 8 h, 10 h, and 24 h.

20 µL of each plasma sample to be tested and a standard curve sample were taken, and an acetonitrile solution containing an internal standard was added. Supernatants were obtained by protein precipitation, diluted, and then assayed by LC/MS/MS. Data were fitted using a non-compartmental model.

### 4.2 Rat pharmacokinetics

SD rats weighing 200-220 g were randomized into groups of 3 in each group after 3-5 days of acclimatization and then intragastrically given the solution of Example 1 at a dose of 10 mg/kg. Plasma samples to be tested were prepared by taking blood from the orbit at time points of 0 min, 15 min, 30 min, 1 h, 2 h, 4 h, 6 h, 8 h, 10 h, and 24 h.

50 µL of each plasma sample to be tested and a standard curve sample were taken, and an acetonitrile solution containing an internal standard was added. Supernatants were obtained by protein precipitation, diluted, and then assayed by LC/MS/MS. Data were fitted using a non-compartmental model.

### 4.3 Dog pharmacokinetics

Beagle dogs weighing 10-12 kg were randomized into groups with 3 in each group after 3-5 days of acclimatization and then intragastrically given the solution of Example 1 at a dose of 2 mg/kg. Plasma samples to be tested were prepared by taking blood from the forelimb vein at time points of 0 min, 15 min, 30 min, 1 h, 2 h, 4 h, 6 h, 8 h, 10 h, and 24 h.

50 µL of each plasma sample to be tested and a standard curve sample were taken, and an acetonitrile solution containing an internal standard was added. Supernatants were obtained by protein precipitation, diluted, and then assayed by LC/MS/MS. Data were fitted using a non-compartmental model. The results are shown in Table 4 below.

**Table 4**

| Example | Route of administration | Dose of administration | AUC₍₀₋ₜ₎ ng*h/ml | AUC_{(0-∞)} ng*h/ml | MRT₍₀₋ₜ₎ h | T_{1/2} h | Tmax h | Cmax ng/ml | Vz Lkg | CLz L/h/kg | Absolute F |
|---|---|---|---|---|---|---|---|---|---|---|---|
| 2 | IV | 1mg/kg | 521 | 539 | 2.11 | 1.7 | / | / | 4.55 | 1.86 | 79.65% |
| | IG | 10mg/kg | 4147 | 4272 | 6.1 | 4.59 | 0.5 | 521 | / | / | |
| 3 | IV | 1mg/kg | 445 | 460 | 2.1 | 1.74 | / | / | 5.45 | 2.18 | 95.54% |
| | IG | 10mg/kg | 4249 | 4329 | 6.05 | 4.01 | 1 | 495 | / | / | |
| 18 | IV | 1mg/kg | 587 | 624 | 1.89 | 2.17 | / | / | 3.9 | 1.6 | 75.88% |
| | IG | 10mg/kg | 4458 | 4501 | 4.99 | 3.51 | 4 | 506 | / | / | |
| 19 | IV | 1mg/kg | 451 | 474 | 1.84 | 2.05 | / | / | 4.82 | 2.11 | 88.26% |
| | IG | 10mg/kg | 3978 | 3998 | 4.72 | 3.07 | 4 | 485 | / | / | |
| 27 | IV | 1mg/kg | 271 | 280 | 1.43 | 1.87 | / | / | 6.11 | 3.58 | 115.39% |
| | IG | 10mg/kg | 3131 | 3186 | 5.7 | 4.12 | 2 | 342 | / | / | |
| 28 | IV | 1mg/kg | 2227 | 2237 | 3.24 | 3.1 | / | / | 1.5 | 0.447 | 77.43% |
| | IG | 10mg/kg | 17241 | 17322 | 5.56 | 3.04 | 4 | 1627 | / | / | |
| 29 | IV | 1mg/kg | 3144 | 3161 | 3.29 | 3.22 | / | / | 1.09 | 0.316 | 85.90% |
| | IG | 10mg/kg | 27003 | 27254 | 5.99 | 3.51 | 4 | 2298 | / | / | |

The test results show that the compounds of the present application have good *in vivo* pharmacokinetic properties (mice, rats, and dogs) and show advantages in Cₘₐₓ, AUC, t_{1/2}, and the like.

### 4.4 Experiment on mouse brain distribution

ICR mice weighing 22-24 g were randomized into groups with 9 in each group after 3-5 days of acclimatization and then intragastrically given the solution of Example 1 at a dose of 20 mg/kg. Plasma samples to be tested were prepared by taking blood from the orbit at time points of 15 min, 30 min, and 8 h.

Tissue collection was performed at time points of 15 min, 30 min, and 8 h, and at corresponding time points, animals were sacrificed by exsanguination, and brain tissues were taken out and homogenized with ice normal saline at a ratio of 1:3 (W/V) to prepare brain homogenate samples to be tested.

20 µL of each plasma sample and brain homogenate sample to be tested as well as a corresponding standard curve sample were taken, and an acetonitrile solution containing an internal standard was added. Supernatants were obtained by protein precipitation, diluted, and then assayed by LC/MS/MS. Data were fitted using a non-compartmental model.

The test results show that the compounds of the present application have high exposure (AUC) in plasma and brain and have a high brain-to-blood ratio (brain/plasma).

### Test Example 5: In Vivo Drug Effect Study

SPF-grade female nude mice were inoculated subcutaneously at the right armpit with 1 × 10⁷ HCT116 cells. When the mean tumor volume reached about 200 mm³, the animals were grouped (the day of grouping was d0).

The compound of the present application was administered at different doses (15 mpk, 30 mpk, or 60 mpk, intragastric administration), with 7 days constituting an administration cycle. The tumor volume was measured 2-3 times a week, and meanwhile, the mice were weighed and the data were recorded; the general behavior of the mice was observed and recorded every day. After the experiment was completed, the tumors were removed, weighed, and photographed.

The detection index and the calculation formulas are as follows:
Tumor volume TV (mm³) = 1/2 × (a × b²), wherein a represents the long diameter of the tumor, and b represents the short diameter of the tumor;

Relative tumor volume RTV = TVₜ/TV₀, wherein TV₀ represents the tumor volume on day 0, and TVt represents the tumor volume at each measurement;

Relative tumor proliferation rate T/C (%) = (T_{RTV}/C_{RTV}) × 100%, wherein T_{RTV} represents RTV of the treatment group, and C_{RTV} represents RTV of the vehicle control group;

Tumor growth inhibition rate TGI (%) = (1 - TW/TW₀) × 100%; wherein TW represents the tumor weight of the treatment group, and TW₀ represents the tumor weight of the vehicle control group; Weight change rate WCR (%) = (Wtt - Wt₀)/Wt₀ × 100%, wherein Wt₀ represents the body weight of the mouse on day 0, and Wtt represents the body weight of the mouse at each measurement.

The test results show that the compound of the present application can inhibit the growth of tumors *in vivo.*

## Claims

1. A compound of formula I-A, a pharmaceutically acceptable salt thereof, or a stereoisomer thereof, 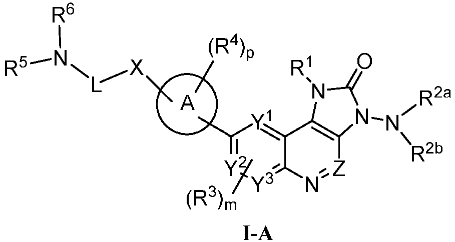 wherein,
R¹ is selected from the group consisting of hydrogen, C₁₋₁₀ alkyl, C₃₋₁₀ cycloalkyl, 3-10 membered heterocycloalkyl, C₃₋₁₀ aryl, and 3-10 membered heteroaryl, the C₁₋₁₀ alkyl, C₃₋₁₀ cycloalkyl, 3-10 membered heterocycloalkyl, C₃₋₁₀ aryl, or 3-10 membered heteroaryl being optionally substituted with one or more halogen, hydroxy, amino, cyano, C₁₋₆ alkyl, C₁₋₆ alkoxy, C₁₋₆ alkyl NH-, (C₁₋₆ alkyl)₂N-, C₃₋₁₀ cycloalkyl, 3-10 membered heterocycloalkyl, C₃₋₁₀ aryl, or 3-10 membered heteroaryl;
R^{2a} and R^{2b} are each independently selected from the group consisting of hydrogen, C₁₋₆ alkyl, C₃₋₁₀ cycloalkyl, 3-10 membered heterocycloalkyl, C₃₋₁₀ aryl, and 3-10 membered heteroaryl, or R^{2a} and R^{2b} are linked to each other to form 3-12 membered heterocycloalkyl, the C₁₋₆ alkyl, C₃₋₁₀ cycloalkyl, 3-10 membered heterocycloalkyl, C₃₋₁₀ aryl, 3-10 membered heteroaryl, or 3-12 membered heterocycloalkyl being optionally substituted with one or more deuterium, halogen, hydroxy, amino, cyano, C₁₋₆ alkyl, C₁₋₆ alkoxy, C₁₋₆ alkyl NH-, (C₁₋₆ alkyl)₂N-, C₃₋₁₀ cycloalkyl, 3-10 membered heterocycloalkyl, C₃₋₁₀ aryl, 3-10 membered heteroaryl, -COC₁₋₆ alkyl, -COOC₁₋₆ alkyl, -OCOC₁₋₆ alkyl, -CONHC₁₋₆ alkyl, -CON(C₁₋₆ alkyl)₂, -SO₂NHC₁₋₆ alkyl, or -SO₂N(C₁₋₆ alkyl)₂;
R³ and R⁴ are each independently selected from the group consisting of halogen, hydroxy, amino, cyano, nitro, C₁₋₆ alkyl, C₁₋₆ alkoxy, C₁₋₆ alkyl NH-, and (C₁₋₆ alkyl)₂N-, the C₁₋₆ alkyl, C₁₋₆ alkoxy, C₁₋₆ alkyl NH-, or (C₁₋₆ alkyl)₂N- being optionally substituted with one or more halogen, hydroxy, amino, cyano, nitro, or -COOH;
p and m are independently selected from the group consisting of 0, 1, 2, 3, and 4;
ring A is selected from the group consisting of phenyl and 5-10 membered heteroaryl;
X is selected from the group consisting of a single bond, -NR^{a}-, -O-, and -S-;
Y¹, Y², Y³, and Z are each independently selected from the group consisting of N and CH, and at least one of Y¹, Y², Y³, and Z is CH;
R^{a} is selected from the group consisting of hydrogen and C₁₋₆ alkyl;
L is selected from the group consisting of C₁₋₆ alkylene, C₃₋₁₀ cycloalkyl, 3-10 membered heterocycloalkyl, -C₁₋₆ alkylene-C₃₋₁₀ cycloalkyl-, -C₁₋₆ alkylene-3-10 membered heterocycloalkyl, -C₃₋₁₀ cycloalkyl-C₁₋₆ alkylene-, and -3-10 membered heterocycloalkyl-C₁₋₆ alkylene-;
R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-12 membered heterocycloalkyl, the C₁₋₆ alkyl, C₃₋₁₀ cycloalkyl, 3-10 membered heterocycloalkyl, or 3-12 membered heterocycloalkyl being optionally substituted with one or more groups selected from the group consisting of the following groups: deuterium, halogen, hydroxy, amino, cyano, C₁₋₆ alkyl, C₁₋₆ alkoxy, C₁₋₆ alkyl NH-, (C₁₋₆ alkyl)₂N-, or C₁₋₆ alkyl substituted with one or more halogen, hydroxy, amino, or cyano.

2. The compound, the pharmaceutically acceptable salt thereof, or the stereoisomer thereof according to claim 1, being selected from a compound of formula **I,** a pharmaceutically acceptable salt thereof, or a stereoisomer thereof, 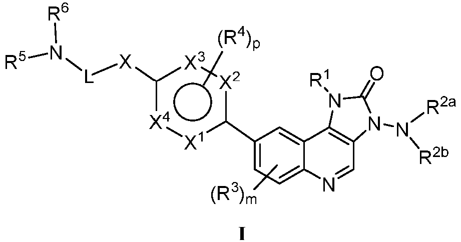 wherein,
R¹ is selected from the group consisting of hydrogen, C₁₋₆ alkyl, C₃₋₁₀ cycloalkyl, 3-10 membered heterocycloalkyl, C₃₋₁₀ aryl, and 3-10 membered heteroaryl, the C₁₋₆ alkyl, C₃₋₁₀ cycloalkyl, 3-10 membered heterocycloalkyl, C₃₋₁₀ aryl, or 3-10 membered heteroaryl being optionally substituted with one or more halogen, hydroxy, amino, cyano, C₁₋₆ alkyl, C₁₋₆ alkoxy, C₁₋₆ alkyl NH-, or (C₁₋₆ alkyl)₂N-;
R^{2a} and R^{2b} are each independently selected from the group consisting of hydrogen and C₁₋₆ alkyl, or R^{2a} and R^{2b} are linked to each other to form 3-10 membered heterocycloalkyl, the C₁₋₆ alkyl or 3-10 membered heterocycloalkyl being optionally substituted with one or more deuterium, halogen, hydroxy, amino, cyano, C₁₋₆ alkyl, C₁₋₆ alkoxy, C₁₋₆ alkyl NH-, or (C₁₋₆ alkyl)₂N-;
R³ and R⁴ are each independently selected from the group consisting of halogen, hydroxy, amino, cyano, C₁₋₆ alkyl, C₁₋₆ alkoxy, C₁₋₆ alkyl NH-, and (C₁₋₆ alkyl)₂N-;
p and m are independently selected from the group consisting of 0, 1, and 2;
X¹, X², X³, and X⁴ are each independently selected from the group consisting of N and CH, and one or more of X¹, X², X³, and X⁴ are N;
X is selected from the group consisting of a single bond, -NR^{a}-, -O-, and -S-;
R^{a} is selected from the group consisting of hydrogen and C₁₋₆ alkyl;
L is selected from the group consisting of C₁₋₆ alkylene, C₃₋₁₀ cycloalkyl, 3-10 membered heterocycloalkyl, -C₁₋₆ alkylene-C₃₋₁₀ cycloalkyl-, -C₁₋₆ alkylene-3-10 membered heterocycloalkyl, -C₃₋₁₀ cycloalkyl-C₁₋₆ alkylene-, and -3-10 membered heterocycloalkyl-C₁₋₆ alkylene-;
R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more deuterium, halogen, hydroxy, amino, cyano, C₁₋₆ alkyl, C₁₋₆ alkoxy, C₁₋₆ alkyl NH-, or (C₁₋₆ alkyl)₂N-.

3. The compound, the pharmaceutically acceptable salt thereof, or the stereoisomer thereof according to claim 1 or 2, wherein R¹ is selected from the group consisting of hydrogen, C₁₋₆ alkyl, C₃₋₁₀ cycloalkyl, 3-10 membered heterocycloalkyl, C₃₋₁₀ aryl, and 3-10 membered heteroaryl, the C₁₋₆ alkyl, C₃₋₁₀ cycloalkyl, 3-10 membered heterocycloalkyl, C₃₋₁₀ aryl, or 3-10 membered heteroaryl being optionally substituted with one or more halogen, hydroxy, amino, cyano, C₁₋₆ alkyl, C₁₋₆ alkoxy, C₁₋₆ alkyl NH-, or (C₁₋₆ alkyl)₂N-;
optionally, R¹ is selected from the group consisting of hydrogen, C₁₋₄ alkyl, C₃₋₈ cycloalkyl, 3-8 membered heterocycloalkyl, C₃₋₈ aryl, and 3-8 membered heteroaryl, the C₁₋₄ alkyl, C₃₋₈ cycloalkyl, 3-8 membered heterocycloalkyl, C₃₋₈ aryl, or 3-8 membered heteroaryl being optionally substituted with one or more halogen, hydroxy, amino, cyano, C₁₋₄ alkyl, C₁₋₄ alkoxy, C₁₋₄ alkyl NH-, or (C₁₋₄ alkyl)₂N-;
optionally, R¹ is selected from the group consisting of hydrogen, C₁₋₄ alkyl, C₃₋₆ cycloalkyl, 3-6 membered heterocycloalkyl, C₃₋₆ aryl, and 3-6 membered heteroaryl, the C₁₋₄ alkyl, C₃₋₆ cycloalkyl, 3-6 membered heterocycloalkyl, C₃₋₆ aryl, or 3-6 membered heteroaryl being optionally substituted with one or more halogen, hydroxy, amino, cyano, C₁₋₄ alkyl, C₁₋₄ alkoxy, C₁₋₄ alkyl NH-, or (C₁₋₄ alkyl)₂N-;
optionally, R¹ is selected from the group consisting of C₁₋₃ alkyl, C₃₋₆ cycloalkyl, 5-6 membered heterocycloalkyl, C₅₋₆ aryl, and 5-6 membered heteroaryl, the C₄₋₆ cycloalkyl, 5-6 membered heteroaryl, C₅₋₆ aryl, or 5-6 membered heteroaryl being optionally substituted with one or more halogen, hydroxy, amino, cyano, C₁₋₃ alkyl, or C₁₋₃ alkoxy;
optionally, R¹ is selected from the group consisting of C₁₋₃ alkyl, C₃₋₅ cycloalkyl, 6-membered heterocycloalkyl, phenyl, and 6-membered heteroaryl, the C₄₋₅ cycloalkyl, 6-membered heterocycloalkyl, phenyl, or 6-membered heteroaryl being optionally substituted with one or more halogen or C₁₋₃ alkoxy;
optionally, R¹ is selected from the group consisting of propyl, cyclopropyl, cyclobutyl, cyclopentyl, 6-membered oxygen-containing heterocycloalkyl, and 6-membered nitrogen-containing heteroaryl, the cyclobutyl, cyclopentyl, 6-membered oxygen-containing heterocycloalkyl, or 6-membered nitrogen-containing heteroaryl being optionally substituted with one or more fluoro or methoxy;
optionally, R¹ is selected from the group consisting of propyl, cyclopropyl, cyclobutyl, cyclopentyl, tetrahydropyranyl, and pyridinyl, the cyclobutyl, cyclopentyl, or pyridinyl being optionally substituted with one or more fluoro or methoxy;
optionally, R¹ is selected from the group consisting of isopropyl,

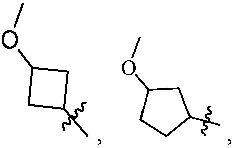

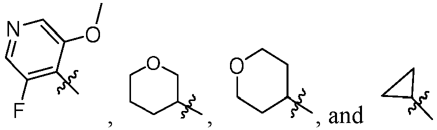

4. The compound, the pharmaceutically acceptable salt thereof, or the stereoisomer thereof according to any one of claims 1-3, wherein R^{2a} and R^{2b} are each independently selected from the group consisting of hydrogen and C₁₋₆ alkyl, or R^{2a} and R^{2b} are linked to each other to form 3-10 membered heterocycloalkyl, the C₁₋₆ alkyl or 3-10 membered heterocycloalkyl being optionally substituted with one or more deuterium, halogen, hydroxy, amino, cyano, C₁₋₆ alkyl, C₁₋₆ alkoxy, C₁₋₆ alkyl NH-, or (C₁₋₆ alkyl)₂N-;
optionally, R^{2a} and R^{2b} are each independently selected from the group consisting of hydrogen and C₁₋₄ alkyl, or R^{2a} and R^{2b} are linked to each other to form 3-8 membered heterocycloalkyl, the C₁₋₄ alkyl or 3-8 membered heterocycloalkyl being optionally substituted with one or more deuterium, halogen, hydroxy, amino, cyano, C₁₋₄ alkyl, C₁₋₄ alkoxy, C₁₋₄ alkyl NH-, or (C₁₋₄ alkyl)₂N-;
optionally, R^{2a} and R^{2b} are each independently selected from the group consisting of hydrogen and C₁₋₄ alkyl, or R^{2a} and R^{2b} are linked to each other to form 3-4 membered heterocycloalkyl, the C₁₋₄ alkyl or 3-4 membered heterocycloalkyl being optionally substituted with one or more deuterium, halogen, hydroxy, amino, cyano, or C₁₋₃ alkyl;
optionally, R^{2a} and R^{2b} are each independently selected from the group consisting of hydrogen and C₁₋₄ alkyl, the C₁₋₄ alkyl being optionally substituted with one or more deuterium, halogen, hydroxy, amino, or cyano;
optionally, R^{2a} and R^{2b} are each independently selected from the group consisting of hydrogen and C₁₋₄ alkyl, the C₁₋₄ alkyl being optionally substituted with one or more deuterium, fluoro, chloro, or bromo;
optionally, R^{2a} and R^{2b} are each independently selected from the group consisting of hydrogen and C₁₋₃ alkyl, the C₁₋₃ alkyl being optionally substituted with one or more deuterium; optionally, R^{2a} and R^{2b} are each independently selected from the group consisting of hydrogen and CH₃-, the CH₃- being optionally substituted with one or more deuterium;
optionally, R^{2a} and R^{2b} are each independently selected from the group consisting of hydrogen, CH₃-, and CD₃-.

5. The compound, the pharmaceutically acceptable salt thereof, or the stereoisomer thereof according to any one of claims 1-4, wherein R³ and R⁴ are each independently selected from the group consisting of halogen, hydroxy, amino, cyano, C₁₋₆ alkyl, C₁₋₆ alkoxy, C₁₋₆ alkyl NH-, and (C₁₋₆ alkyl)₂N-;
optionally, R³ and R⁴ are each independently selected from the group consisting of halogen, hydroxy, amino, cyano, C₁₋₃ alkyl, C₁₋₃ alkoxy, C₁₋₃ alkyl NH-, and (C₁₋₃ alkyl)₂N-;
optionally, R³ and R⁴ are each independently selected from the group consisting of halogen and C₁₋₃ alkoxy;
optionally, R³ is selected from the group consisting of fluoro, chloro, bromo, and C₁₋₃ alkoxy; optionally, R³ is selected from the group consisting of fluoro and methoxy;
optionally, R⁴ is selected from the group consisting of halogen, hydroxy, amino, cyano, methyl, and methoxy.

6. The compound, the pharmaceutically acceptable salt thereof, or the stereoisomer thereof according to any one of claims 1-5, wherein p and m are independently selected from the group consisting of 0, 1, and 2;
optionally, p is selected from the group consisting of 0 and 1;
optionally, p is 0;
optionally, m is selected from the group consisting of 0 and 1.

7. The compound, the pharmaceutically acceptable salt thereof, or the stereoisomer thereof according to any one of claim 1 and claims 3-6, wherein ring A is selected from the group consisting of phenyl, 5-6 membered heteroaryl, and 9-10 membered heteroaryl;
optionally, ring A is selected from the group consisting of 5-6 membered heteroaryl and 9-10 membered heteroaryl;
optionally, ring A is selected from the group consisting of pyridinyl, pyrimidinyl, pyrazolyl, imidazolyl, triazolyl, pyrazinyl, quinolinyl, isoquinolinyl, indolyl, isoindolyl, triazinyl, benzimidazolyl, and imidazopyridinyl;
optionally, ring A is selected from the group consisting of pyridinyl and pyrimidinyl.

8. The compound, the pharmaceutically acceptable salt thereof, or the stereoisomer thereof according to any one of claim 1 and claims 3-7, wherein Y¹, Y², Y³, and Z are each independently selected from the group consisting of N and CH, and 1, 2, 3, or 4 of Y¹, Y², Y³, and Z are CH;
optionally, Y¹ is N, and Y² and Y³ are CH; or
Y² is N, and Y¹ and Y³ are CH; or
Y³ is N, and Y¹ and Y² are CH; or
Y¹, Y², or Y³ is CH; or
Y¹, Y², Y³, or Z is CH;
optionally, Z is selected from the group consisting of N and CH; or
Z is N; or Z is CH.

9. The compound, the pharmaceutically acceptable salt thereof, or the stereoisomer thereof according to any one of claims 2-6, wherein X¹, X², X³, and X⁴ are each independently selected from the group consisting of N and CH, and one or more of X¹, X², X³, and X⁴ are N;
optionally, X¹, X², X³, and X⁴ are each independently selected from the group consisting of N and CH, and 1 or 2 of X¹, X², X³, and X⁴ are N;
optionally, X⁴ is N, and X¹, X², and X³ are each independently selected from the group consisting of N and CH;
optionally, X⁴ is N, X³ is selected from the group consisting of N and CH, and X¹ and X² are CH;
optionally, X⁴ is N, and X¹, X², or X³ is CH;
optionally, X³ and X⁴ are N, and X¹ and X² are CH.

10. The compound, the pharmaceutically acceptable salt thereof, or the stereoisomer thereof according to any one of claims 1-9, wherein X is selected from the group consisting of -NR^{a}- and -O-;
optionally, X is selected from the group consisting of -NH- and -O-;
optionally, X is -O-.

11. The compound, the pharmaceutically acceptable salt thereof, or the stereoisomer thereof according to any one of claims 1-10, wherein R^{a} is selected from the group consisting of hydrogen and C₁₋₆ alkyl;
optionally, R^{a} is selected from the group consisting of hydrogen and C₁₋₄ alkyl;
optionally, R^{a} is selected from the group consisting of hydrogen and C₁₋₃ alkyl;
optionally, R^{a} is selected from the group consisting of hydrogen and methyl;
optionally, R^{a} is hydrogen.

12. The compound, the pharmaceutically acceptable salt thereof, or the stereoisomer thereof according to any one of claims 1-11, wherein L is selected from the group consisting of C₁₋₆ alkylene, C₃₋₁₀ cycloalkyl, 3-10 membered heterocycloalkyl, -C₁₋₆ alkylene-C₃₋₁₀ cycloalkyl-, -C₁₋₆ alkylene-3-10 membered heterocycloalkyl, -C₃₋₁₀ cycloalkyl-C₁₋₆ alkylene-, and -3-10 membered heterocycloalkyl-C₁₋₆ alkylene-;
optionally, L is selected from the group consisting of C₁₋₄ alkylene, C₃₋₈ cycloalkyl, 3-8 membered heterocycloalkyl, -C₁₋₄ alkylene-C₃₋₈ cycloalkyl-, -C₁₋₄ alkylene-3-8 membered heterocycloalkyl-, -C₃₋₈ cycloalkyl-C₁₋₄ alkylene-, and -3-8 membered heterocycloalkyl-C₁₋₄ alkylene-;
optionally, L is selected from the group consisting of C₁₋₄ alkylene, C₃₋₆ cycloalkyl, 3-6 membered heterocycloalkyl, -C₁₋₄ alkylene-C₃₋₆ cycloalkyl-, -C₁₋₄ alkylene-3-6 membered heterocycloalkyl-, -C₃₋₆ cycloalkyl-C₁₋₄ alkylene-, and -3-6 membered heterocycloalkyl-C₁₋₄ alkylene-;
optionally, L is selected from the group consisting of C₁₋₄ alkylene, C₃₋₆ cycloalkyl, -C₁₋₄ alkylene-C₃₋₆ cycloalkyl-, and -C₃₋₆ cycloalkyl-C₁₋₄ alkylene-;
optionally, L is selected from the group consisting of C₂₋₄ alkylene, C₄₋₆ cycloalkyl, -C₁₋₂ alkylene-C₄₋₆ cycloalkyl-, and -C₄₋₆ cycloalkyl-C₁₋₂ alkylene-;
optionally, L is selected from the group consisting of C₂₋₃ alkylene, C₄₋₆ cycloalkyl, -CH₂-C₄ cycloalkyl-, and -C₄ cycloalkyl-CH₂-;
optionally, L is selected from the group consisting of -CH₂CH₂-, -CH₂CH₂CH₂-,

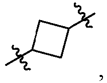

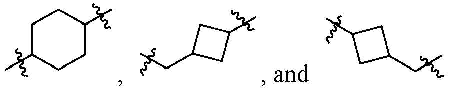

13. The compound, the pharmaceutically acceptable salt thereof, or the stereoisomer thereof according to any one of claims 1-12, wherein R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more groups selected from the group consisting of the following groups: deuterium, halogen, hydroxy, amino, cyano, C₁₋₆ alkyl, C₁₋₆ alkoxy, C₁₋₆ alkyl NH-, (C₁₋₆ alkyl)₂N-, or C₁₋₆ alkyl substituted with one or more halogen, hydroxy, amino, or cyano;
optionally, R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more groups selected from the group consisting of the following groups: deuterium, halogen, hydroxy, amino, cyano, C₁₋₆ alkyl, C₁₋₆ alkoxy, C₁₋₆ alkyl NH-, (C₁₋₆ alkyl)₂N-, or C₁₋₆ alkyl substituted with one or more halogen; or
R⁵ and R⁶ are each independently selected from the group consisting of hydrogen, C₁₋₄ alkyl, C₃₋₈ cycloalkyl, and 3-8 membered heterocycloalkyl, or R⁵ and R⁶ are linked to each other to form 3-8 membered heterocycloalkyl, the C₁₋₄ alkyl, C₃₋₈ cycloalkyl, or 3-8 membered heterocycloalkyl being optionally substituted with one or more groups selected from the group consisting of the following groups: deuterium, halogen, hydroxy, amino, cyano, C₁₋₄ alkyl, C₁₋₄ alkoxy, C₁₋₄ alkyl NH-, (C₁₋₄ alkyl)₂N-, or C₁₋₆ haloalkyl; or
R⁵ and R⁶ are each independently selected from the group consisting of C₁₋₄ alkyl and C₃₋₆ cycloalkyl, or R⁵ and R⁶ are linked to each other to form 3-7 membered heterocycloalkyl, the C₃₋₆ cycloalkyl or 3-7 membered heterocycloalkyl being optionally substituted with one or more groups selected from the group consisting of the following groups: deuterium, halogen, hydroxy, amino, cyano, C₁₋₃ alkyl, C₁₋₃ alkoxy, or C₁₋₄ haloalkyl; or
R⁵ and R⁶ are each independently selected from the group consisting of C₁₋₄ alkyl and C₃₋₆ cycloalkyl, or R⁵ and R⁶ are linked to each other to form 3-7 membered heterocycloalkyl, the 3-7 membered heterocycloalkyl being optionally substituted with one or more groups selected from the group consisting of the following groups: deuterium, halogen, hydroxy, amino, cyano, C₁₋₃ alkyl, C₁₋₃ alkoxy, or C₁₋₄ haloalkyl; or
R⁵ and R⁶ are each independently selected from the group consisting of C₁₋₃ alkyl and C₃₋₄ cycloalkyl, or R⁵ and R⁶ are linked to each other to form 4-, 5-, 6-, or 7-membered heterocycloalkyl, the heterocycloalkyl being optionally substituted with one or more halogen or C₁₋₃ haloalkyl; or
R⁵ and R⁶ are each independently selected from the group consisting of C₁₋₃ alkyl and C₃₋₄ cycloalkyl; or
R⁵ and R⁶ are linked to each other to form 4-, 5-, 6-, or 7-membered heterocycloalkyl, the heterocycloalkyl being optionally substituted with one or more halogen or C₁₋₃ haloalkyl; or
R⁵ and R⁶ are linked to each other to form 4-, 5-, or 6-membered heterocycloalkyl, the heterocycloalkyl being optionally substituted with one or more halogen or C₁₋₃ haloalkyl; or
R⁵ and R⁶ are linked to each other to form 4-membered heterocycloalkyl, the heterocycloalkyl being optionally substituted with one or more halogen or C₁₋₃ haloalkyl; or
R⁵ and R⁶ are linked to each other to form 5-membered heterocycloalkyl, the heterocycloalkyl being optionally substituted with one or more halogen or C₁₋₃ haloalkyl; or
R⁵ and R⁶ are linked to each other to form 6-membered heterocycloalkyl, the heterocycloalkyl being optionally substituted with one or more halogen or C₁₋₃ haloalkyl; or
R⁵ and R⁶ are each independently selected from the group consisting of methyl and cyclopropyl, or R⁵ and R⁶ are linked to each other to form azetidinyl, pyrrolidinyl, piperidinyl, azaspiroheptyl, and azabicycloheptyl, the azetidinyl or pyrrolidinyl being optionally substituted with one or more F or -CH₂F; or
R⁵ and R⁶ are each independently selected from the group consisting of methyl and cyclopropyl, or R⁵ and R⁶ are linked to each other to form azetidinyl, pyrrolidinyl, or piperidinyl, the azetidinyl, pyrrolidinyl, or piperidinyl being optionally substituted with one or more halogen or C₁₋₃ haloalkyl; or
R⁵ and R⁶ are each independently selected from the group consisting of methyl and cyclopropyl, or R⁵ and R⁶ are linked to each other to form azetidinyl, pyrrolidinyl, or piperidinyl, the azetidinyl or pyrrolidinyl being optionally substituted with one or more F or -CH₂F;
optionally, R⁵ and R⁶ are each independently selected from the group consisting of hydrogen, C₁₋₆ alkyl, C₃₋₁₀ cycloalkyl, and 3-10 membered heterocycloalkyl, or R⁵ and R⁶ are linked to each other to form 3-10 membered heterocycloalkyl, the C₁₋₆ alkyl, C₃₋₁₀ cycloalkyl, or 3-10 membered heterocycloalkyl being optionally substituted with one or more deuterium, halogen, hydroxy, amino, cyano, C₁₋₆ alkyl, C₁₋₆ alkoxy, C₁₋₆ alkyl NH-, or (C₁₋₆ alkyl)₂N-; or
R⁵ and R⁶ are each independently selected from the group consisting of hydrogen, C₁₋₄ alkyl, C₃₋₈ cycloalkyl, and 3-8 membered heterocycloalkyl, or R⁵ and R⁶ are linked to each other to form 3-8 membered heterocycloalkyl, the C₁₋₄ alkyl, C₃₋₈ cycloalkyl, or 3-8 membered heterocycloalkyl being optionally substituted with one or more deuterium, halogen, hydroxy, amino, cyano, C₁₋₄ alkyl, C₁₋₄ alkoxy, C₁₋₄ alkyl NH-, or (C₁₋₄ alkyl)₂N-; or
R⁵ and R⁶ are each independently selected from the group consisting of C₁₋₄ alkyl and C₃₋₆ cycloalkyl, or R⁵ and R⁶ are linked to each other to form 3-6 membered heterocycloalkyl, the C₃₋₆ cycloalkyl or 3-6 membered heterocycloalkyl being optionally substituted with one or more deuterium, halogen, hydroxy, amino, cyano, C₁₋₃ alkyl, or C₁₋₃ alkoxy; or
R⁵ and R⁶ are each independently selected from the group consisting of C₁₋₃ alkyl and C₃₋₄ cycloalkyl, or are linked to each other to form 5-6 membered heterocycloalkyl; or
R⁵ and R⁶ are each independently selected from the group consisting of methyl and cyclopropyl, or are linked to each other to form pyrrolidinyl or piperidinyl.

14. The compound, the pharmaceutically acceptable salt thereof, or the stereoisomer thereof according to any one of claims 2-6 and claims 9-13, wherein the structural fragment 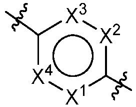 is selected from the group consisting of

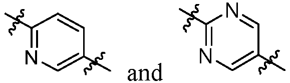

15. The compound, the pharmaceutically acceptable salt thereof, or the stereoisomer thereof according to any one of claims 1-14, wherein the structural fragment 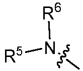 is selected from the group consisting of 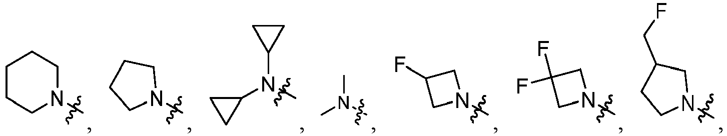 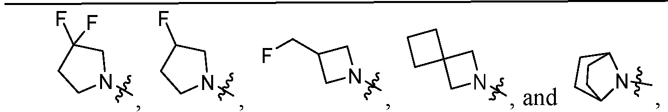 or the structural fragment 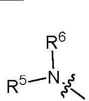 is selected from the group consisting of 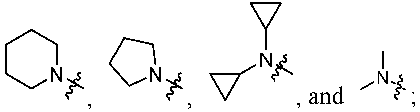 optionally, the structural fragment 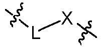 is selected from the group consisting of 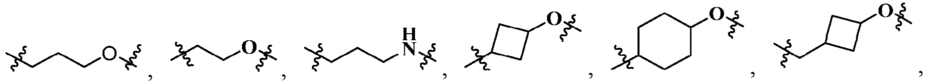 and 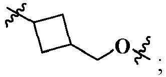 or the structural fragment 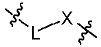 is selected from the group consisting of 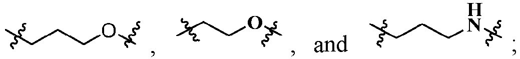 or the structural fragment 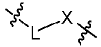 is selected from the group consisting of 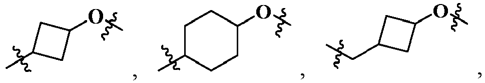 and 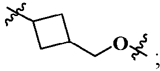 or the structural fragment 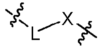 is selected from the group consisting of 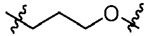 and 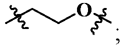 or the structural fragment 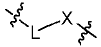 is selected from the group consisting of 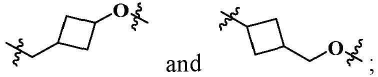 optionally, the structural fragment 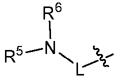 is selected from the group consisting of 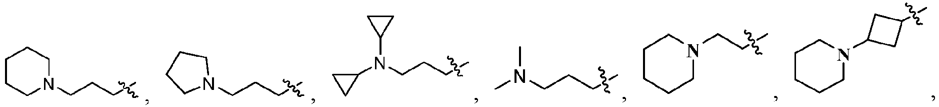 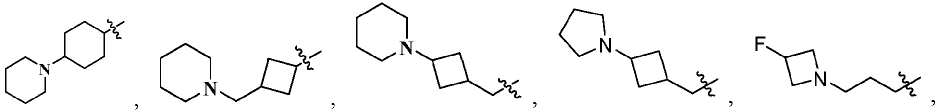 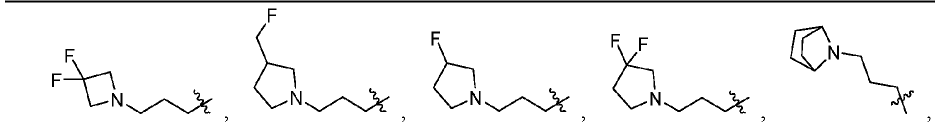 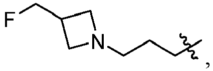 and 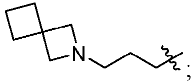 or the structural fragment 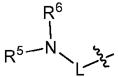 is selected from the group consisting of 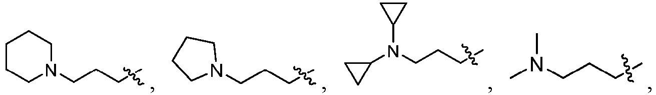 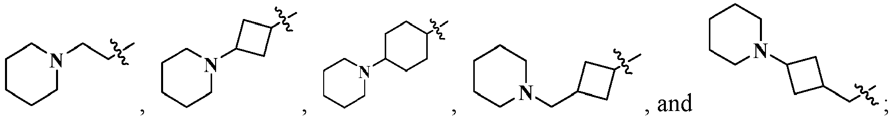 optionally, the structural fragment 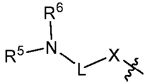 is selected from the group consisting of 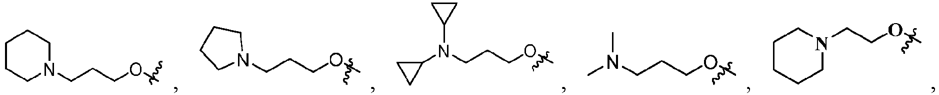 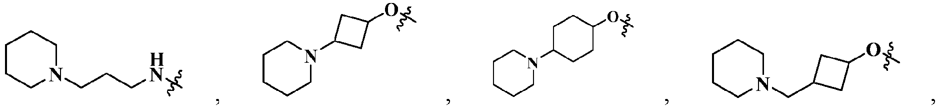 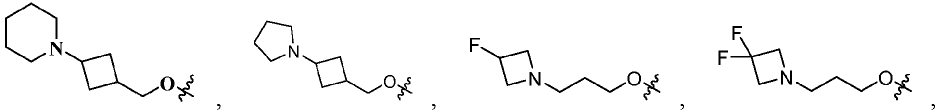 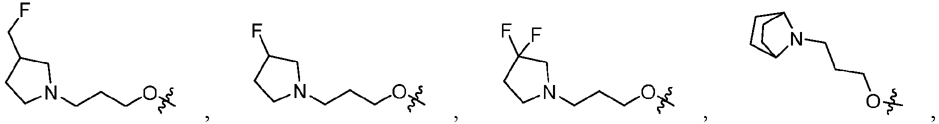 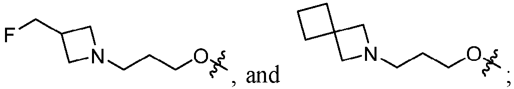 or the structural fragment 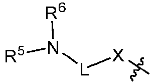 is selected from the group consisting of

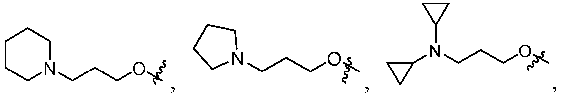

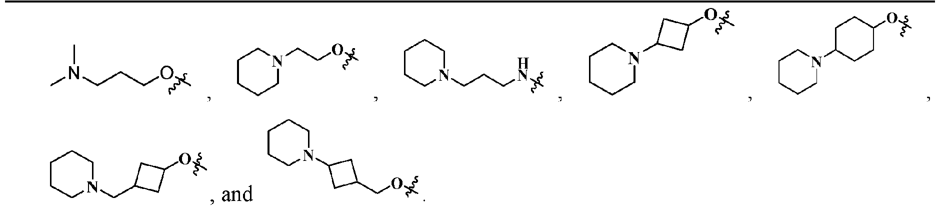

16. The compound, the pharmaceutically acceptable salt thereof, or the stereoisomer thereof according to any one of claims 1-15, wherein the compound of formula I-A or formula I is selected from a compound of formula II,

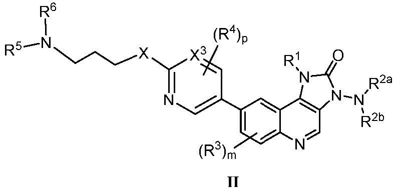

17. The compound, the pharmaceutically acceptable salt thereof, or the stereoisomer thereof according to any one of claims 1-16, wherein the compound of formula I-A, formula I, or formula II is selected from the group consisting of a compound of formula III and a compound of formula IV,

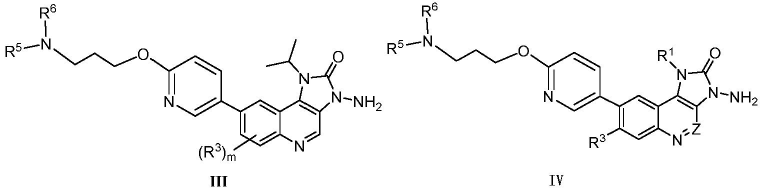

18. Compounds, pharmaceutically acceptable salts thereof, or stereoisomers thereof as follows: 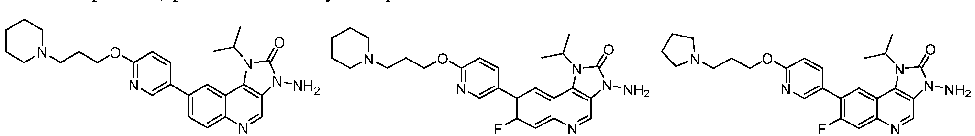 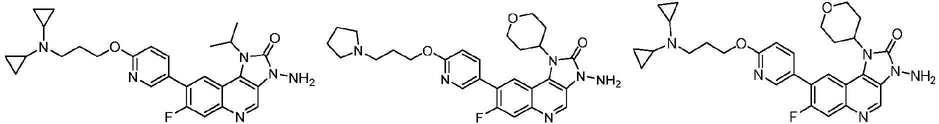 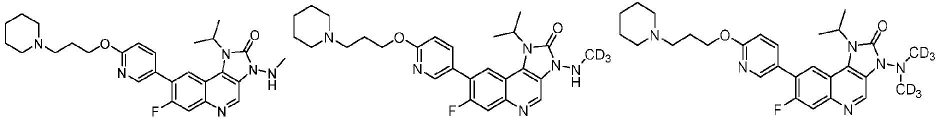 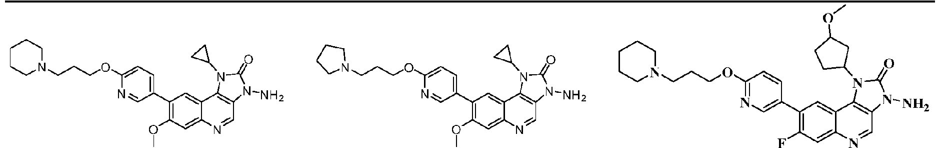 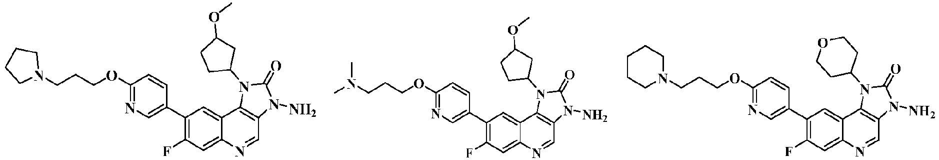 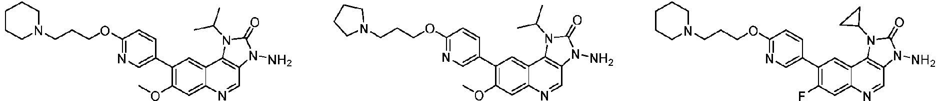 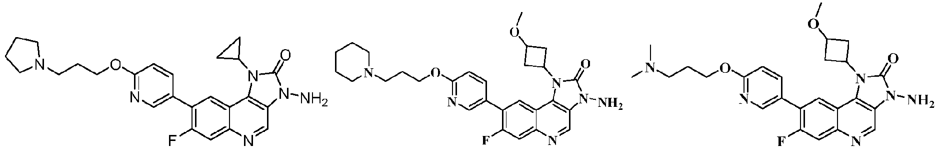 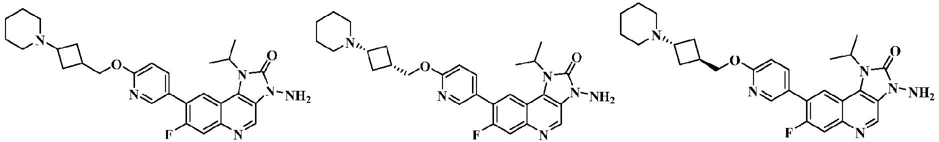 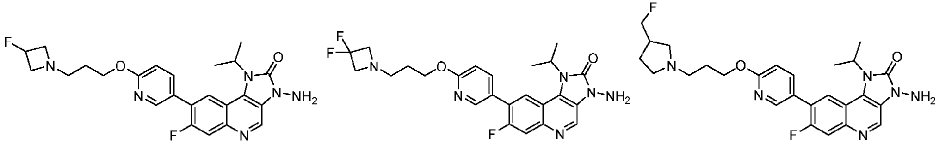 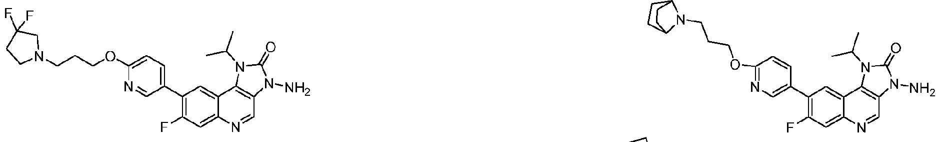 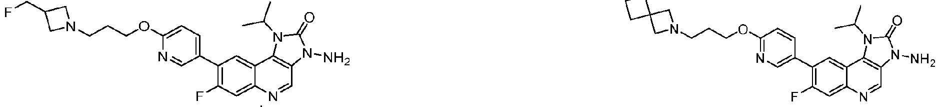 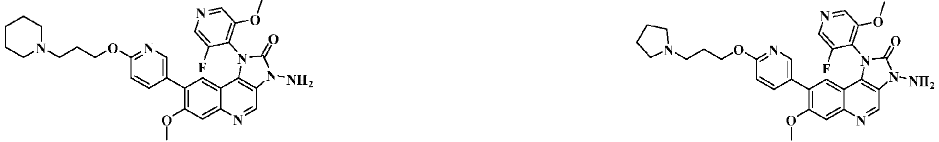 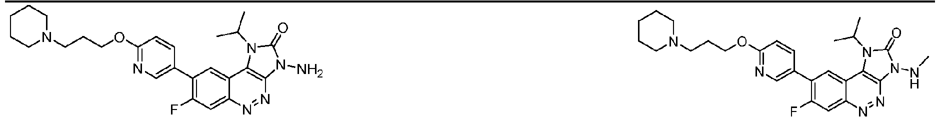 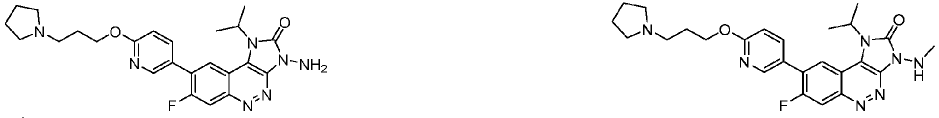 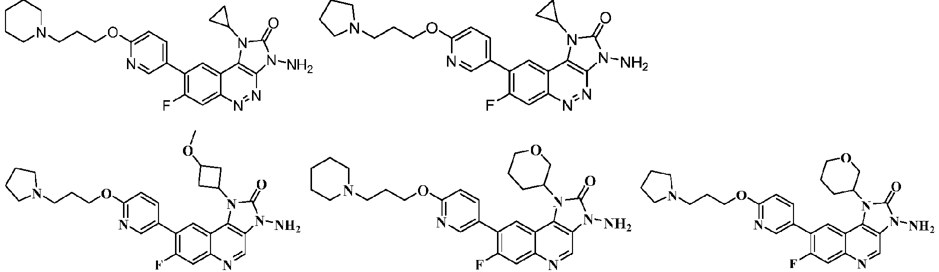 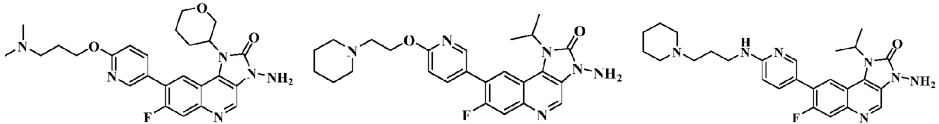 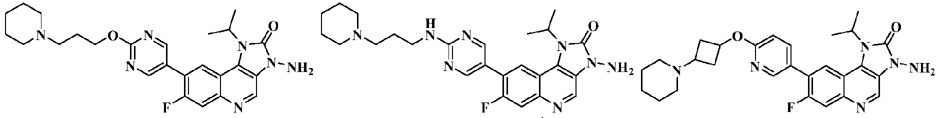 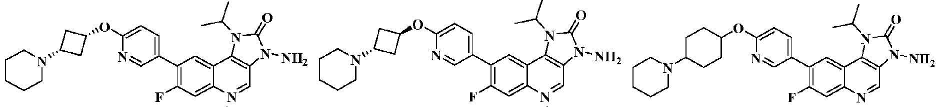 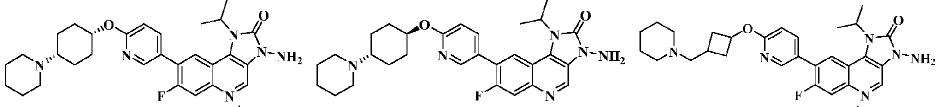 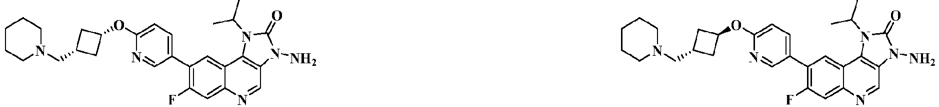 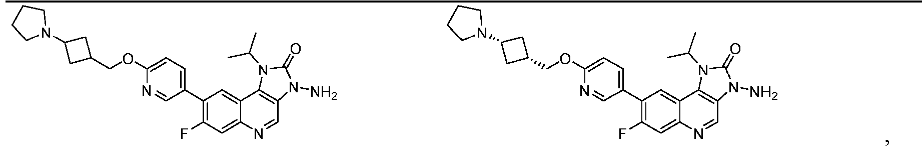 or

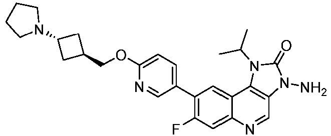

19. A pharmaceutical composition, comprising the compound, the pharmaceutically acceptable salt thereof, or the stereoisomer thereof according to any one of claims 1-18.

20. A method for treating tumors, comprising administering to a mammal, preferably a human, in need of the treatment a therapeutically effective amount of the compound, the pharmaceutically acceptable salt thereof, or the stereoisomer thereof according to any one of claims 1-18 or the pharmaceutical composition according to claim 19.

21. Use of the compound, the pharmaceutically acceptable salt thereof, or the stereoisomer thereof according to any one of claims 1-18 or the pharmaceutical composition according to claim 19 in preparation of a medicament for treating tumors.

22. Use of the compound, the pharmaceutically acceptable salt thereof, or the stereoisomer thereof according to any one of claims 1-18 or the pharmaceutical composition according to claim 19 for treating tumors.

23. The compound, the pharmaceutically acceptable salt thereof, or the stereoisomer thereof according to any one of claims 1-18 or the pharmaceutical composition according to claim 19 for use in treating tumors.
